# EUROPÄISCHE PATENTSCHRIFT

(11) **EP 1 805 212 B1**
(45) Veröffentlichungstag und Bekanntmachung des Hinweises auf die Patenterteilung: **10.06.2009**
(21) Anmeldenummer: 05797316.6
(22) Anmeldetag: 08.10.2005
(51) Int. Cl.: C07K 11/02

(54) **DESOXO-NONADEPSIPEPTIDE**
DESOXO-NONADEPSIPEPTIDES
DESOXO-NONADEPSIPEPTIDES

(30) Priorität: 20.10.2004 DE 102004051023
(43) Veröffentlichungstag der Anmeldung: 11.07.2007
(73) Patentinhaber: AiCuris GmbH & Co. KG, 42117 Wuppertal (DE)
(72) Erfinder: VON NUSSBAUM, Franz, 40219 Düsseldorf (DE); BRUNNER, Nina, 45279 Essen (DE); ENDERMANN, Rainer, 42113 Wuppertal (DE); FÜRSTNER, Chantal, 45478 Mühlheim/Ruhr (DE); HARTMANN, Elke, 42111 Wuppertal (DE); RAGOT, Jacques, 40625 Düsseldorf (DE); SCHIFFER, Guido, 42111 Wuppertal (DE); SCHUHMACHER, Joachim, 42113 Wuppertal (DE); SVENSTRUP, Niels, 42555 Velbert (DE); TELSER, Joachim, 51061 Köln (DE); ANLAUF, Sonja, 42115 Wuppertal (DE); BRÜNING, Michael-Alexander, 13469 Berlin (DE)
(74) Vertreter: Weller, Wolfgang
(86) Internationale Anmeldenummer: PCT/EP2005/010858
(87) Internationale Veröffentlichungsnummer: WO 2006/042655

(56) Entgegenhaltungen:
- EP-A- 0 196 042
- WO-A-01/05814
- WO-A-20/04099239
- US-A- 4 754 018
- EGNER B J ET AL: "Monitoring the solid phase synthesis of analogues of lysobactin and the katanosins using in situ MALDI-TOF MS" TETRAHEDRON, ELSEVIER SCIENCE PUBLISHERS, AMSTERDAM, NL, Bd. 53, Nr. 41, 13. Oktober 1997 (1997-10-13), Seiten 14021-14030, XP004618631 ISSN: 0040-4020

## Beschreibung

Die Erfindung betrifft Nonadepsipeptide und Verfahren zu ihrer Herstellung sowie ihre Verwendung zur Herstellung von Arzneimitteln zur Behandlung und/oder Prophylaxe von Krankheiten, insbesondere bakteriellen Infektionskrankheiten.

Die bakterielle Zellwand wird durch eine Reihe von Enzymen synthetisiert (Zellwandbiosynthese) und ist essentiell für das Überleben bzw. die Vermehrung von Mikroorganismen. Die Struktur dieses Makromoleküls ebenso wie die an ihrer Synthese beteiligten Proteine sind innerhalb der Bakterien stark konserviert. Aufgrund ihrer essentiellen Natur und Einheitlichkeit ist die Zellwandbiosynthese ein idealer Angriffspunkt für neue Antibiotika (D.W.Green, The bacterial cell wall as a source of antibacterial targets, Expert Opin. Ther. Targets, 2002, 6, 1-19).

Vancomycin und Penicilline sind Inhibitoren der bakteriellen Zellwandbiosynthese und stellen erfolgreiche Beispiele für die antibiotische Potenz dieses Wirkprinzips dar. Sie werden seit mehreren Jahrzehnten in der Klinik zur Behandlung von bakteriellen Infektionen, vor allem mit Grampositiven Erregern eingesetzt. Durch das wachsende Auftreten von resistenten Keimen, z.B. Methicillin-resistenten Staphylokokken, Penicillin-resistenten Pneumokokken und Vancomycinresistenten Enterokokken (F. Baquero, Gram-positive resistance: challenge for the development of new antibiotics, J. Antimicrob. Chemother., 1997, 39, Suppl A: 1-6; A.P. Johnson, D.M. Livermore, G.S. Tillotson, Antimicrobial susceptibility of Gram-positive bacteria: what's current, what's anticipated ?, J. Hosp. Infect., 2001, (49), Suppl A: 3-11) sowie jüngst auch erstmals Vancomycinresistenten Staphylokokken (B. Goldrick, First reported case of VRSA in the United States, Am. J. Nurs., 2002, 102, 17) verlieren diese Substanzen zunehmend ihre therapeutische Wirksamkeit.

Die vorliegende Erfindung beschreibt eine neue Klasse von Zellwandbiosynthese-Inhibitoren ohne Kreuzresistenzen zu bekannten Antibiotika-Klassen.

Der Naturstoff Lysobactin und einige Derivate sind als antibakteriell wirksam beschrieben in US 4,754,018. Die Isolierung und antibakterielle Wirksamkeit von Lysobactin ist auch in EP-A-196 042 und JP 01132600 beschrieben. WO04/099239 beschreibt Derivate des Lysobactins mit antibakterieller Wirksamkeit.

Die antibakterielle Wirkung von Lysobactin und Katanosin A wird weiterhin beschrieben in O'Sullivan, J. et al., J. Antibiot. 1988, 41, 1740 - 1744, Bonner, D. P. et al., J. Antibiot. 1988, 41, 1745 - 1751, Shoji, J. et al., J. Antibiot. 1988, 41, 713 - 718 und Tymiak, A. A. et al., J. Org. Chem. 1989, 54, 1149 - 1157.

Eine Aufgabe der vorliegenden Erfindung ist es, alternative Verbindungen mit vergleichbarer oder verbesserter antibakterieller Wirkung, besserer Löslichkeit und besserer Verträglichkeit zur Behandlung von bakteriellen Erkrankungen bei Menschen und Tieren zur Verfügung zu stellen.

Gegenstand der Erfindung sind Verbindungen der Formel 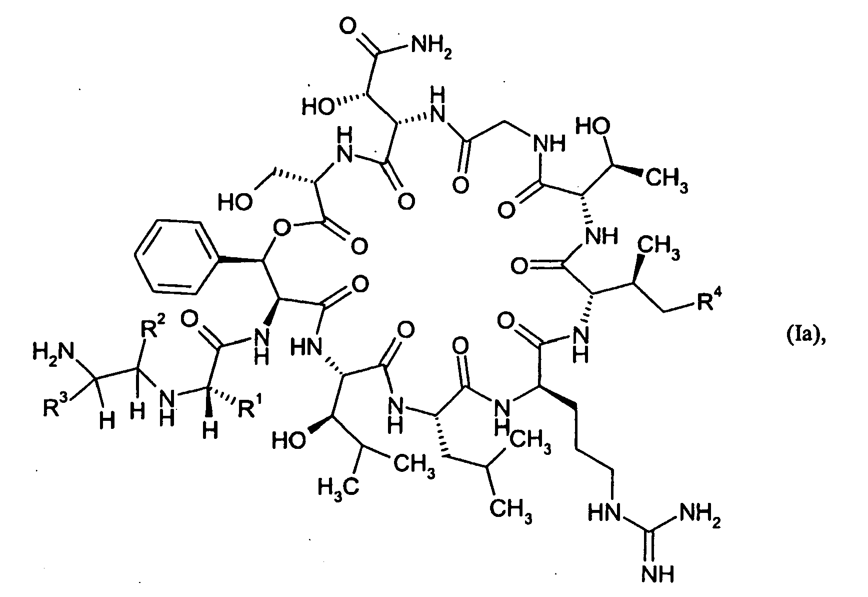 in welcher
- R¹: C₁-C₂-Alkyl bedeutet,
wobei C₁-C₂-Alkyl substituiert ist mit einem Substituenten ausgewählt aus der Gruppe bestehend aus Trimethylsilyl, Alkoxycarbonyl, C₃-C₆-Cycloalkyl, Alkoxy substituiertes Phenyl, 2-Pyridyl und 3-Pyridyl,
oder
C₄-C₈-Alkyl bedeutet,
- R²: Wasserstoff oder C₁-C₄-Alkyl bedeutet,
- R³: C₁-C₂-Alkyl bedeutet,
wobei C₁-C₂-Alkyl substituiert ist mit einem Substituenten Trimethylsilyl,
oder
C₄-C₆-Alkyl bedeutet,
- R⁴: Wasserstoff oder Methyl bedeutet,
und ihre Salze, ihre Solvate und die Solvate ihrer Salze.

Erfindungsgemäße Verbindungen sind die Verbindungen der Formel (I) und (Ia) und deren Salze, Solvate, Solvate der Salze und Prodrugs, die von Formel (I) und (Ia) umfassten Verbindungen der nachfolgend genannten Formeln und deren Salze, Solvate, Solvate der Salze und Prodrugs sowie die von Formel (I) und (Ia) umfassten, nachfolgend als Ausführungsbeispiele genannten Verbindungen und deren Salze, Solvate, Solvate der Salze und Prodrugs, soweit es sich bei den von Formel (I) und (Ia) umfassten, nachfolgend genannten Verbindungen nicht bereits um Salze, Solvate, Solvate der Salze und Prodrugs handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegenden Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche. Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldüsopropylamin, Monoethanolamin, Diethanolamin, Triethanolamin, Dicyclohexylamin, Dimethylaminoethanol, Prokain, Dibenzylamin, *N*-Methylmorpholin, Arginin, Lysin, Ethylendiamin und *N*-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:

Alkyl per se und "Alk" und "Alkyl" in Alkoxy und Alkoxycarbonyl steht für einen linearen oder verzweigten Alkylrest mit in der Regel 1 bis 6, vorzugsweise 1 bis 4, besonders bevorzugt 1 bis 3 Kohlenstoffatomen, beispielhaft und vorzugsweise für Methyl, Ethyl, n-Propyl, Isopropyl, *tert*-Butyl, 2,2-Dimethylprop-1-yl, n-Pentyl und n-Hexyl.

Alkoxy steht beispielhaft und vorzugsweise für Methoxy, Ethoxy, n-Propoxy, Isopropoxy, *tert-*Butoxy, n-Pentoxy und n-Hexoxy.

Alkoxycarbonyl steht beispielhaft und vorzugsweise für Methoxycarbonyl, Ethoxycarbonyl, n-Propoxycarbonyl, Isopropoxycarbonyl, *tert*-Butoxycarbonyl, n-Pentoxycarbonyl und n-Hexoxycarbonyl.

Cycloalkyl steht für eine Cycloalkylgruppe mit in der Regel 3 bis 6 Kohlenstoffatomen, beispielhaft und vorzugsweise für Cyclopropyl, Cyclobutyl, Cyclopentyl und Cyclohexyl.

In den Formeln der Gruppen steht der Endpunkt der Linie, neben der jeweils ein * steht, nicht für ein Kohlenstoffatom beziehungsweise eine CH₂-Gruppe, sondern ist Bestandteil der Bindung zu dem Stickstoffatom, an das die Gruppe der Formel gebunden ist.

Bevorzugt im Rahmen der vorliegenden Erfindung sind Verbindungen der Formel 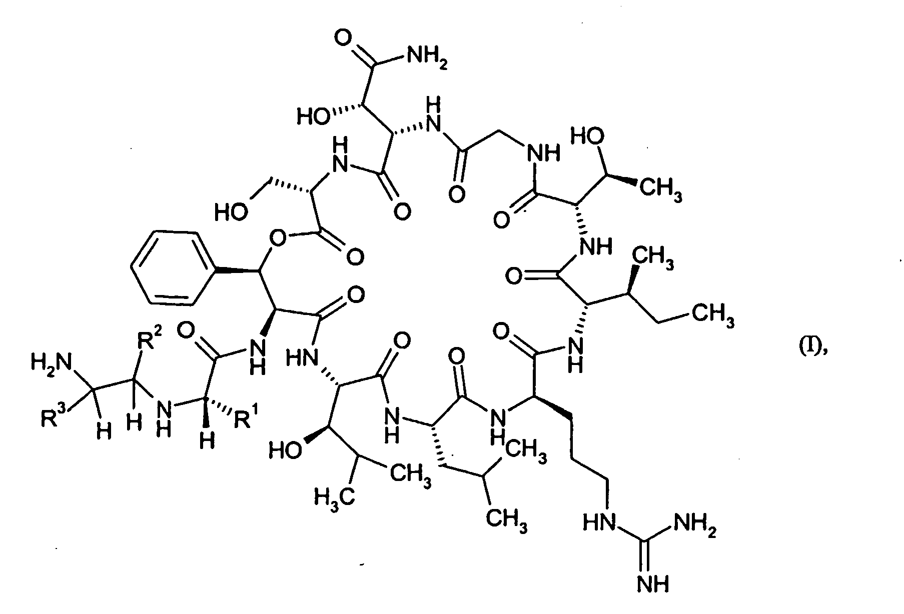 in welcher
- R¹: C₁-C₂-Alkyl bedeutet,
wobei C₁-C₂-Alkyl substituiert ist mit einem Substituenten ausgewählt aus der Gruppe bestehend aus Trimethylsilyl, Alkoxycarbonyl, C₃-C₆-Cycloalkyl, Alkoxy substituiertes Phenyl, 2-Pyridyl und 3-Pyridyl,
oder
C₄-C₈-Alkyl bedeutet,
- R²: Wasserstoff oder C₁-C₄-Alkyl bedeutet,
- R³: C₁-C₂-Alkyl bedeutet,
wobei C₁-C₂-Alkyl substituiert ist mit einem Substituenten Trimethylsilyl,
oder
C₄-C₆-Alkyl bedeutet,
und ihre Salze, ihre Solvate und die Solvate ihrer Salze.

Bevorzugt sind auch Verbindungen der Formel (I), in welcher
- R¹: C₁-C₂-Alkyl bedeutet
wobei C₁-C₂-Alkyl substituiert ist mit einem Substituenten Trimethylsilyl,
oder
C₄-C₆-Alkyl bedeutet,
- R²: Wasserstoff oder Methyl bedeutet,
- R³: C₁-C₂-Alkyl bedeutet,
wobei C₁-C₂-Alkyl substituiert ist mit einem Substituenten Trimethylsilyl,
oder
C₄-C₆-Alkyl bedeutet,
und ihre Salze, ihre Solvate und die Solvate ihrer Salze.

Besonders bevorzugt sind Verbindungen der Formel (I), in welcher
- R¹: Trimethylsilylmethyl oder 2,2-Dimethylprop-1-yl bedeutet,
- R²: Wasserstoff bedeutet,
- R³: Trimethylsilylmethyl oder 2,2-Dimethylprop-1-yl bedeutet,
und ihre Salze, ihre Solvate und die Solvate ihrer Salze.

Bevorzugt sind auch Verbindungen der Formel (I), in welcher R¹ 2,2-Dimethylprop-1-yl bedeutet.

Bevorzugt sind auch Verbindungen der Formel (I), in welcher R² Wasserstoff bedeutet.

Bevorzugt sind auch Verbindungen der Formel (I), in welcher R³ 2,2-Dimethylprop-1-yl bedeutet.

Die in den jeweiligen Kombinationen bzw. bevorzugten Kombinationen von Resten im einzelnen angegebenen Restedefinitionen werden unabhängig von den jeweiligen angegebenen Kombinationen der Reste beliebig auch durch Restedefinitionen anderer Kombination ersetzt.

Ganz besonders bevorzugt sind auch Kombinationen von zwei oder mehreren der oben genannten Vorzugsbereiche.

Gegenstand der Erfindung ist weiterhin ein Verfahren zur Herstellung der Verbindungen der Formeln (Ia), wobei die Verbindung der Formel 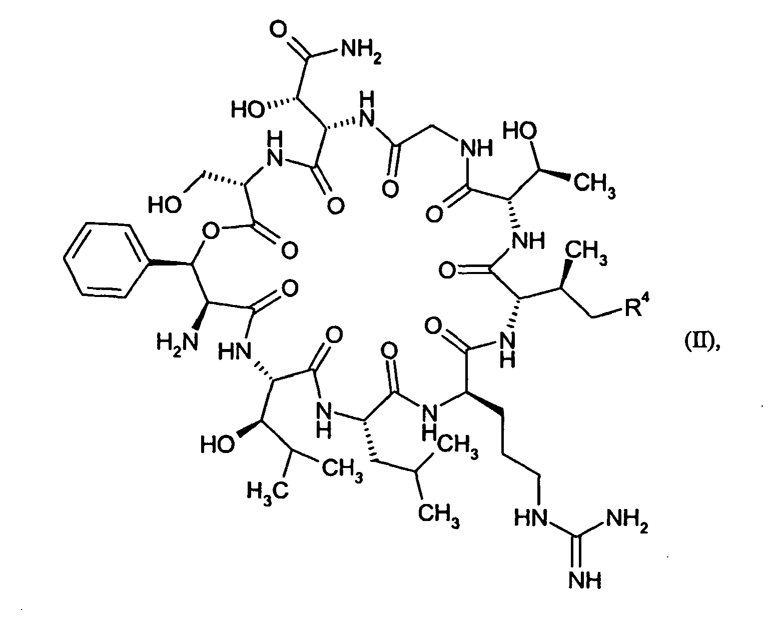 in welcher
- R⁴: die oben angegebene Bedeutung hat,
mit Verbindungen der Formel 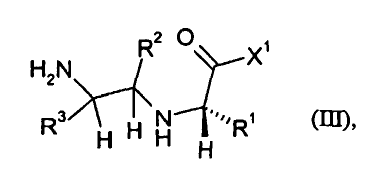 in welcher
- R¹, R² und R³: die oben angegebene Bedeutung haben, und
- X¹: Halogen, bevorzugt Brom, Chlor oder Fluor, oder Hydroxy bedeutet,
umgesetzt werden.

Falls X¹ für Halogen steht, erfolgt die Umsetzung im Allgemeinen in inerten Lösungsmitteln, gegebenenfalls in Gegenwart einer Base, bevorzugt in einem Temperaturbereich von -30°C bis 50°C bei Normaldruck.

Inerte Lösungsmittel sind beispielsweise Tetrahydrofuran, Methylenchlorid, Pyridin, Dioxan oder Dimethylformamid, bevorzugt ist Pyridin oder Dimethylformamid.

Als inerte Lösungsmittel sind Tetrahydrofuran oder Methylenchlorid bevorzugt.

Basen sind beispielsweise Triethylamin, Diisopropylethylamin oder N-Methylmorpholin, bevorzugt ist Diisopropylethylamin.

Falls X¹ für Hydroxy steht, erfolgt die Umsetzung im Allgemeinen in inerten Lösungsmitteln, in Gegenwart eines Dehydratisierungsreagenzes, gegebenenfalls in Gegenwart einer Base, bevorzugt in einem Temperaturbereich von -30°C bis 50°C bei Nonnaldruck.

Inerte Lösungsmittel sind beispielsweise Halogenkohlenwasserstoffe wie Dichlormethan oder Trichlormethan, Kohlenwasserstoff wie Benzol, Nitromethan, Dioxan, Dimethylformamid oder Acetonitril. Ebenso ist es möglich, Gemische der Lösemittel einzusetzen. Besonders bevorzugt ist Dichlormethan oder Dimethylformamid.

Als Dehydratisiemngsreagenzien eignen sich hierbei beispielsweise Carbodiimide wie z.B. *N,N'-*Diethyl-, *N*,*N*,'-Dipropyl-, *N*,*N*'-Diisopropyl-, *N*,*N'-*Dicyclohexylcarbodiimid, *N*-(3-Dimethylaminoisopropyl)-*N*'-ethylcarbodiimid-hydrochlorid (EDC), *N*-Cyclohexylcarbodiimid-*N*'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2*-tert-*Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)-phosphoniumhexafluorophosphat, oder *O*-(Benzotriazol-1-yl)-*N,N,N',N'*-tetra-methyluroniumhexafluorophosphat (HBTU), 2-(2-Oxo-1-(2H)-pyridyl)-1,1,3,3-tetramethyluroniumtetrafluoroborat (TPTU) oder *O*-(7-Azabenzotriazol-1-yl)-*N,N,N,N*'-tetramethyl-uroniumhexafluorophosphat (HATU), oder 1-Hydroxybenzotriazol (HOBt), oder Benzotriazol-1-yloxytris(dimethylamino)-phosphoniumhexafluorophosphat (BOP), oder N-Hydroxysuccinimid, oder Mischungen aus diesen, mit Basen.

Basen sind beispielsweise Alkalicarbonate, wie z.B. Natrium- oder Kaliumcarbonat, oder -hydrogencarbonat, oder organische Basen wie Trialkylamine z.B. Triethylamin, *N*-Methylmorpholin, *N*-Methylpiperidin, 4-Dimethylaminopyridin oder Diisopropylethylamin.

Vorzugsweise wird die Kondensation mit HATU oder mit EDC in Gegenwart von HOBt durchgeführt.

Die Verbindungen der Formel (III) tragen gegebenenfalls Schutzgruppen, so dass sich in diesen Fällen der Umsetzung der Verbindung der Formel (II) mit Verbindungen der Formel (III) eine Abspaltung der Schutzgruppen mit z.B. Trifluoressigsäure oder durch Hydrogenolyse nach dem Fachmann bekannten Methoden anschließt.

Die Verbindung der Formel (II) lässt sich durch doppelten Edman-Abbau aus Lysobactin (Beispiel 1A) synthetisieren, wie im experimentellen Teil unter Beispiel 2A bis 5A beschrieben.

Die Verbindungen der Formel (III) sind bekannt oder lassen sich nach bekannten Verfahren aus den entsprechenden Edukten synthetisieren, zum Beispiel nach folgendem Syntheseschema:

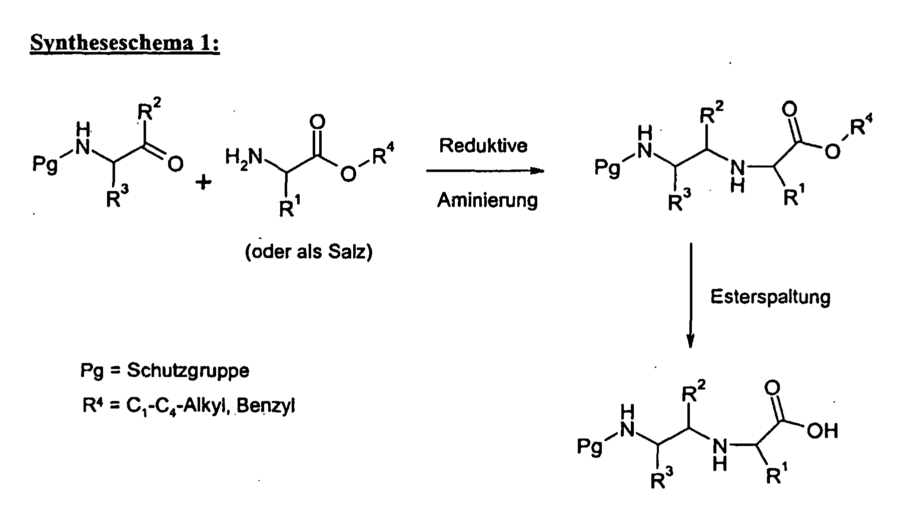

Die Herstellung der erfindungsgemäßen Verbindungen kann durch folgende Syntheseschemata verdeutlicht werden.

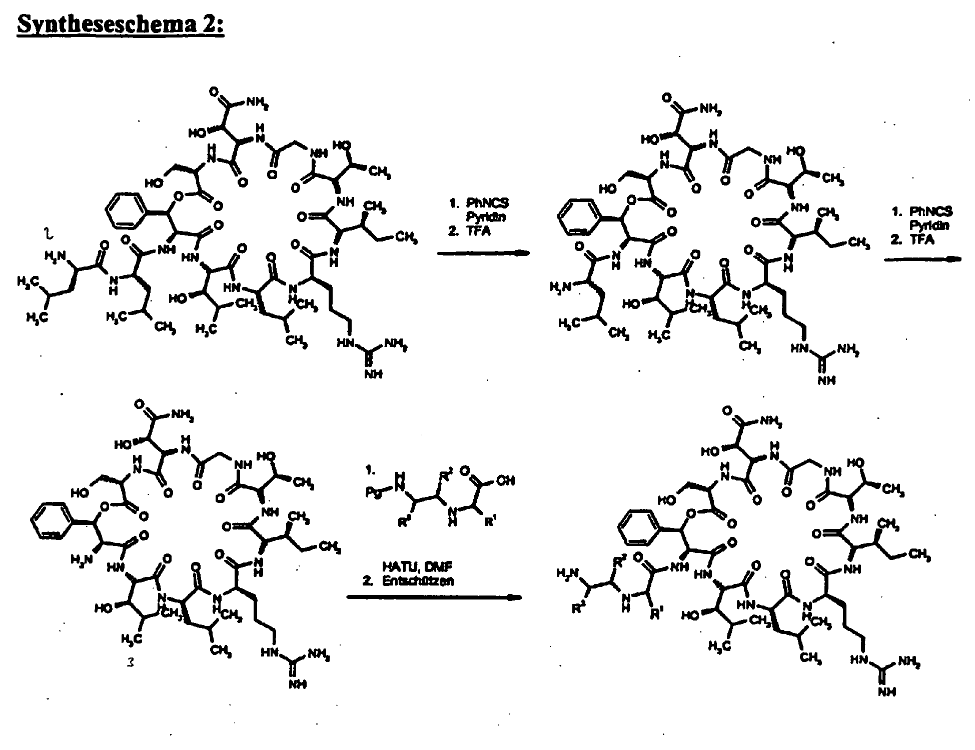

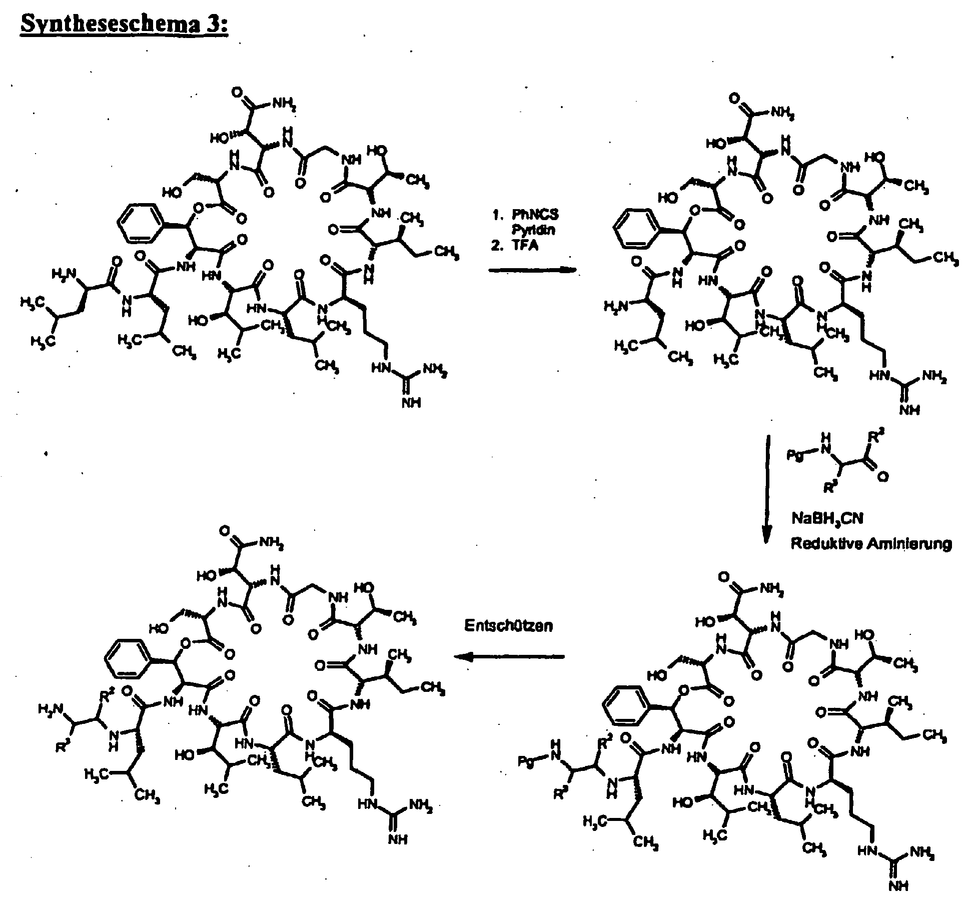

Die erfindungsgemäßen Verbindungen zeigen ein nicht vorhersehbares, wertvolles pharmakologisches Wirkspektrum. Sie zeigen eine antibakterielle Wirkung.

Sie eignen sich daher zur Verwendung als Arzneimittel zur Behandlung und/oder Prophylaxe von Krankheiten bei Menschen und Tieren.

Die erfindungsgemäßen Verbindungen zeichnen sich durch eine bessere Löslichkeit gegenüber Lysobactin aus.

Die beschriebenen Nonadepsipeptide wirken als Inhibitoren der bakteriellen Zellwandbiosynthese.

Besonders wirksam sind die erfindungsgemäßen Zubereitungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Grundsätzlich können die erfindungsgemäßen Zubereitungen gegen alle Bakterien und bakterienähnlichen Mikroorganismen verwendet werden, die im Besitz einer bakteriellen Zellwand (Murein sacculus) bzw. den dazugehörigen Enzymsystemen sind, beispielsweise durch die folgenden Erreger oder durch Mischungen der folgenden Erreger:

Gram-negative Kokken (*Neisseria gonorrhoeae*) sowie Gram-negative Stäbchen wie Enterobakteriaceen, z.B. *Escherichia coli, Hämophilus influenzae,* Pseudomonas, Klebsiella, Citrobacter (C. *freundii, C. divernis),* Salmonella und Shigella; ferner Enterobacter (*E. aerogenes, E. agglomerans*), Hafnia, Serratia (*S. marcescens*), Providencia, Yersinia, sowie die Gattung Acinetobacter, Branhamella und Chlamydia. Darüber hinaus umfasst das antibakterielle Spektrum strikt anaerobe Bakterien wie z.B. *Bacteroides fragilis,* Vertreter der Gattung Peptococcus, Peptostreptococcus sowie die Gattung Clostridium; ferner Mykobakterien, z.B. M. *tuberculosus.* Besonders ausgeprägte Wirkung zeigen die erfindungsgemäßen Verbindungen gegen Gram-positive Kokken, z.B. Staphylokokken (*S. aureus, S. epidermidis, S. haemolyticus, S. carnosus*), Enterokokken (*E. faecalis, E. faecium*) und Streptokokken (*S. agalactiae, S. pneumoniae, S. pyogenes*).

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Zubereitungen verhindert, gebessert oder geheilt werden können, seien beispielsweise genannt:

Infektionskrankheiten beim Menschen wie z.B. unkomplizierte und komplizierte Harnwegsinfekte, unkomplizierte Haut- und Oberflächeninfektionen, komplizierte Haut- und Weichteilinfektionen, im Hospital und ambulant erworbene Lungenentzündung, nosokomiale Pneumonien, akute Exazerbationen und sekundäre bakterielle Infektionen der chronischen Bronchitis, akute Otitis media, akute Sinusitis, streptokokkale Pharyngitis, bakterielle Meningitis, unkomplizierte gonokokkale und nicht-gonokokkale Urethritis/Cervizitis, akute Prostatitis, Endocarditis, unkomplizierte und komplizierte intra-abdominale Infektionen, gynäkologische Infektionen, pelvische Entzündungskrankheit, bakterielle Vaginose, akute und chronische Osteomyelitis, akute bakterielle Arthritis, empirische Therapie in febrilen neutropenischen Patienten, desweiteren Bakterämien, MRSA Infektionen, akute infektiöse Diarrhoe, *Helicobacter pylori* Infektionen, postoperative Infektionen, odontogene Infektionen, ophthalmologische Infektionen, postoperative Infektionen (inkl. periproktaler Abszess, Wundinfektionen, Galleninfektionen, Mastitis und akute Appendizitis), zystische Fibrose und Bronchiectasis.

Außer beim Menschen können bakterielle Infektionen auch bei anderen Spezies behandelt werden. Beispielhaft seien genannt:
Schwein: Diarrhoe, Enterotoxamie, Sepsis, Dysenterie, Salmonellose, Metritis-Mastitis-Agalaktiae-Syndrom, Mastitis;
Wiederkäuer (Rind, Schaf, Ziege): Diarrhoe, Sepsis, Bronchopneumonie, Salmonellose, Pasteurellose, Genitalinfektionen;
Pferd: Bronchopneumonien, Fohlenlähme, puerperale und postpuerperale Infektionen, Salmonellose;
Hund und Katze: Bronchopneumonie, Diarrhoe, Dermatitis, Otitis, Harnwegsinfekte, Prostatitis;

Geflügel (Huhn, Pute, Wachtel, Taube, Ziervögel und andere): E. coli-Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie z.B. Pasteurella, Brucella, Campylobacter, Listeria, Erysipelothris, Corynebakterien, Borellia, Treponema, Nocardia, Rikettsia, Yersinia, erweitert.

Weiterer Gegenstand der vorliegenden Erfindung ist der Einsatz der erfindungsgemäßen Verbindungen zur Behandlung und/oder Prophylaxe von Erkrankungen, insbesondere von bakteriellen Infektionskrankheiten.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Bevorzugt werden die erfindungsgemäßen Verbindungen zur Herstellung von Arzneimitteln verwendet, die zur Prophylaxe und/oder Behandlung von bakteriellen Erkrankungen geeignet sind.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer antibakteriell wirksamen Menge der erfindungsgemäßen Verbindungen.

Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen. Bevorzugte Kombinationswirkstoffe sind antibakteriell wirkende Verbindungen, die ein anderes Wirkspektrum, insbesondere ergänzendes Wirkspektrum, haben und/oder synergistisch zu den erfindungsgemäßen Verbindungen sind.

Die erfindungsgemäßen Verbindungen können systemisch und/oder lokal wirken. Zu diesem Zweck können sie auf geeignete Weise appliziert werden, wie z.B: oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführte Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich als vorteilhaft erwiesen, bei intravenöser Applikation Mengen von etwa 0.001 bis 100 mg/kg, vorzugsweise etwa 0.1 bis 10 mg/kg Körpergewicht zur Erzielung wirksamer Ergebnisse zu verabreichen, und bei oraler Applikation beträgt die Dosierung etwa 0.01 bis 50 mg/kg, vorzugsweise 0.5 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests sind, sofern nicht anders angegeben, Gewichtsprozente; Ausbeuten in den Beispielen sind Molprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen

- Allg.: Allgemein(e)
- Area: (Peak)fläche
- ber.: berechnet
- BHI: Brain Heart Infusion
- Boc: tert-Butyloxycarbonyl
- br.: breites Signal (bei NMR-Spektren)
- Bsp.: Beispiel
- d: Dublett (bei NMR-Spektren)
- DC: Dünnschichtchromatographie
- DCI: direkte chemische Ionisation (bei MS)
- DCM: Dichlormethan
- DIEA: *N,N*-Diisopropylethylamin
- DMSO: Dimethylsulfoxid
- DMF: *N,N-*Dimethylformamid
- d. Th.: der Theorie
- EDC: 1 -Ethyl-3-(3-dimethylaminopropyl)carbodiimide (auch EDCI)
- EDCxHCl: 1-Ethyl-3-(3-dimethylaminopropyl)carbodümide-hydrochlorid
- EE: Ethylacetat (Essigsäureethylester)
- EI: Elektronenstoß-Ionisation (bei MS)
- ESI: Elektrospray-Ionisation (bei MS)
- Fp.: Schmelzpunkt
- gef.: gefunden
- ges.: gesättigt
- h: Stunde
- HATU: *O*-(7-Azabenzotriazol-1-yl)-*N,N,N',N'*-tetramethyluronium-hexafluorphosphat
- HOBt: 1-Hydroxybenzotriazol
- HPLC: Hochdruck-, Hochleistungsflüssigchromatographie
- HR: High Resolution (Hochauflösung)
- i. V.: im Vakuum
- konz.: konzentriert
- LC-MS: Flüssigchromatographie-gekoppelte Massenspektroskopie
- LDA: Lithium-Diisopropylamid
- m: middle (mittel) (bei UV- und IR-Spektren)
- m: Multiplett (bei NMR-Spektren)
- MALDI: Matrix-Assisted Laser Desorption/Ionization
- MHK: Minimale Hemmkonzentration
- min: Minute/Minuten
- MRSA: Methicillin-resistenter *Staphylococcus aureus*
- MS: Massenspektroskopie
- NCCLS: National Committee for Clinical Laboratory Standards
- neg.: negativ
- NMM: *N*-Methylmorpholin
- NMR: Kernresonanzspektroskopie (nuclear magnetic resonance)
- p.a.: pro analysi
- Pd-C: Palladium auf Kohle
- pos.: positiv
- proz.: Prozentig
- quant.: quantitativ
- RP-HPLC: Reverse Phase HPLC
- RT: Raumtemperatur
- Rₜ: Retentionszeit (bei HPLC)
- s: strong (stark) (bei UV- und IR-Spektren)
- s: Singulett (bei NMR-Spektren)
- TBTU: O-(Benzotriazol-1-yl)-N,N,N;N'-tetramethyluronium-Tetrafluoroborat
- TCTU: O-(1 H-6-Chlorbenzotriazol-1-yl)-1,1,3,3-tetramethyluronium-Tetrafluoroborat
- TFA: Trifluoressigsäure
- TFE: 2,2,2-Trifluorethanol
- THF: Tetrahydrofuran
- TOF: Flugzeit (time of flight)
- UV: Ultraviolett
- Vis: sichtbar (visible)
- VRSA: Vancomycin-resistenter *Staphylococcus aureus*
- w: weak (schwach) (bei UV- und IR-Spektren)
- Z, Cbz: Benzyloxycarbonyl

### Literatur

Zur Nomenklatur der Peptide und Cyclodepsipeptide vergleiche:
1. A Guide to IUPAC Nomenclature of Organic Compounds (Recommendations 1993), 1993, Blackwell Scientific publications.
2. Nomenclature and symbolism for amino acids and peptides. Recommendations 1983. IUPAC-IUB Joint Commission on Biochemical Nomenclature, UK. Biochemical Journal 1984, 219, 345-373. Sowie zitierte Literatur.

### Allgemeine Methoden LC-MS, BR-MS, HPLC und Gelchromatographie

**Methode 1 (HPLC):** Gerät: HP1100 mit DAD (G1315A) und Autosampler (G1329A), Autosamplertherme (G1330A, 5°C), Degaser (G1322A) und Binäre Pumpe (G1312A); Vorsäule: Waters Symmetry C-18, 10 x 2.1 mm, 3.5 µm; Analytiksäule: Waters Symmetry C-18, 50 x 2.1 mm, 3.5 µm; Säulenofen: 45°C; Eluent A: Wasser/0.05% Trifluoressigsäure; Eluent B: Acetonitril/0.05% Trifluoressigsäure; Fluss: 0.4 ml/min.; Gradient 0-100% B in 9 min, hiernach 3 min bei 100% B, hiernach Regeneration der Säule.

**Methode 2 (LC-MS):** Gerät: Micromass LCT; Ionisation: ESI positiv/negativ; HP1100 mit DAD und Autosampler; Ofen 40°C; Säule: Waters Symmetry C-18, 50 x 2.1 mm, 3.5 µm; Eluent A: 0.1% Ameisensäure/Acetonitril, Eluent B: 0.1% Ameisensäure/Wasser; Fluss: 0.5 ml/min.; Gradient: 0-1 min 0% A, 1-6 min 90% A, 6-8 min 100% A, 8-10 min 100% A, 10-15 0% A.

**Methode 3 (HPLC):** Gerät: Gilson Abimed HPLC; UV Detektor 254 nm; binäres Pumpensystem; Säule: Nucleosil RP-18, 7 µm; 250 x 50 mm; Fluss: 30 ml/min; Eluent A: Wasser/0.1% Trifluoressigsäure, Eluent B: Acetonitril/0.1% Trifluoressigsäure; Gradient: 0-40 min 20-25% B, 40-60 min 25% B, 60-110 min 25-50% B, 110-120 min 50% B, 120-130 min 50-100% B, 130-160 min 100% B, anschließend Regeneration der Chromatographiesäule.

**Methode 4 (HPLC):** Gerät: Gilson Abimed HPLC; UV Detektor 254 nm; binäres Pumpensystem; Säule: Nucleosil RP-18, 7 µm; 250 x 50 mm; Fluss: 40 ml/min; Eluent A: Wasser/0.05% Trifluoressigsäure, Eluent B: Acetonitril/0.05% Trifluoressigsäure; Gradient: 0-105 min 20-25% B, 105-111 min 25% B, 111-131 min 25-27% B, 131-157 min 27-35% B, 157-192 min 35-40% B, 192-207 min 40-45% B, anschließend Regeneration der Chromatographiesäule.

**Methode 5 (HPLC):** Gerät: Gilson Abimed HPLC; UV Detektor 254 nm; binäres Pumpensystem; Säule: Nucleosil RP-18, 7 µm; 250 x 50 mm; Fluss: 40 ml/min; Eluent A: Wasser/0.05% Trifluoressigsäure, Eluent B: Acetonitril/0.05% Trifluoressigsäure; Gradient: 0-40 min 20-25% B, 40-105 min 25% B, 105-130 min 25-27% B, 130-170 min 27-40% B, 170-190 min 40% B, 190-210 min 40-45% B, anschließend Regeneration der Chromatographiesäule.

**Methode 6 (Gelchromatographie an Sephadex LH-20):** Gelchromatographie wird ohne Druck an Sephadex LH-20 (Firma Pharmacia) durchgeführt. Es wird nach UV-Aktivität (UV Detektor für 254 nm, Firma Knauer) fraktioniert (Fraktionssammler ISCO Foxy 200). Säulendimensionen: 32 x 7 cm (1000-100 µmol Maßstab); 30 x 4 cm (100-10 µmol Maßstab); 25 x 2 cm (10-1 µmol Maßstab).

**Methode 7 (präparative HPLC; Symmetry):** Gerät: Gilson Abimed HPLC; UV Detektor 210 nm; binäres Pumpensystem; Säule: SymmetryPrep^{™}C₁₈, Firma Waters, 7 µm; 300 x 19 mm; Fluss: 20 ml/min; Eluent A: 0.05% Trifluoroessigsäure in Wasser, Eluent B: 0.05% Trifluoroessigsäure in Acetonitril; Gradient: 0-3 min 10% B, 3-30 min Gradientrampe 10-90% B, 30-38 min 90% B, 38-45 min 10% B.

**Methode 8 (präparative HPLC; Symmetry; TFA):** Gerät: Gilson Abimed HPLC; UV Detektor 210 nm; binäres Pumpensystem; Säule: SymmetryPrep^{™}C₁₈, Firma Waters, 7 µm; 300 x 19 mm; Fluss: 7 ml/min; Eluent A: Wasser/0.1-0.25% Trifluoressigsäure, Eluent B: Acetonitril; Gradient: 0-8 min 5% B, 8-40 min 5-60% B, 40-60 min 60% B, 60-75 min 60-100% B, 75-80 min 100% B, anschließend Regeneration der Chromatographiesäule.

**Methode 9 (präparative HPLC; Kromasil, Essigsäure):** Gerät: Gilson Abimed HPLC; UV Detektor 210 nm; binäres Pumpensystem; Säule: Kromasil-100A C₁₈, 5 µm; 250 x 20 mm; Fluss: 25 ml/min; Eluent A: Wasser/0.25-0.5% Essigsäure, Eluent B: Acetonitril; Gradient: 0-3 min 5% B, 3-30 min 5-100% B, 30-38 min 100% B, anschließend Regeneration der Chromatographiesäule.

**Methode 10 (präparative HPLC; Symmetry; sensitive für Endprodukte):** Gerät: Gilson Abimed HPLC; UV Detektor 210 nm; binäres Pumpensystem; Säule: SymmetryPrep^{™}C₁₈, Firma Waters, 7 µm; 300 x 19 mm; Eluent A: 0.05% Trifluoressigsäure in Wasser, Eluent B: 0.05% Trifluoroessigsäure in Acetonitril; Gradient: 0-5 min 5% B mit Flussrate 20 ml/min, 5-30 min Gradientrampe von 5 bis 60% B mit folgender Flussratenerhöhungen: 22 ml/min ab 6 min, 23 ml/min ab 10 min, 24 ml/min ab 15 min; 30-35 min Gradientrampe von 60% bis 98% B mit Flussrateverminderung zu 21 ml/min ab 38 min; 40-45 min 10% B.

**Methode 11 (LC-MS):** Gerätetyp MS: Micromass ZQ; Gerätetyp HPLC: HP 1100 Series; UV DAD; Säule: Grom-Sil 120 ODS-4 HE 50 x 2 mm, 3.0 µm; Eluent A: Wasser/0.025% Ameisensäure/l, Eluent B: Acetonitril/0.025% Ameisensäure; Gradient: 0-2.9 min 0-70% B, 2.9-3.1 min 70-90% B, 3.1-4.5 min 70-90% B; Ofen: 50°C, Fluss: 0.8 ml/min, UV-Detektion: 210 nm.

**Methode 12 (LC-MS):** Gerätetyp MS: Micromass LCT (ESI pos./neg.); Gerätetyp HPLC: HP 1100 Series; UV DAD 1100 Series; Säule SymmetryPre^{™}C₁₈, Firma Waters, 50 x 2.1 mm, 3.5 µm; Eluent A: Wasser/0.1% Ameisensäure, Eluent B: Acetonitril/0.1% Ameisensäure; Gradient: 0-1 min 0% B, 1-5.5 min 0-95% B, 5.5-8 min 95% B, 8-8.1 min 95-0% B, 8.1-10 min 0% B, anschließend Regeneration der Chromatographiesäule. Ofen: 40°C, Fluss: 0.5 ml/min (bei 8.1-10 min kurzfristig auf 1 ml/min), UV-Detektion: 210 nm.

**Methode 13 (HPLC):** Gerätetyp HPLC: HP 1050 Series; UV DAD 1100 Series; Säule Symmetry-Prep^{™}C₁₈, Firma Waters, 50 x 2.1 mm, 3.5 µm; Eluent A: Wasser/0.05% Trifluoressigsäure, Eluent B: Acetonitril; Gradient: 0-9 min 0-100% B, 9-11 min 100% B, 11-12 min 100-0% B, anschließend Regeneration der Chromatographiesäule. Ofen: 40°C, Fluss: 0.4 ml/min, UV-Detektion: 210 nm.

**Methode 14 (LC-MS):** Gerätetyp MS: Micromass ZQ; Gerätetyp HPLC: Waters Alliance 2795; Säule: Phenomenex Synergi 2 µm Hydro-RP Mercury 20 x 4 mm; Eluent A: Wasser/0.25% Ameisensäure, Eluent B: Acetonitril/0.25% Ameisensäure; Gradient: 0.0-2.5 min, 90-30% A, Fluss 1-2 ml/min, 2.5-3.0 min, 30-5% A, Fluss 2.0 min, 3.0-4.5 min, 5% A; Ofen: 50°C; UV-Detektion: 210 nm.

**Methode 15 (analytische HPLC):** Gerätetyp HPLC: HP 1050 Series; UV DAD 1100 Series; Säule: Kromasil C₁₈, 60 x 2 mm, 3.5 µm; Eluent A: Wasser/0.5% Perchlorsäure, Eluent B: Acetonitril; Gradient: 0-0.5 min 2% B, 0.5-4.5 min 2-90% B, 4.5-9.0 min 90% B, 9.0-9.2 min 90-2% B, 9.2-10.0 min 2% B; Fluss: 0.75 ml/min, Ofen: 30°C, W-Detektion 210 nm.

**Methode 16 (LC-MS):** Instrument: Micromass Quattro LCZ mit HPLC Agilent Serie 1100; Säule: Phenomenex Synergi 2 µ Hydro-RP Mercury 20 mm x 4 mm; Eluent A: 11 Wasser + 0.5 ml 50%ige Ameisensäure, Eluent B: 11 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 208-400 nm.

**Methode 17 (LC-MS):** Gerätetyp MS: Micromass ZQ; Gerätetyp HPLC: HP 1100 Series; UV DAD; Säule: Phenomenex Synergi 2 µ Hydro-RP Mercury 20 mm x 4 mm; Eluent A: 11 Wasser + 0.5 ml 50%ige Ameisensäure, Eluent B: 11 Acetonitril + 0.5 ml 50%ige Ameisensäure; Gradient: 0.0 min 90%A -> 2.5 min 30%A -> 3.0 min 5%A → 4.5 min 5%A; Fluss: 0.0 min 1 ml/min, 2.5 min/3.0 min/4.5 min. 2 ml/min; Ofen: 50°C; UV-Detektion: 210 nm.

Methode 18 (LC-MS): Instrument: Micromass Quattro LCZ mit HPLC Agilent Serie 1100; Säule: Phenomenex Synergi 2 µm Hydro-RP Mercury 20 mm x 4 mm; Eluent A: 11 Wasser +0.5 ml 50%ige Ameisensäure, Eluent B: 11 Acetonitril + 0.5 ml 50% Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 208-400 nm.

**Methode 19 (analytische HPLC):** Gerät: Agilent 1100 mit DAD (G1315B), binärer Pumpe (G1312A), Autosampler (G1313A), Lösemittel-Entgaser (G1379A) und Säulenthermostat (G1316A); Säule: Agilent Zorbax Eclipse XDB-C8 4.6 x 150 x 5 mm; Säulentemperatur: 30°C; Eluent A: 0.05% 70% Perchlorsäure in Wasser; Eluent B: Acetonitril; Fluss: 2.00 ml/min; Gradient: 0-1 min 10% B, Rampe, 4-5 min 90% B, Rampe, 5.5 min 10% B.

**Methode 20 (MALDI-MS):** Die *MALDI-MSIMS-Untersuchungen* werden auf einem 4700 Proteomics Analyzer (Applied Biosystems, Framingham, MA, USA) durchgeführt, der mit TOF/TOF Ionenoptik und 200 Hz Nd:YAG Laser (355 nm) ausgestattet ist. Die Quasimolekülionen werden in der Ionenquelle mit 8 kV beschleunigt, mit einem elektrischen Deflektor selektiert (MS1), und in einer Stoßzelle, die zwischen MS1 und MS2 angeordnet ist, mit Argonatomen gestoßen. Die entstehenden Fragmentionen werden mit 15 kV nachbeschleunigt und mit dem zweiten Flugzeit-Massenanalysator (MS2) charakterisiert.

**Methode 21 (TOF-HR-MS):** *TOF-HR-MS-ESI+* Spektren werden mit einem Micromass LCT Gerät (Kapillarspannung: 3.2 KV, Cone-Spannung: 42 V, Quellentemperatur: 120°C, Desolvations-Temperatur: 280°C) aufgenommen. Hierzu wird eine Spritzenpumpe (Firma Harvard Apparatus) für die Probenzufiihrung verwendet. Als Standart dient Leucin-Enkephalin (Tyr-Gly-Gly-Phe-Leu).

**Methode 22 (präparative HPLC):** Gerät: Gilson Abimed HPLC; UV-Detektor 210 nm; binäres Pumpensystem; Säule: Reprosil ODS-A, 5 µm, 250 x 20 mm; Eluent A: 0.2% Trifluoressigsδure in Wasser, Eluent B: Acetonitril; Flussrate: 25 ml/min; Säulentemperatur 40°C; 0-10 min 20% B, 10-15 min 80% B.

**Methode 23 (FT-ICR-HR-MS):** Die Massenpräzisionsmessungen werden an einem hochauflösenden Apex II Fourier-Transform Ionen-Cyclotron-Resonanz Massenspektrometer (Bruker Daltonik GmbH, Bremen) durchgeführt, das mit einem 7 Tesla Magneten, einer externen Elektrospray-Ionenquelle und einem Unix-basierten XMASS-Datensystem ausgestattet ist. Die Massenauflösung beträgt ca. 40.000 (50%-Tal-Definition).

**Methode 24 (LC-MS):** Instrument: Micromass Platform LCZ mit HPLC Agilent Serie 1100; Säule: Phenomenex Synergi 2 µ Hydro-RP Mercury 20 mm x 4 mm; Eluent A:11 Wasser + 0.5 ml 50%ige Ameisensäure, Eluent B: 11 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.

**Methode 25 (präparative HPLC)** Gerät: Gilson Abimed HPLC; UV-Detektor 210 nm; binäres Pumpensystem; Säule: Grom-Sil SNr.4051 120 ODS-4HE, 10 µm, 250 x 40 mm; Eluent A: 0.1 % Trifluoressigsäure in Wasser, Eluent B: 0.1% Trifluoressigsäure in Acetonitril; Flussrate: 50 ml/min; 0-3 min 10% B, 3-27 min Gradient Rampe, 27-35min 95% B, 35-40 min 10% B.

### Allgemeine Arbeitsvorschriften

### Allgemeine Arbeitsvorschrift 1 (Edman^{0,5 und 1.5})

Zu einer Lösung des N-terminal freien Peptids (0.3 mmol) in trockenem Pyridin (30 ml) wird unter Argonschutzgasatmosphäre Phenylisothiocyanat (50 mmol) getropft. Das Reaktionsgemisch wird bei 37°C solange (ca. 1 h) gerührt bis die analytische HPLC-Kontrolle (Methode 13) ausreichenden Umsatz anzeigt (>95%). Das Reaktionsgemisch wird unter Temperaturkontrolle (<40°C) im Vakuum eingeengt und dann lyophilisiert.

### Allgemeine Arbeitsvorschrift 2 (Edman^{1,0 und 2,0})

Unter Argonschutzgasatmosphäre wird der Peptid-Thioharnstoff (0.2 mmol) als Feststoff bei starkem Rühren mit trockener Trifluoressigsäure versetzt und dann bei 40°C solange gerührt (ca. 20 min) bis die analytische HPLC-Kontrolle ausreichenden Umsatz (>95%) anzeigt. Das Reaktionsgemisch wird zügig bei Raumtemperatur (Temperaturkontrolle) im Vakuum eingeengt. Um das Rohprodukt von weiterer Trifluoressigsäure zu befreien, wird das Rohprodukt in Dichlormethan aufgenommen und erneut im Vakuum von Lösungsmittel befreit. Dieser Vorgang wird mehrfach mit Toluol (zweimal) und mit Dichlormethan (zweimal) wiederholt. Abschließend wird das Rohprodukt lyophilisiert.

### Allgemeine Arbeitsvorschrift 3 (Reduktive Aminierung)

### (Herstellung der Desoxo(dipeptid)-ester Beispiele 25A bis 37A und der Beispiele 51A bis 54A)

Der N-geschützte Aminoaldehyd (1.2 -1.3 Äq.), das Amin (Aminoester oder Beispiel 3A, als Hydrochlorid oder TFA-Salz) (1 Äq.) und Natriumacetat (1 Äq.) werden in wasserfreiem DMF vorgelegt und bei 0°C mit Natriumcyanoborhydrid (2 Äq.) versetzt. Die resultierende Lösung wird bis zur vollständigen Umsetzung des Amins von 0°C bis RT gerührt (Umsetzung über analytische HPLC (Methode 19) oder LC-MS (Methode 17) bestimmt).

Danach wird bei kleinen Ansätzen (bis ca. 0.7 mmol) die komplette Reaktionsmischung durch HPLC gereinigt (Methode 7). Bei größeren Ansätzen wird zuerst über eine Sephadex-Säule (Methode 6) vorgereinigt, und das erhaltene Produkt nochmals über eine präparative HPLC (Methode 7) nachgereinigt. Das Produkt wird durch Lyophilisierung der geeigneten Fraktionen isoliert.

Pyridyl-haltige Verbindungen ergeben nach diesem Verfahren oft einen Pyridyl-Cyanoborankomplex. In diesem Fall wird dieser in Ethanol gelöst und 24 h auf Rückfluss erhitzt. Es entsteht der freie Desoxodipeptidsäureester. Dieser wird mit wenig Dimethylsulfoxid versetzt, von Ethanol am Rotavapor befreit und durch HPLC (Methode 7) gereinigt.

### Allgemeine Arbeitsvorschrift 4 (Desoxo(dipeptid)säure aus dem Methylester)

### (Herstellung der Beispiele 38A bis 49A)

Der Desoxo(dipeptid)methylester wird in Methanol gelöst und mit einem Überschuß Lithiumhydroxid (5-10 Äq., Lösung 1N in Wasser) bei RT verrührt bis die analytische HPLC (Methode 19) vollständige Verseifung zeigt. Danach wird mit 1N Salzsäure angesäuert, am Rotavapor eingeengt und durch präparative HPLC gereinigt. Das Produkt wird durch Lyophilisierung der geeigneten Fraktionen isoliert.

**Allgemeine Arbeitsvorschrift 5** (Hydrogenolytische Freisetzung der Desoxo(dipeptid)säure aus dem Benzylester)

### (Herstellung von Beispiel 50A)

Der Desoxo(dipeptid)benzylester wird in Ethanol (10-15 ml / mmol) gelöst und in Gegenwart von Pd (10% auf Kohle) als Katalysator (40-50 mg / mmol) unter 1 atm. Wasserstoff hydriert. Nach vollständiger Reaktion (nachgewiesen durch HPLC (Methode 19)) wird der Katalysator abfiltriert und mit Methanol gewaschen, dann das Filtrat einrotiert. Der Rückstand wird im Hochvakuum getrocknet.

### Allgemeine Arbeitsvorschrift 6 (Kondensation der Desoxo(dipeptid)säure mit Ed2.0)

### (Herstellung der Beispiele 55A bis 67A)

Die Desoxo(dipeptid)säure (2 Äq.) und die Verbindung aus Beispiel 5A (1 Äq.) werden unter Argon und Eiskühlung in DMF vorgelegt, mit NMM (4 Äq.), HATU (2.1 Äq.) und nochmals NMM (5 Äq.) versetzt und bei 0°C bis zur vollständigen Umsetzung gerührt (Umsetzung über analytische HPLC (Methode 19) oder LC-MS (Methode 17) bestimmt). Danach wird bei kleinen Ansätzen (bis ca. 0.2 mmol) die komplette Reaktionsmischung durch HPLC gereinigt (Methode 7). Bei größeren Ansätzen wird zuerst über eine Sephadex-Säule (Methode 6) vorgereinigt, und das erhaltene Produkt nochmals über eine präparative HPLC (Methode 7) nachgereinigt. Das Produkt wird durch Lyophilisierung der geeigneten Fraktionen isoliert.

### Allgemeine Arbeitsvorschrift 7 (Abspaltung der tert-Butoxycarbonyl-Schutzgruppe)

### (Herstellung der Beispiele 1-6)

Der Boc-geschützte Edukt wird in Dichlomethan / TFA 3:1 gelöst (20-200 ml / mmol) und bei RT bis zu vollständiger Umsetzung (5-20 min., Kontrolle durch HPLC (Methode 19)) rühren lassen. Anschliessend wird die Mischung mit 1,2-Dichlorethan verdünnt und am Rotavapor eingeengt. Der Rückstand wird in Wasser / Acetonitril aufgenommen, lyophylisiert, dann wieder in Wasser gelöst und durch präparative HPLC (Methode 10) gereinigt. Das Endprodukt wird durch Lyophilisierung der geeigneten Fraktionen erhalten.

### Allgemeine Arbeitsvorschrift 8 (Abspaltung der Benzyloxycarbonyl-Schutzgruppe)

### (Herstellung der Beispiele 7-17)

Der Z-geschützte Edukt wird in Isopropanol (15-100 ml / mmol) gelöst und in Gegenwart von Pd (10% auf Kohle) als Katalysator (50-200 mg / mmol) unter 1 atm. Wasserstoff hydriert. Nach vollständiger Reaktion (nachgewiesen durch HPLC (Methode 19)) wird der Katalysator abfiltriert und mit Methanol gewaschen, dann das Filtrat einrotiert. Der Rückstand wird in Wasser gelöst und über präparative HPLC (Methode 10) gereinigt. Das Endprodukt wird durch Lyophilisierung der geeigneten Fraktionen erhalten.

### Allgemeine Arbeitsvorschrift 9 (Hydrolytische Probenvorbereitung für MALDI-MS)

Das zu öffnende Depsipeptid (z.B. Lysobactin, 0.05 µMol) wird in einem Microvial zunächst mit einem Borat-Salzsäure Puffer (Fa. Merck) pH 8 (250 µl) versetzt. Man lässt über Nacht stehen, versetzt mit Essigsäure (100 µl) und gefriertrocknet die Probe. Das Rohprodukt wird ohne weitere Reinigungsschritte mittels MALDI-MS-Sequenzierung untersucht.

### Ausgangsverbindungen

### Beispiel 1A

### D-Leucyl-N¹- {(3S,6S,12S,15S,18R,21S,24S,27S,28R)-6-[(1S)-2-amino-1-hydroxy-2-oxoethyl]-18-(3-{[amino(imino)methyl]amino}propyl)-12-[(1S)-1-hydroxyethyl]-3-(hydroxymethyl)-24-[(1R)-1-hydroxy-2-methylpropyl]-21-isobutyl-15-[(1S)-1-methylpropyl]-2,5,8,11,14,17,20,23,26-nonaoxo-28-phenyl-1-oxa-4, 7,10,13,16,19,22,25-octaazacyclooctacosan-27-yl}-L-leucinamid-bistrifluoracetat (Lysobactin)

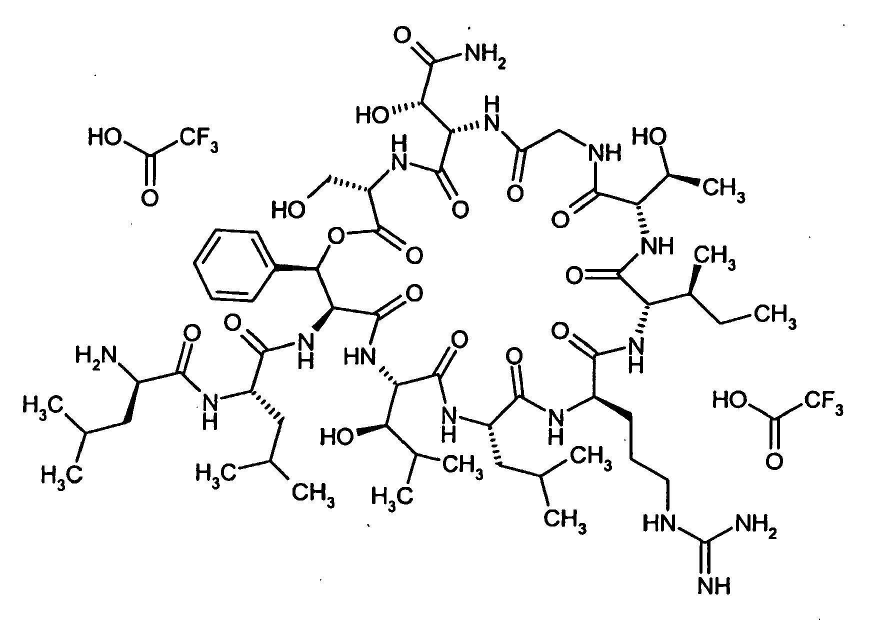

### Fermentation:

Kultur Medium:
YM: Hefe-Malz Agar: D-Glucose (4 g/l), Hefe Extrakt (4 g/l), Malz Extrakt (10 g/l), 1 Liter Lewatit Wasser. Vor dem Sterilisieren (20 Minuten bei 121°C) wird der pH auf 7.2 eingestellt.
HPM: Mannit (5.4 g/l), Hefe Extrakt (5 g/l), Fleischpepton (3 g/l).
Arbeitskonserve: Der lyophilisierte Stamm (ATCC 53042) wird in 50 ml YM Medium angezogen.
Kolbenfermentation: 150 ml YM Medium oder 100 ml HPM Medium in einem 11 Erlenmeyerkolben werden mit 2 ml der Arbeitskonserve beimpft und für 30-48 Stunden bei 28°C auf einem Schüttler bei 240 Upm wachsen gelassen.
30 1 Fermentation: 300 ml der Kolbenfermentation (HPM Medium) werden zum Animpfen einer sterilen 30 l Nährmedienlösung verwandt (1 ml Antifoam SAG 5693/1). Diese Kultur wird für 21 Stunden bei 28°C, 300 Upm und einer Belüftung mit steriler Luft von 0.3 vvm wachsen gelassen. Der pH wird mit 1 M Salzsäure auf pH = 7.2 konstant gehalten. Insgesamt werden während der Kultivierungszeit 880 ml 1 M Salzsäure zugeführt.
Hauptkultur (200 1): 15 x 150 ml YM Medium in 1 1 Erlenmeyerkolben werden mit 2 ml der Arbeitskonserve beimpft und bei 28°C für 48 Stunden und 240 Upm auf dem Schüttler wachsen gelassen. 2250 ml dieser Kultur werden zum Beimpfen einer sterilen 200 l Nährmedienlösung (YM) verwandt (1ml Antifoam SAG 5693/l) und für 18.5 Stunden bei 28°C, 150 Upm und einer Belüftung mit steriler Luft von 0.3 vvm wachsen gelassen.

Zur Kontrolle des Fermentationsverlaufs werden stündlich Proben (50 ml) entnommen. 2 ml dieser Kulturbrühe werden mit 1 ml Methanol (0.5% Trifluoressigsäure) versetzt und über einen 0.45 µm Filter filtriert. 30 µl dieser Suspension werden mittels HPLC analysiert (Methode 1 und Methode 2).

Nach 18.5 Stunden wird die Kulturbrühe der Hauptkultur bei 17000 Upm in Überstand und Sediment getrennt.

### Isolierung:

Der Überstand (183 1) wird mit konzentrierter Trifluoressigsäure bzw. Natronlauge auf pH 6.5-7 eingestellt und auf eine Lewapolsäule (OC 1064, 60 1 Inhalt) aufgetragen. Anschließend wird mit reinem Wasser, Wasser/Methanol 1:1 und anschließend mit reinem Methanol (mit 0.1% Trifluoressigsäure) eluiert. Diese organische Phase wird im Vakuum bis zu einem verbleibenden wässrigen Rest von 11.5 l eingeengt.

Die verbleibende wässrige Phase wird an Kieselgel C₁₈ gebunden und aufgetrennt (MPLC, Biotage Flash 75, 75 x 30 cm, KP-C18-WP, 15-20 µm, Fluss: 30 ml; Eluent: Acetonitril/ Wasser mit 0.1% Trifluoressigsäure; Gradient: 10%, 15% and 40% Acetonitril). Die 40% Acetonitril Phase, die die Hauptmenge von Beispiel 1 A enthält, wird im Vakuum eingeengt und anschließend lyophilisiert (ca. 13 g). Dieses Feststoffgemisch wird in 1.2 g Portionen zunächst an einer präparativen HPLC (Methode 3), anschließend durch Gelfiltration an Sephadex LH-20 (5 x 70 cm, Acetonitril/Wasser 1:1, jeweils mit 0.05% Trifluoressigsäure) und einer weiteren präparativen HPLC (Methode 4) getrennt.

Dieser Prozess liefert 2250 mg Beispiel 1A.

Das Sediment wird in 4 1 Aceton/Wasser 4:1 aufgenommen, mit 2 kg Celite versetzt, mit Trifluoressigsäure auf pH = 6 eingestellt, ausgerührt und zentrifugiert. Das Lösungsmittel wird im Vakuum eingeengt und der Rückstand gefriergetrocknet. Das erhaltene Lyophilisat (89.9 g) wird in Methanol aufgenommen, abfiltriert, eingeengt und an Kieselgel (Methode 5) getrennt. Beispiel 1A wird danach per Gelfiltration (Sephadex LH-20, 5 x 68 cm, Wasser/Acetonitril 9:1 (mit 0.05% Trifluoressigsäure), Fluss: 2.7 ml/min, Fraktionsgröße 13.5 ml) zur Reinsubstanz aufgereinigt.

Dieser Prozess liefert 447 mg Beispiel 1A.
HPLC (Methode 1): Rₜ = 6.19 min
MS (ESIpos): m/z = 1277 (M+H)⁺
¹H NMR (500.13 MHz, *d₆*-DMSO): δ = 0.75 (d, 3H), 0.78 (d, 6H), 0.80 (t, 3H), 0.82 (d, 3H), 0.90 (d, 3H), 0.91 (d, 3H), 0.92 (d, 3H), 0.95 (d, 3H), 0.96 (d, 3H), 1.05 (m, 1H), 1.19 (d, 3H), 1.25 (m, 2H), 1.50 (m, 4H), 1.51 (m, 2H), 1.55 (m, 1 H), 1.61 (m, 1H), 1.65 (m, 1H), 1.84 (m, 1H), 1.85 (m, 1H), 1.86 (m, 1H), 1.89 (m, 1H), 1.95 (m, 1H), 2.75 (m, 2H), 3.40 (m, 1H), 3.52 (m, 2H), 3.53 (dd, 1H), 3.64 (m, 2H), 3.66 (m, 1H), 3.68 (dd, 1H), 3.73 (m, 2H), 4.00 (dd, 1H), 4.02 (br., 1H), 4.13 (br., 1H), 4.32 (dd, 1H), 4.39 (t, 1H), 4.55 (m, 1H), 4.75 (dd, 1H), 5.19 (t, 1H), 5.29 (d, 1H), 5.30 (br., 1H), 5.58 (m, 2H), 6.68 (m, 3H), 6.89 (d, 1H), 6.93 (m, 3H), 6.94 (br., 1H), 6.98 (d, 1H), 7.12 (br., 1H), 7.20 (br., 2H), 7.23 (m, 2H), 7.42 (m, 2H), 7.54 (d, 1H), 7.5 (d, 1H), 8.32 (br., 1H), 9.18 (br., 1H), 9.20 (m, 2H), 9.50 (br., 1H).
¹³C-NMR (125.77 MHz, *d₆*-DMSO): δ = 10.3, 15.3, 19.0, 19.2, 19.6, 20.0, 20.9, 22.0, 22.4, 23.0, 23.2, 24.3, 24.4, 25.0, 25.4, 26.0, 27.8, 30.9, 35.4, 39.5, 40.8, 40.9, 41.6, 44.1, 51.5, 52.7, 55.9, 56.2, 56.4, 57.9, 58.8, 60.2, 61.1, 62.6, 70.1, 71.6, 71.7, 75.5, 128.1, 128.6, 136.7, 156.8, 168.2, 170.1, 170.4, 171.2, 171.5, 171.9, 172.2, 172.4, 173.7.

Die Zuordnung der Signale erfolgte nach der in der Literatur beschriebenen Zuordnung (T. Kato, H. Hinoo, Y. Terui, *J. Antibiot.,* **1988**, *61*, 719-725).

### Beispiel 2A

### N-(Anilinocarbonothioyl)-D-leucyl-N¹-{(3S,6S,12S,15S,18R,21S,24S,27S,28R)-6-[(1S)-2-amino-1-hydroxy-2-oxoethyl]-18-(3-{[amino(imino)methyl]amino)propyl)-12-[(1S)-1-hydroxyethyl]-3-(hydroxymethyl)-24-[(1R)-1-hydroxy-2-methylpropyl]-21-isobutyl-15-[(1S)-1-methylpropyl]-2,5,8,11,14,17,20,23,26-nonaoxo-28-phenyl-1-oxa-4,7,10,13,16,19,22,25-octaazacyclooctacosan-27-yl}-L-leucinamid-monotrifluoracetat

### {N-(Anilinocarbonothioyl)lysobactin-monotrifluoracetat}

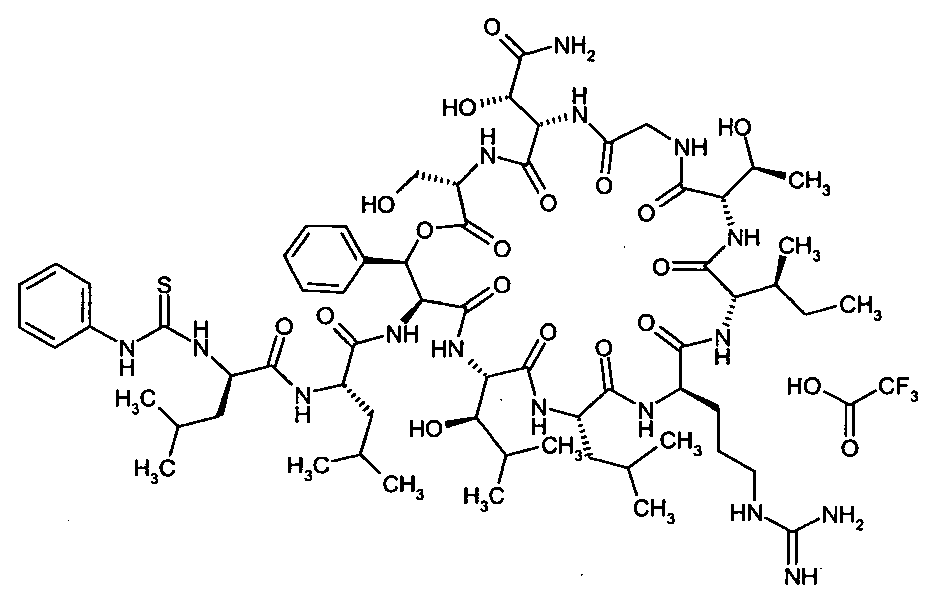

Gemäß der Allgemeinen Arbeitsvorschrift 1 wird Lysobactin-bistrifluoracetat (500 mg, 0.33 mmol) (Beispiel 1A) umgesetzt. Man erhält 600 mg (quant.) Produkt, das ungereinigt weiter umgesetzt werden kann.

Zur weiteren Aufreinigung kann das Rohprodukt gelchromatographiert (Methode 6; Methanol/0.1% Essigsäure) werden. Die Produkt-haltigen Fraktionen werden im Vakuum bei Raumtemperatur eingeengt und dann lyophilisiert. Man erhält das Produkt in 80% Ausbeute.

HPLC/UV-Vis (Methode 13): Rₜ = 6.84 min,
λₘₐₓ (qualitativ) = 220 nm (s), 248 (m), 269 (m).
LC-MS (Methode 11): Rₜ = 2.64 min;
MS (ESIpos.): m/z (%) = 706.5 (50) [M + 2H]²⁺, 1412 (20) [M + H]⁺;
LC-MS (Methode 12) : Rₜ = 4.95 min;
MS (ESIpos.): m/z (%) = 1412 (100) [M + H]⁺.

### Beispiel 3A

### N¹-{(3S,6S,12S,15S,18R,21S,24S,27S,28R)-6-[(1S)-2-Amino-1-hydroxy-2-oxoethyl]-18-(3-{[amino(imino)methyl]amino} propyl)-12-[(1S)-1-hydroxyethyl]-3-(hydroxy-methyl)-24-[(1R)-1-hydroxy-2-methylpropyl]-21-isobutyl-15-[(1S)-1-methylpropyl]-2,5,8,11,14,17,20,23,26-nonaoxo-28-phenyl-1-oxa-4,7,10,13,16,19,22,25-octaazacyclooctacosan-27-yl}-L-leucinamid-bistrifluoracetat

### {Des-D-leucyllysobactin-bistrifluoracetat}

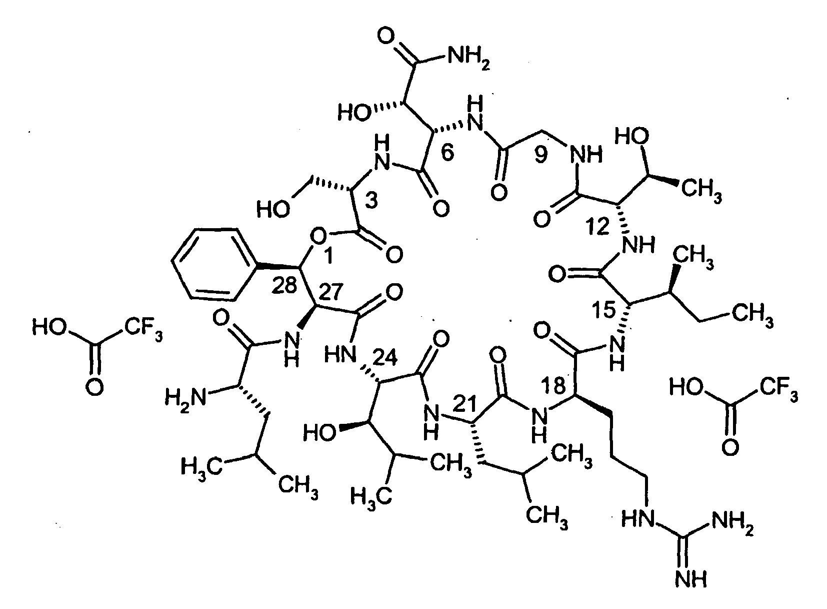

Thioharnstoff (Beispiel 2A) (300 mg, 0.2 mmol) wird gemäß der Allgemeinen Arbeitsvorschrift 2 umgesetzt. Das Rohprodukt wird gelchromatographiert (Methode 6; Methanol/0.25% Essigsäure) und anschließend mittels präparativer HPLC (Methode 8) feingereinigt. Man erhält 147 mg (65% d. Th.) Produkt.
HPLC/UV-Vis (Methode 13): Rₜ = 4.96 min,
λₘₐₓ (qualitativ) = 220 nm (s), 255-270 (w).
LC-MS (Methode 12): Rₜ = 3.84 min;
MS (ESIpos.): m/z (%) = 582.4 (100) [M + 2H]²⁺, 1164 (20) [M + H]⁺.
FT-ICR-HR-MS (Methode 23):
C₅₂H₈₈N₁₄O₁₆ [M + 2H]²⁺ ber. 582.32459, gef. 582.32460;
C₅₂H₈₇N₁₄NaO₁₆ [M + H + Na]²⁺ ber. 593.31556, gef. 593.31564.

Zur Aminosäure-Sequenzbestimmung wird eine analytische Probe des Produkts nach der Allgemeinen Arbeitsvorschrift 9 hydrolysiert.

MALDI-MS (Methode 20): m/z (%) = 1181.7 (100) [M + H]⁺.

### Alternatives Darstellungsverfahren in größerem Maßstab:

Beispiel 1A (6.47 g, 4.30 mmol) wird unter Argonatmosphäre in Pyridin (90 ml) gelöst. Dann wird Phenylisothiocyanat (1.16 g, 8.60 mmol, 2 Äquivalente) zugegeben und die Reaktionsmischung bei 37°C für 1 h gerührt. Anschließend wird das Lösungsmittel am Rotationsverdampfer abdestilliert und der Rückstand über Nacht am Ölpumpenvakuum getrocknet. Man erhält das Zwischenprodukt Beispiel 2A in einer Rohausbeute von 6.60 g. Das Zwischenprodukt wird ohne Reinigung weiter umgesetzt. Dazu wird Beispiel 2A (6.60 g) unter Argonatmosphäre in Trifluoressigsäure (107 ml) gelöst und 30 min bei Raumtemperatur gerührt. Dann wird die Lösung am Rotationsverdampfer unter Vakuum aufkonzentriert, kurz am Ölpumpenvakuum getrocknet, in Methyl-tert-butylether (250 ml) aufgenommen und so lange heftig gerührt, bis ein pulvriger amorpher Feststoff entstanden ist. Dieser wird mit Vakuum abfiltriert und mit Methyl-tert-butylether (200 ml) gewaschen, dann wird noch mit Dichlormethan (zweimal 100 ml) nachgewaschen. Der Feststoff wird in einen Kolben überführt und am Ölpumpenvakuum getrocknet. Man erhält Beispiel 3A in einer Rohausbeute von 6.0 g (quant.). Das Produkt kann ohne weitere Reinigung umgesetzt werden.

### Beispiel 4A

### N²-(Anilinocarbonothioyl)-N¹- {(3S,6S,12S,15S,18R,21S,24S,27S,28R)-6-[(1S)-2-amino-1-hydroxy-2-oxoethyl]-18-(3-{[amino(imino)methyl]aminolpropyl)-12-[(1S)-1-hydroxyethyl]-3-(hydroxymethyl)-24-[(1R)-1-hydroxy-2-methylpropyl]-21-isobutyl-15-[(1S)-1-methylpropyl]-2,5,8,11,14,17,20,23,26-nonaoxo-28-phenyl-1-oxa-4,7,10,13,16,19,22,25-octaazacyclooctacosan-27-yl}-L-leucinamid-monotrifluoracetat

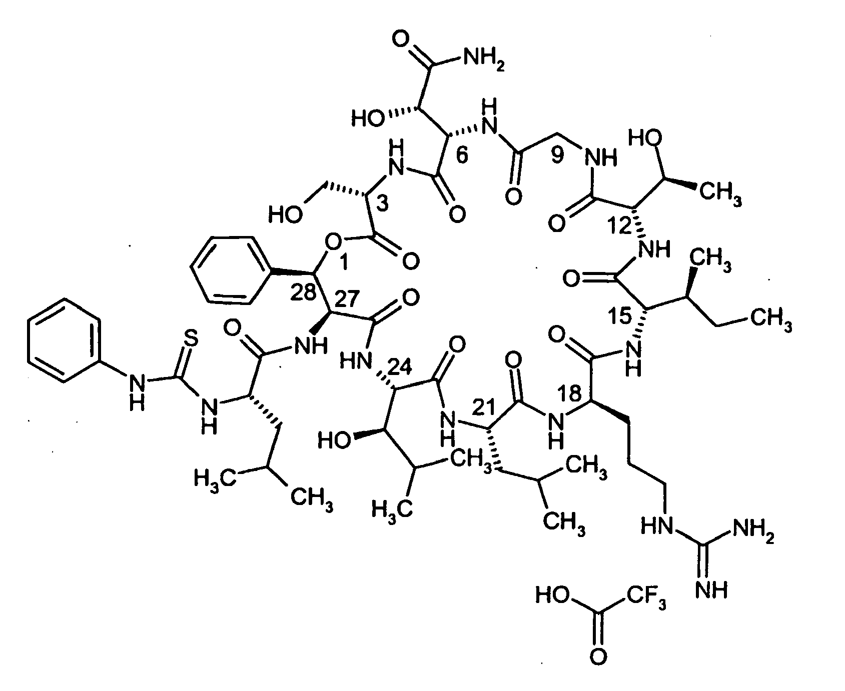

Gemäß der Allgemeinen Arbeitsvorschrift 1 wird Des-D-leucyllysobactin-bistrifluoracetat (Beispiel 3A, 255 mg, 0.18 mmol) umgesetzt. Man erhält 322 mg (quant.) Produkt, das ungereinigt weiter umgesetzt werden kann.

Zur weiteren Aufreinigung kann das Rohprodukt gelchromatographiert (Methode 6; Methanol/0.1% Essigsäure) werden. Die Produkt-haltigen Fraktionen werden im Vakuum bei Raumtemperatur eingeengt und dann lyophilisiert.
HPLC/UV-Vis (Methode 13): Rₜ = 6.56 min,
λₘₐₓ (qualitativ) = 220 nm (s), 245 (m), 268 (m).
LC-MS (Methode 12): Rₜ = 4.85 min;
MS (ESIpos.): m/z (%) = 1299 (100) [M + H]⁺.

### Beispiel 5A

### (2S)-2-{(3S,6S,12S,15S,18R,21S,24S,27S,28R)-27-Amino-18-(3-{[amino(imino)methyl]amino}-propyl)-12-[(1S)-1-hydroxyethyl]-3-(hydroxymethyl)-24-[(1R)-1-hydroxy-2-methylpropyl]-21-isobutyl-15-[(1S)-1-methylpropyl]-2,5,8,11,14,17,20,23,26-nonaoxo-28-phenyl-1-oxa-4,7,10,13,16,19,22,25-octaazacyclooctacosan-6-yl) -2-hydroxyethanamid-bistrifluoracetat

### {Des(1-D-leucyl-2-L-leucyl)lysobactin-bistrifluoracetat}

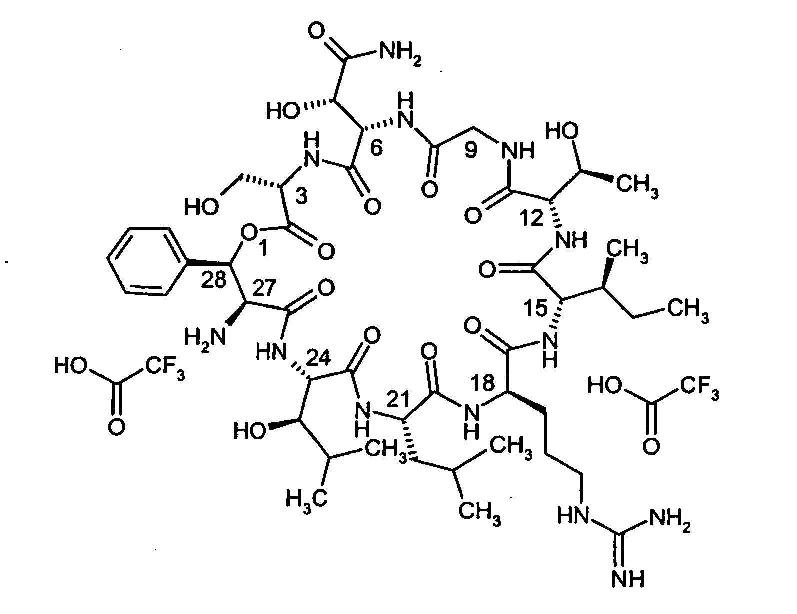

Der Thioharnstoff (Beispiel 4A, 66 mg, 34 µmol) wird gemäß der Allgemeinen Arbeitsvorschrift 2 umgesetzt. Das Rohprodukt kann durch zügige Gelchromatographie (Methode 6; Methanol/0.25% Essigsäure) vorgereinigt werden. Präparative HPLC (Methode 8 oder Methode 9 gefolgt von anschließendem Umsalzen des Chromatographieprodukts durch Zusatz von TFA (100 µmol)) liefert 45 mg (75% d. Th.) Produkt.
HPLCIUV-Vis (Methode 13): Rₜ = 4.71 min,
λₘₐₓ (qualitativ) = 220 nm (s), 255-270 (w).
LC-MS (Methode 11): Rₜ = 1.65 min;
MS (ESIpos.): m/z (%) = 526 (100) [M + 2H]²⁺, 1051 (15) [M + H]⁺.

### Alternatives Darstellungsverfahren in größerem Maßstab:

Beispielverbindung 3A (6.00 g, 4.30 mmol) wird unter Argonatmosphäre in Pyridin (92 ml) gelöst. Dann wird Phenylisothiocyanat (8.75 g, 64.68 mmol, 15 Äquivalente) zugegeben und die Reaktionsmischung bei 37°C für 1 Stunde gerührt. Anschließend wird das Lösungsmittel am Rotationsverdampfer abdestilliert und der Rückstand über Nacht am Ölpumpenvakuum getrocknet. Man erhält Beispiel 4A in einer Rohausbeute von 6.0 g. Das Zwischenprodukt wird ohne Reinigung weiter umgesetzt. Dazu wird das rohe Beispiel 4A unter Argonatmosphäre in Trifluoressigsäure (82 ml) gelöst und 30 min bei Raumtemperatur gerührt. Dann wird die Lösung am Rotationsverdampfer unter Vakuum aufkonzentriert, kurz am Ölpumpenvakuum getrocknet, in Methyl-*tert-*butylether (250 ml) aufgenommen und so lange heftig gerührt, bis ein pulvriger amorpher Feststoff entstanden ist. Dieser wird mit Vakuum abfiltriert und mit weiterem Methyl-tert-butylether (200 ml) gewaschen, dann wird noch mit zwei Portionen je 100 ml Dichlormethan nachgewaschen. Der Feststoff wird in einen Kolben überführt und am Ölpumpenvakuum getrocknet. Man erhält die Titelverbindung in einer Rohausbeute von 5.4 g (quant.). Das Produkt wird durch präparative HPLC weiter aufgereinigt (Methode 22). Man erhält 1.79 g der Titelverbindung (32% d. Th.).

### Vorstufen aus Aminosäuren:

### Beispiel 6A und Beispiel 7A

Die Synthese erfolgt nach M. Merget, K. Günther, M. Bernd, E. Günther, R. Tacke, J. Organomet. Chem. 2001 628, 183-194. Die Trennung der Enantiomere erfolgt durch präparative HPLC an chiraler Phase (Gilson Abimed HPLC, UV-Detektor 212 nm, Säule: Daicel Chiralpak AD-H 5 µm; 250. x 20 mm; Fluss: 15 ml/min; Eluent A: *iso*-Hexan, Eluent B: 0.2% Essigsäure/1% Wasser/2-Propanol; isokratisch).

### Beispiel 6A

### (2R)-N-tert-Butoxycarbonyl-3-trimethylsilyl-alanin

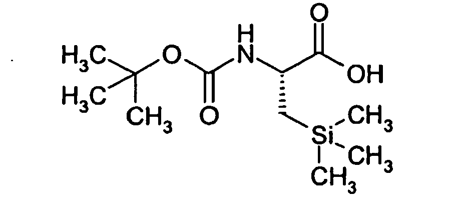

Präparative HPLC: Rₜ = 9.27 min
[α]_{D}²⁰ = -1.6 (c = 0.66, Methanol)

### Beispiel 7A

### (2S)-N-tert-Butoxycarbonyl-3-trimethylsilyl-alanin

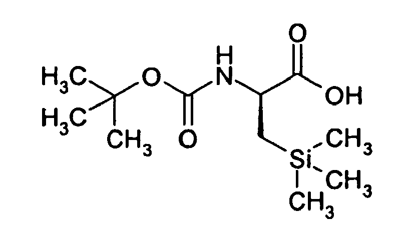

Präparative HPLC: Rₜ = 4.16 min
[α]_{D}²⁰ = +1.1 (*c* = 0.83, Methanol)

### Beispiel 8A

### (2R)-3-Trimethylsilyl-alaninmethylester Hydrochlorid

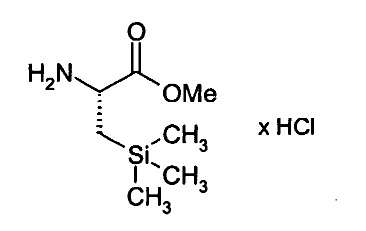

Die Verbindung aus Beispiel 6A (300 mg) wird in Methanol (3 ml) gelöst und auf 0°C abgekühlt. Trimethylsilylchlorid (590 mg, 4.7 Äq.) wird in 30 min. zugetropft und die Mischung über Nacht auf RT erwärmt. Die flüchtigen Komponenten werden am Rotavapor und anschließend im Hochvakuum entfernt. Man erhält die Zielverbindung (224 mg, 92% d. Th.).

¹H-NMR (DMSO, 300 MHz): δ = 8.40 (br s, 3H), 3.93 (dd, J = 5.3, 10.9 Hz, 1H), 3.73 (s, 3H), 1.71-0.97 (m, 2H), 0.03 (s, 9H).

### Beispiel 9A

### (2R)-N-Benzyloxycarbonyl-3-trimethylsilyl-alaninmethylester

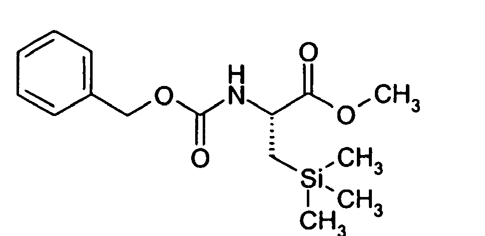

Die Verbindung aus Beispiel 8A (2.0 g) wird in THF (40 ml) unter Argon bei 0°C vorgelegt. Triethylamin (2.3 g, 2.4 Äq.) wird zugegeben und anschließend eine Lösung von N-Benzyloxycarbonyloxysuccinimid (2.83 g, 1.2 Äq.) in 20 ml THF zugetropft. Dann wird die Mischung über Nacht bei RT gerührt, zur Hälfte eingeengt, mit Essigsäureethylester verdünnt, zweimal mit Wasser und einmal mit gesättigter Natriumchlorid-Lösung gewaschen. Die organische Phase wird über Natriumsulfat getrocknet und einrotiert. Der Rückstand wird über präparative HPLC (Methode 25) gereinigt. Man erhält die Zielverbindung (2.32 g, 79% d. Th.)
HPLC (Methode 15): Rₜ = 4.96 min.
MS (DCI / NH₃): m/z = 327 [M+NH₄]⁺ (100); 310 [M+H]⁺ (3).

### Beispiele 10A

### (2R)-N-Benzyloxycarbonyl-3-trimethylsilyl-alaninol

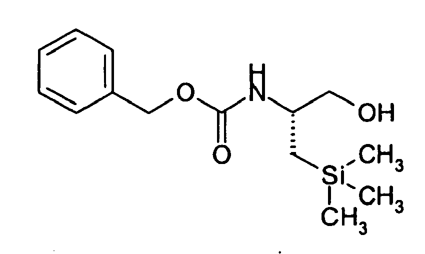

Zu einer Lösung von Beispiel 9A (2.32 g, 7.5 mmol) in THF (14 ml) werden Lithiumchlorid (0.64 g, 15 mmol) und Natriumborhydrid (0.57 g, 15 mmol) und dann 22 ml Ethanol zugegeben. Die Mischung wird über Nacht bei RT gerührt. Zur Aufarbeitung wird unter Kühlung mit Eiswasser mit einer Zitronensäurelösung (10%ig in Wasser) auf pH 4 gestellt, dann mit 200 ml Wasser verdünnt und dreimal mit Dichlormethan extrahiert. Die vereinigten organischen Phasen werden auf Magnesiumsulfat getrocknet, filtriert und im Vakuum eingeengt. Der Rückstand wird über HPLC (Methode 25) getrennt und die geeigneten Fraktion im Vakuum eingeengt. Man erhält Beispiel 10A als Öl (1.24 g, 59% d. Th).
HPLC (Methode 15): Rₜ = 4.48 min.;
MS (DCI / NH₃): m/z = 299 [M+NH₄]⁺ (100); 282 [M+H]⁺ (13);
¹H-NMR (CDCl₃, 300 MHz): δ = 7.33 (m, 5H), 5.08 (s, 2H), 4.72 (br.m, 1H), 3.83 (br.m, 1H), 3.65 (br.m, 1H), 3.45 (br.m, 1H), 2.13 (br.s, 1H), 0.84-0.58 (m, 2H), 0.03 (s, 9H).

### Beispiel 11A

### (2R)-N-Benzyloxycarbonyl-3-trimethylsilyl-alaninal

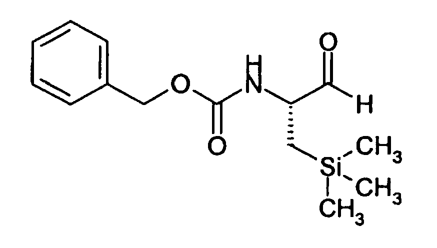

Eine Lösung des Pyridin-SO₃-Komplexes (2.04 g, 12.8 mmol) in wasserfreiem DMSO (12 ml) wird zu einer Lösung von Beispiel 10A (0.36 g, 1.28 mmol) und N,N-Diisopropylethylamin (1.65 g, 12.8 mmol) in 7 ml DMSO unter Argon bei 10°C zugegeben. Das Kühlbad wird entfernt und die Mischung 20 min. nachgerührt. Es werden 400 ml Eiswasser zugegeben und die Lösung wird dreimal mit Diethylether extrahiert. Die vereinigten organischen Phasen werden zweimal mit einer Zitronensäurelösung (10%ig in Wasser), einmal mit Wasser, einmal mit gesättigter Bicarbonatlösung und einmal mit gesättigter Natriumchlorid-Lösung gewaschen, über Magnesiumsulfat getrocknet, filtriert und einrotiert. Beispiel 11A wird als Öl erhalten (0.39 g, 93% d. Th.) und direkt als Rohprodukt weiter eingesetzt.
MS (DCI / NH₃): m/z = 297 [M+NH₄]⁺ (100); 280 [M+H]⁺ (25).
¹H-NMR (CDCl₃, 300 MHz): δ = 9.53 (s, 1H), 7.35 (m, 5H), 5.13 (s, 2H), 1.12 (dd, J = 5.8, 14.0 Hz, 1H), 0.78 (dd, J = 9.4, 14.0 Hz, 1H), 0.07 (s, 9H).

### Beispiel 12A

### (2R)-N-Benzyloxycarbonyl-D-3-tert-butyl-alaninol

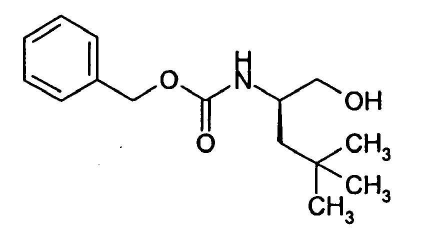

Zu einer eisgekühlten Lösung von N-Benzyloxycarbonyl-D-(3-tert-butyl)-alanin-dicyclohexylamin-Salz (1.5 g, 3.26 mmol) in wasserfreiem THF (15 ml) wird langsam eine Lösung von Boran-Tetrahydrofuran-Komplex in THF (1M, 8.14 ml, 8.14 mmol) zugetropft und dann die Lösung innerhalb von 1.5 h unter Rühren auf RT gebracht. Es wird unter Kühlung mit Wasser versetzt, in vacuo etwas eingeengt, mit gesättigter Natirumchlorid-Lösung verdünnt und dreimal mit Diethylether extrahiert. Die vereinigten organischen Phasen werden zweimal mit 0.1N Salzsäure, zweimal mit gesättigter Natriumhydrogencarbonat-Lösung und einmal mit gesättigter Natriumchlorid-Lösung gewaschen, über Natriumsulfat getrocknet und einrotiert. Der Rückstand wird durch präparative HPLC (Methode 25) gereinigt und die geeigneten Fraktionen einrotiert und dann im Hochvakuum getrocknet. Ausbeute: 485 mg (56% d. Th.).
HPLC (Methode 15): Rₜ = 4.4 min.
MS (DCI / NH₃): m/z = 283 [M+NH₄]⁺ (100); 266 [M+H]⁺ (60).
¹H-NMR (CDCl₃, 300 MHz): δ = 7.35 (m, 5H), 5.12 (s, 2H), 4.72 (br.d, J = ca. 5H), 3.83 (m, 1H), 3.63 (dd, J = 4.0, 10.7 Hz, 1H), 3.51 (dd, J = 6.3, 10.7 Hz, 1H), 1.44 (dd, J = 2.9, 14.7 Hz, 1H), 1.28 (dd, J = 8.7, 14.7 Hz, 1H), 0.95 (s, 9H).

### Beispiel 13A

### (2R)-N-tert-Butoxycarbonyl-3-trimethylsilyl-alaninol

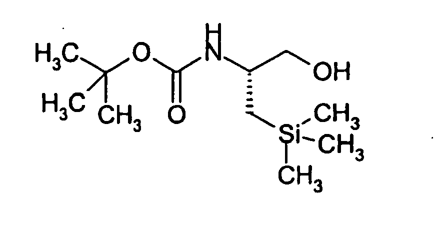

Die Herstellung erfolgt ausgehend von Beispiel 6A (2.45 g, 9.37 mmol) mit Boran-Tetrahydrofuran-Komplex (2 Äq.) nach dem gleichen Verfahren wie für Beispiel 12A beschrieben. Man erhält Beispiel 13A (1.70 g, 73% d. Th.) nach Chromatographie über Kieselgel (Dichlormethan / Methanol 100:3).
HPLC (Methode 15): Rₜ = 4.4 min.
MS (ESI pos): m/z = 270 [M+Na]⁺ (46); 192 (100).
¹H-NMR (CDCl₃, 300 MHz): δ = 4.60-4.38 (br.m, 1H), 3.87-3.72 (br.m, 1H), 3.69-3.56 (br.m, 1H), 3.50-3.38 (br.m, 1H), 2.40 (br.s, 1H) 1.44 (s, 9H), 0.82-0.66 (m, 2H), 0.04 (s, 9H).

### Beispiel 14A

### (2R)-N-tert-Butoxycarbonyl-3-trimethylsilyl-alaninal

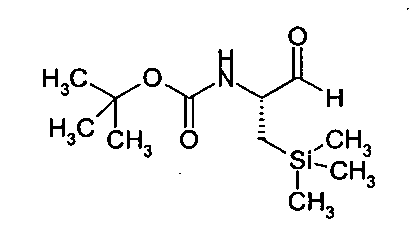

Die Titelverbindung wird aus Beispiel 13A (620 mg, 2.51 mmol) analog zur Synthese von Beispiel 11A hergestellt. Ausbeute 550 mg (80% d. Th.). Beispiel 14A wird ohne weitere Reinigung umgesetzt.

¹H-NMR (CDCl₃, 300 MHz): δ = 9.50 (s, 1H), 4.93-4.82 (br.m, 1H), 4.26 (br.q, J = ca. 7-8 Hz, 1H), 1.44 (s, 9H), 1.09 (br. dd, J = 5.3,14.8 Hz, 1H), 0.74 (dd, J = 10.0, 14.8 Hz, 1H), 0.08 (s, 9H).

In Analogie zu den oben beschriebenen Synthesevorschriften werden aus käuflichen Aminosäurenderivaten folgende Ausgangsverbindungen hergestellt:

### Beispiel 15A

### (2S)-N-Benzyloxycarbonyl-L-3-tert-butyl-alaninol

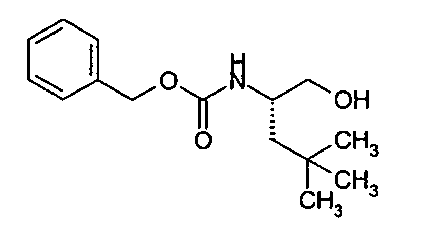

HPLC (Methode 15): Rₜ= 4.42 min.;
MS (DCI / NH₃): m/z = 283 [M+NH₄]⁺ (100); 266 [M+H]⁺ (58);
¹H-NMR (CDCl₃, 300 MHz): δ = 7.33 (m, 5H), 5.11(s, 2H), 4.71 (m, 1H), 3.83 (m, 1H), 3.62 (dd, J = 4.2, 10.5 Hz, 1H), 3.45 (dd, J = 6.3, 10.9 Hz, 1H), 1.43 (dd, J = 2.9, 14.6 Hz, 1H), 1.29 (dd, J = 8.9, 14.6, 1H), 0.93 (s, 9H).

### Beispiel 16A

### (2S)-N-Benzyloxycarbonyl-L-3-tert-butyl-alaninal

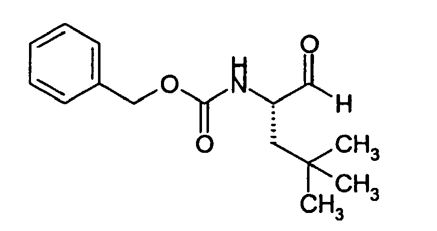

MS (DCI / NH₃): m/z = 281 [M+NH_{4]}⁺ (100); 264 [M+H]⁺ (20).
¹H-NMR (CDCl₃, 300 MHz): δ = 9.58 (s, 1H), 7.36 (m, 5H), 5.12 (s, 2H), 5.06 (m, 1H), 4.33 (dt, J = 3.8, 8.7 Hz, 1H), 1.83 (dd, J = 3.8, 14.6 Hz, 1H), 1.30 (dd, J = 8.3, 14.7 Hz, 1H), 1.00 (s, 9H).

### Beispiel 17A

### (2R)-N-Benzyloxycarbonyl-D-3-tert-butyl-alaninal

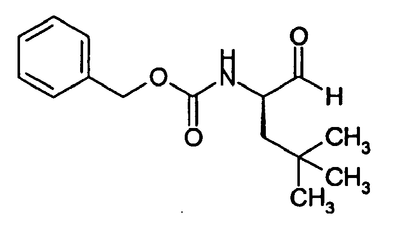

MS (DCI / NH₃): m/z = 281 [M+NH₄]⁺ (100); 264 [M+H]⁺ (18).
¹H-NMR (CDCl₃, 300 MHz): δ = 9.58 (s, 1H), 7.36 (m, 5H), 5.12 (s, 2H), 5.08 (m, 1H), 4.33 (dt, J = 3.8, 8.7 Hz, 1H), 1.83 (dd, J = 3.8, 14.6 Hz, 1H), 1.30 (dd, J = 8.3, 14.7 Hz, 1H), 1.00 (s, 9H).

### Beispiel 18A

### (2S)-N-Benzyloxycarbonyl-L-leucinal

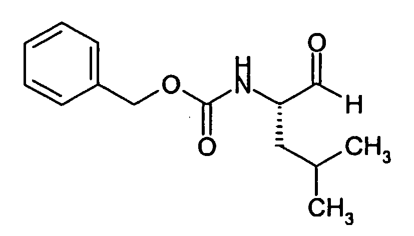

MS (DCI / NH₃): m/z = 267 [M+NH₄]⁺ (100); 250 [M+H]⁺ (8).
¹H-NMR (CDCl₃, 400 MHz): δ = 9.61 (s, 1H), 7.38 (m, 5H), 5.18 (m, 1H), 5.02 (s, 2H), 4.35 (m, 1H), 1.62-1.55 (m, 2H), 1.41 (m, 1H), 0.96 (d, J = 7 Hz, 3H), 0.94 (d, J = 7 Hz, 3H).

### Beispiel 19A

### (2R)-N-Benzyloxycarbonyl-D-leucinal

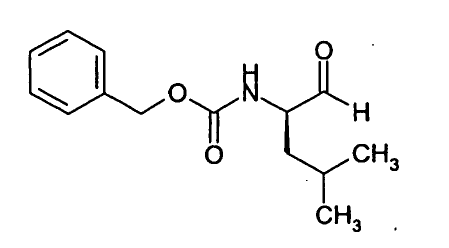

MS (DCI / NH₃): m/z = 267 [M+NH₄]⁺ (100); 250 [M+H]⁺ (10).
¹H-NMR (CDCl₃, 300 MHz): δ = 9.61 (s, 1H), 7.38 (m, 5H), 5.18 (m, 1H), 5.12 (m, 2H), 4.35 (m, 1H), 1.85-1.50 (m, 2H), 1.41 (m, 1H), 0.95 (m, 6H).

### Beispiel 20A

### (2S)-N-tert-Butoxycarbonyl-L-3-tert-butyl-alaninal

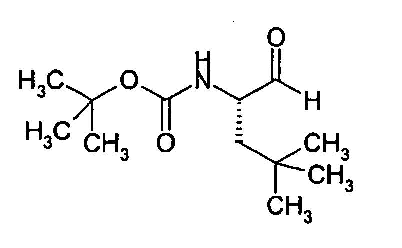

MS (DCI / NH₃): m/z = 247 [M+NH₄]⁺ (15); 230 [M+H]⁺ (31), 191 (100).
¹H-NMR (CDCl₃, 400 MHz): δ = 9.58 (s, 1H), 4.82 (m, 1H), 4.25 (m, 1H), 1.80 (dd, J = 2, 14.6 Hz, 1H), 1.44 (s, 9H), 1.25 (dd, J = 8.7, 14.6 Hz, 1H), 1.00 (s, 9H).

### Beispiel 21A

### (2R)-N-tert-Butoxycarbonyl-D-3-tert-butyl-alaninal

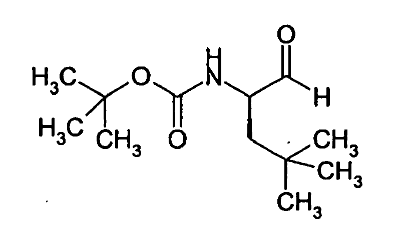

MS (DCI/NH₃): m/z = 247 [M+NH₄]⁺ (13); 230 [M+H]⁺ (28), 191 (100).
¹H-NR4R (CDCl₃, 400 MHz): δ = 9.58 (s, 1H), 4.82 (m, 1H), 4.25 (m, 1H), 1.80 (dd, J = 2, 14.6 Hz, 1H), 1.44 (s, 9H), 1.25 (dd, J = 8.7, 14.6 Hz, 1H), 1.00 (s, 9H).

### Beispiel 22A

### L-β-(3-Pyridyl)-alaninmethylester-bis-hydrochlorid

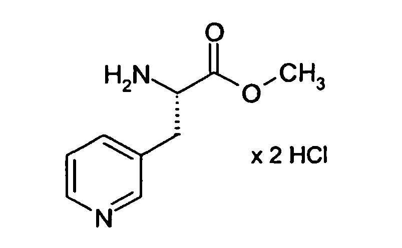

Analog zur Herstellung von Beispiel 8A wird das käufliche Boc-L-β-(3-Pyridyl)-alanin mit Trimethylsilylchlorid in Methanol behandelt. Nach vollständiger Umsetzung wird die Reaktionsmischung mit Diethylether versetzt und das ausgefallene kristalline Produkt abfiltriert. Das Produkt wird mit kaltem Diethylether gewaschen und im Hochvakuum getrocknet. Ausbeute: 94% d. Th.
HPLC (Methode 15): Rₜ = 4.29 min.
¹H-NMR (DMSO, 400 MHz): δ = 8.86 (s, 1H), 8.82 (br. s, 3H), 8.80 (d, J = 5.5 Hz, 1H), 8.38 (d, J = 8.0 Hz, 1H), 7.92 (dd, J = 5.5, 8.0 Hz, 1H), 4.50 (m, 1H), 3.74 (s, 3H), 3.38 (m [ABX], 2H).

### Beispiel 23A

### L-β-(3,4-Dimethoxyphenyl)-alanin-methylester Hydrochlorid

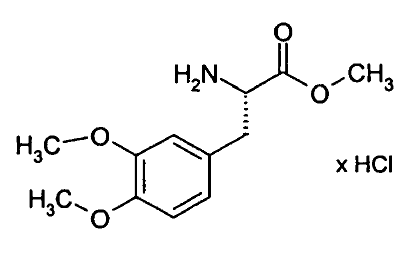

Das käufliche L-β-(3,4-Dimethoxyphenyl)-alanin (225 mg, 1 mmol) wird in Methanol (5 ml) vorgelegt, mit Thionylchlorid (73 µl, 1 Äq.) versetzt und die Mischung 1 h auf Rückfluss erhitzt, dann einrotiert und im Hochvakuum getrocknet. Man erhält 277 mg Produkt.
¹H-NMR (DMSO, 300 MHz): δ = 8.55 (br.s, 3H), 6.89 (d, J = 8.0 Hz, 1H), (d, J = 1.9 Hz, 1H), 6.73 (dd, J = 1.9, 8.0 Hz, 1H), 4.25 (br.t, J = ca. 6 Hz, 1H), 3.75 (s, 3H), 3.73 (s, 3H), 3.71 (s, 3H), 3.08 (d, J = 6.2 Hz, 2H).

### Beispiel 24A

### L-β-tert-Butyl-alaninmethylester Hydrotrifluoracetat

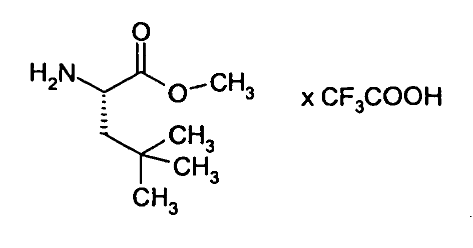

Der (2*S*)-*N*-*tert*-Butoxycarbonyl-L-3-*tert*-butyl-alaninmethylester (529 mg, 2.04 mmol) wird in TFA / Dichlormethan 1:3 (3 ml) bei RT gerührt, die Lösung einrotiert und im Hochvakuum getrocknet. Man erhält 540 mg (97% d. Th.) Produkt.
¹H-NMR (DMSO, 300 MHz): δ = 8.33 (br.s, 3H), 3.95 (dd, J = 4.7, 7.5 Hz, 1H), 3.76 (s, 3H), 1.80 (dd, J = 7.5, 14.4 Hz, 1H), 1.59 (dd, J = 4.7, 14.4 Hz, 1H), 0.92 (s, 9H).

### Desoxodipeptidester

Nach der allgemeinen Arbeitsvorschrift 3 werden die folgenden Beispiele 25A bis 37A hergestellt:

### Beispiel 25A

### N-[(2R)-2-Benzyloxycarbonylamino-4,4-dimethylpentyl]-β-tert-butyl-alaninmethylester Hydrotrifluoracetat

### (Z-D-β-tBu-Ala(ΨCH₂NH)-L-β-tBu-Ala-OMe).TFA

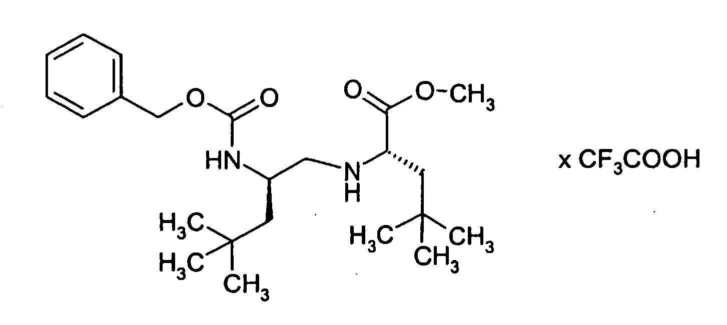

Herstellung aus Beispiel 17A und Beispiel 24A. Ausbeute: 220 mg (65% d. Th.).
HPLC (Methode 15): Rₜ= 4.7 min.;
MS (DCI / NH₃): m/z = 407 [M+H]⁺ (100);
¹H-NMR (CDCl₃, 300 MHz): δ = 7.34 (s, 5H), 5.52 (br.d, J = 6.6 Hz, 1H), 5.14 (q, J= 12 Hz, 1H), 5.06 (q, J= 12 Hz, 1H), 4.09-3.95 (m, 2H), 3.79 (s, 3H), 3.25-3.03 (m, 2H), 2.01-1.78 (m, 2H), 1.50 (dd, J = 7.8, 15, 1H), 1.34 (m, 1H), 0.94 (s, 9H), 0.92 (s, 9H).

### Beispiel 26A

### N-[(2S)-2-Benzyloxycarbonylamino-4,4-dimethylpentyl]-β-tert-butyl-alaninmethylester Hydrotrifluoracetat

### (Z-L-β-tBu-Ala(ΨCH₂NH)-L-β-tBu-Ala-OMe).TFA

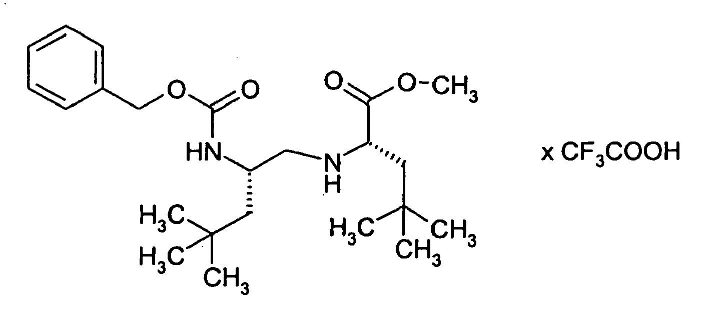

Herstellung aus Beispiel 16A und Beispiel 24A. Ausbeute: 860 mg (36% d. Th.).
HPLC (Methode 15): Rₜ= 4.7 min.;
MS (ESI pos): m/z = 407 [M+H]⁺ (100);
¹H-NMR (CDCl₃, 300 MHz): δ = 7.32 (m, 5H), 5.73 (br.d, J = 8.5 Hz, 1H), 5.13 (d, J= 12 Hz, 1H), 5.04 (d, J= 12 Hz, 1H), 3.99 (m, 1H), 3.84 (m, 1H), 3.80 (s, 3H), 3.33 (dd, J = 10.5, 12 Hz, 1H), 2.89 (dd, J = 3.5, 12 Hz, 1H), 1.95-1.68 (m, 2H), 1.53 (dd, J = 8.7, 14.7, 1H), 1.34 (br.d, J = 15, 1H), 0.93 (s, 9H), 0.91 (s, 1H).

### Beispiel 27A

### N-[(2R)-2-Benzyloxycarbonylamino-4-methyl-pentyl]-β-(3-pyridyl)-alaninmethylester-bis-hydrotrifluoracetat

### (Z-D-Leu(ΨCH₂NH)-L-β-(3-pyridyl)-Ala-OMe).2TFA

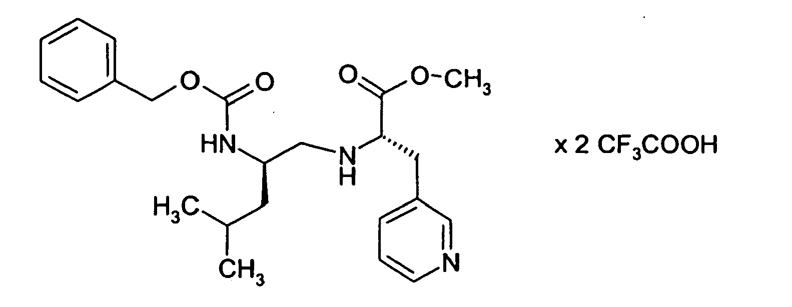

Herstellung aus Beispiel 19A und Beispiel 22A. Ausbeute: 800 mg (60% d. Th.).
LC-MS (Methode 16): Rₜ= 1.67 min. ES⁺: m/z = 414 [M+H]⁺, ES': 458 [M+HCOOH-H]⁺;
¹H-NMR (CDCl₃, 300 MHz): δ = 8.68 (s, 1H), 8.51 (d, J = 4.4 Hz, 1H), 8.1 (br.d, J = 7.2 Hz, 1H), 7.54 (dd, J = 5.1, 7.7 Hz, 1H), 7.38-7.28 (m, 5H), 5.12 (m, 1H), 5.03 (d, J= 12.2 Hz, 1H), 4.97 (d, J= 12.2 Hz, 1H), 3.99 (m, 1H), 3.84 (m, 1H), 3.79 (s, 3H), 3.33-2.99 (m, 3H), 2.85 (m, 1H), 11.61 (m, 1H), 1.42-1.20 (m, 2H), 0.82-0.98 (m, 6H).

### Beispiel 28A

### N-[(2S)-2-Benzyloxycarbonylamino-4-methyl-pentyl]-β-(3-pyridyl)-alaninmethylester-bis-hydrotrifluoracetat

### (Z-L-Leu(ΨCH₂NH)-L-β-(3-pyridyl)-Ala-OMe).2TFA

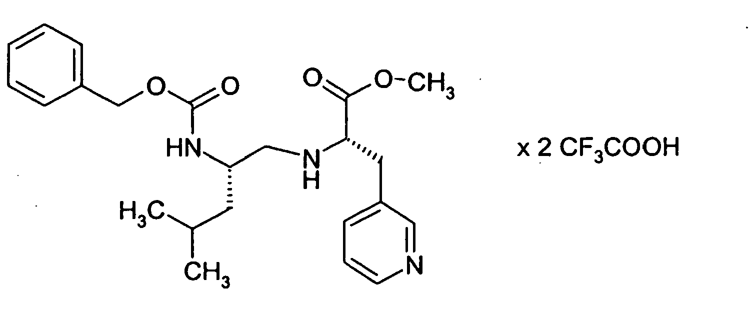

Herstellung aus Beispiel 18A und Beispiel 22A. Ausbeute 410 mg (48% d. Th.).
LC-MS (Methode 14): Rₜ= 1.51 min. ES⁺: m/z = 414 [M+H]⁺, ES⁻: 458 [M+HCOOH-H]⁻.
¹H-NMR (CDDCl₃, 400 MHz): δ = 8.90 (s, 1H), 8.66 (d, J = 5.5 Hz, 1H), 8.33 (d, J = 8.3 Hz, 1H), 7.69 (dd, J = 5.5, 7.8 Hz, 1H), 7.39-7.28 (m, 5H), 5.36 (d, J = 7.1, 1H), 5.06 (m [AB], 2H), 4.35 (t, J = 5.1 Hz, 1H), 3.84 (s, 3H), 3.54 (d, J = 5.4 Hz, 1H), 3.28 (t, J = 10.6 Hz, 1H), 3.08 (d, J = 10.5 Hz, 1H), 1.62 (m, 1H), 1.45 (ddd, J = 5.4, 9.1, 14.3 Hz , 1H), 1.29 (ddd, J = 4.9, 8.4, 13.9 Hz, 2H), 0.90 (d, J = 6.6 Hz, 3H), 0.90 (d, J = 6.4 Hz, 3H).

### Beispiel 29A

### N-[(2S)-2-tert-Butoxycarbonylamino-4,4-dimethylpentyl]-β-(3-pyridyl)-alaninmethylester-bis-hydrotrifluoracetat

### (Boc-L-β-tBu-Ala(ΨCH₂NH)-L-β-(3-pyridyl)-Ala-OMe).2TFA

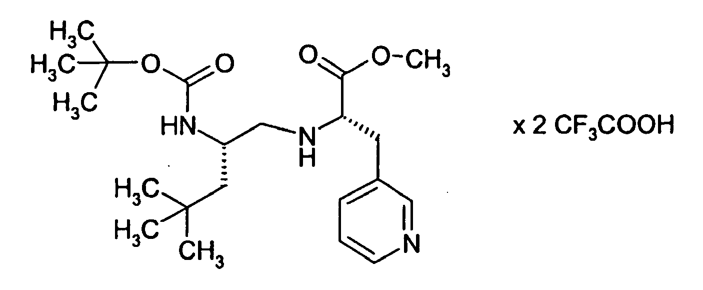

Herstellung aus Beispiel 20A und Beispiel 22A. Ausbeute: 160 mg (59% d. Th.).
HPLC (Methode 15): Rₜ= 4.0 min.;
MS (ESI pos): m/z = 394 [M+H]⁺(92), 338 (80), 294 (100).
¹H-NMR (CDCl₃, 400 MHz): δ = 8.51 (s, 1H), 8.75 (d, J = 5.4 Hz, 1H), 8.48 (d, J = 7.8 Hz, 1H), 7.83 (dd, J = 5.5, 7.8 Hz, 1H), 4.98 (br.d, J = 6.1 Hz, 1H), 4.38 (t, J= 5.6 Hz, 1H), 3.87 (s, 3H), 3.67 -3.52 (m, 2H), 3.35 (t, J = 10.5 Hz, 1H), 2.98 (d, J = 11.7 Hz, 1H), 1.44 (dd, J = 9.0, 14.9 Hz, 1H), 1.40 (s, 9H), 1.31 (dd, J = 2.6,14.8 Hz, 1H), 0.93 (s, 9H).

### Beispiel 30A

### N-[(2S)-2-Benzyloxycarbonylamino-4-methyl-pentyl]-β-(3,4-dimethoxyphenyl)-alaninmethylester-hydrotrifluoracetat

### (Z-L-Leu(ΨCH₂NH)-L-β-(3,4-dimethoxyphenyl)-Ala-OMe).TFA

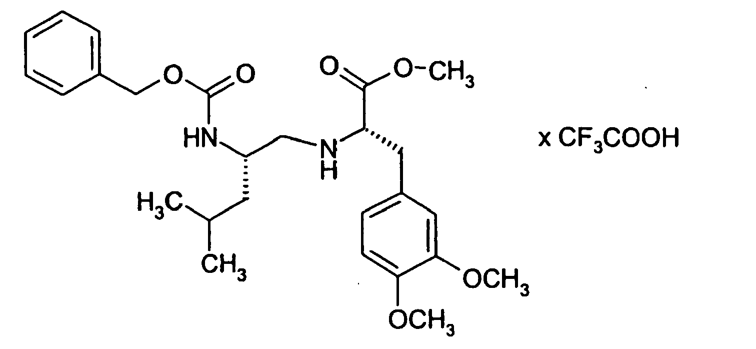

Herstellung aus Beispiel 18A und Beispiel 23A. Ausbeute 200 mg (32% d. Th.).
LC-MS (Methode 16): Rₜ= 1.91 min. ES⁺: m/z = 473 [M+H]⁺, ES⁻: 517 [M+HCOOH-H]⁻.
¹H-NMR (DMSO, 300 MHz): δ = 9.50-9.00 (br.s, 2H), 7.40-723 (m, 6H), 6.90 (d, J = 8.1 Hz, 1H), 6.82 (d, J= 1.5 Hz, 1H), 6.71 (dd, J= 1.6, 8.2 Hz, 1H), 5.05 (q [AB], J = 12.5 Hz, 2H), 4.30 (m, 1H), 3.85 (m, 1H), 3.75 (s, 3H), 3.73 (s, 3H), 3.64 (s, 3H), 3.20 (dd, J = 5.1, 13.7 Hz, 1H), 3.07-2.93 (m, 3H), 1.58 (m, 1H), 1.38 (ddd, J = 4.6, 9.5, 14.1 Hz, 1H), 1.22 (ddd, J = 4.5, 8.8, 14 Hz, 1H), 0.86 (t, J = 6.4 Hz, 6H).

### Beispiel 31A

### N-[(2R)-2-Benzyloxycarbonylamino-4-methyl-pentyl]-β-(3,4-dimethoxyphenyl)-alaninmethylesterhydrotrifluoracetat

### (Z-D-Leu(ΨCH₂NH)-L-β-(3,4-dimethoxyphenyl)-Ala-OMe).TFA

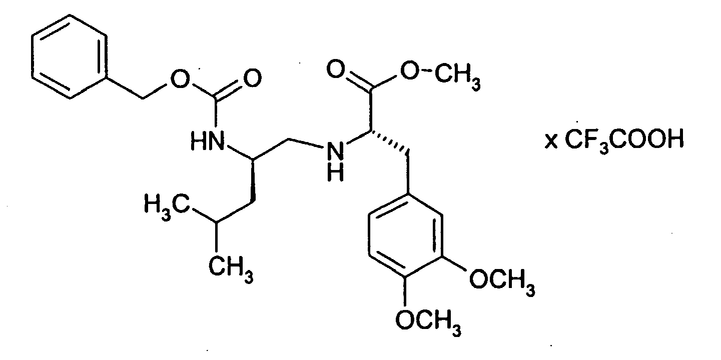

Herstellung aus Beispiel 19A und Beispiel 23A. Ausbeute 190 mg (31% d. Th.).
LC-MS (Methode 16): Rₜ= 1.66 min. ES⁺: m/z = 473 [M+H]⁺, ES⁻: 517 [M+HCOOH-H]⁻.
¹H-NMR (CDCl₃, 400 MHz): δ = 7.38-7.23 (m, 5H), 6.82-6.77 (m, 2H), 6.67 (dd, J= 1.8, 7.8 Hz, 1H), 5.18 (d, J= 6.2 Hz, 1H), 4.99 (s, 2H), 4.23 (t, J = 5.4 Hz, 1H), 3.98 (m, 1H), 3.90 (s, 3H), 3.81 (s, 3H), 3.72 (s, 3H), 3.34 (d, J = 12.0 Hz, 1H), 3.27 (d, J = 5.4 Hz, 2H), 2.94 (t, J = 10.8 Hz, 1H), 1.59 (m, 1H),1.42-1.25 (m, 2H), 0.90 (t, J = 6.5 Hz, 6H).

### Beispiel 32A

### N-[(2R)-2-tert-Butoxycarbonylamino-3-trimethylsilyl-propyl]-β-(3-pyridyl)-alaninmethylesterhydrotrifluoracetat

### (Boc-L-β-Trimethylsilyl-Ala(ΨCH₂NH)-L-β-(3-pyhdyl)-Ala-OMe)TFA

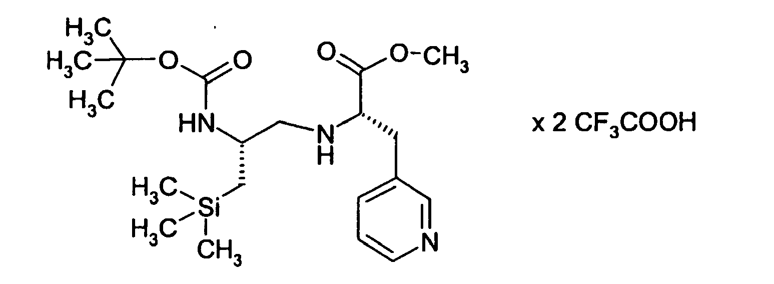

Herstellung aus Beispiel 14A und Beispiel 22A. Ausbeute: 270 mg (73% d. Th.).
HPLC (Methode 15): Rₜ= 4.2 min.;
MS (ESI pos): m/z (%) = 410 [M+H]⁺(100), 354 (77), 310 (73), 293 (70), 221 (97).
¹H-NMR (CDCl₃, 300 MHz): δ = 8.85 (s, 1H), 8.71 (d, J = 5.2 Hz, 1H), 8.47 (br.d, J = 7.4 Hz, 1H), 7.80 (br.dd, J = 4.8, 7.6 Hz, 1H), 4.94 (br.d, J = 6.0 Hz, 1H), 4.40-4.30 (m, 1H), 3.99-3.84 (m, 1H), 3.89 (s, 3H), 3.65-3.52 (m, 2H), 3.32 (br.t, J = ca. 10 Hz, 1H), 2.98 (br.d, J = 10.5 Hz, 1H), 1.41 (s, 9H), 0.90 (dd, J = 10.5, 15.0, 1H), 0.75 (dd, J = 4.5, 15.0, 1H), 0.05 (s, 9H).

### Beispiel 33A

### N-[((2R)-2-tert-Butoxycarbonylamino-3-trimethylsilyl-propyl]-leucinbenzylesterhydrotrifluoracetat.

### (Boc-L-β-Trimethylsilyl-Ala(ΨCH₂NH)-L-Leu-OBn)TFA

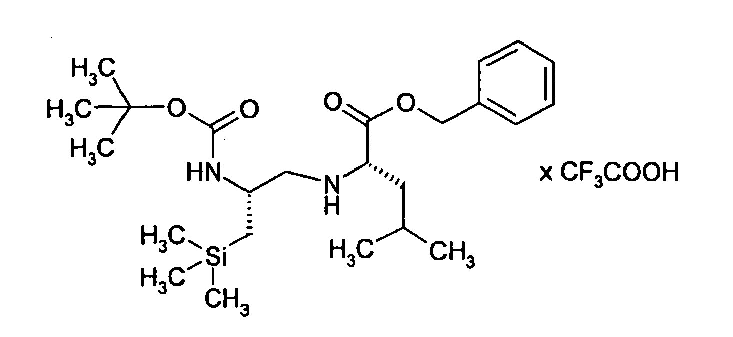

Herstellung aus Beispiel 14A und L-Leucinbenzylester-hydrotosylat. Ausbeute 180 mg (28% d. Th.).
HPLC (Methode 15): Rₜ = 5.1 min.
¹H-NMR (CDCl₃, 400 MHz): δ = 7.41-7.34 (m, 5H), 5.23 (m [AB], 2H), 5.06 (br.d, J = ca. 5.0 Hz, 1H), 4.03-3.92 (m, 2H), 3.30 (t, J = 10.5 Hz, 1H), 2.91 (d, J = 11.0 Hz, 1H), 1.80 (m, 2H), 1.66 (m, 1H), 1.43 (s, 9H), 0.91 (d, J = 2.2 Hz, 3H), 0.89 (d, J = 1.9 Hz, 3H), 0.85 (dd, J = 10.3, 14.7 Hz, 1H), 0.68 (dd, J = 5.0, 14.6 Hz, 1H), 0.04 (s, 9H).

### Beispiel 34A

### N-[(2R)-2-Benzyloxycarbonylamino-3-trimethylsilyl-propyl]-β-tert-butyl-alaninmethylesterhydrotrifluoracetat.

### (Z-L-β-Trimethylsilyl-Ala(ΨCH₂NH)-L-β-tBu-Ala-OMe)TFA

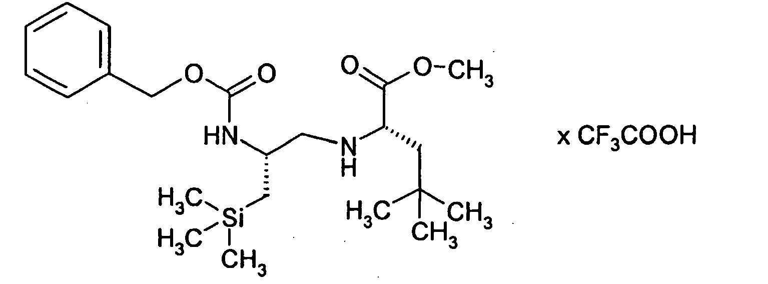

Herstellung aus Beispiel 11A und Beispiel 24A. Ausbeute 390 mg (66% d. Th.).
HPLC (Methode 15): Rₜ = 4.8 min.
MS (ESI pos.): m/z = 423 [M+H]⁺ (100).
¹H-NMR (CDCl₃, 300 MHz): δ = 7.33 (s, 5H), 5.63 (d, J = 0.9 Hz, 1H), 5.16 (d, J = 11.5 Hz, 1H), 5.05 (d, J = 11.5 Hz, 1H), 4.07-3.90 (m, 1H), 3.85 (dd, J = 4.5, 7.9 Hz, 1H), 3.81 (s, 3H), 3.35 (dd, J = 9.8, 11.3 Hz, 1H), 2.93 (dd, J = 2.5, 12.5 Hz, 1H), 1.95-1.80 (m, 2H), 0.95 (dd, J = 10.2, 14.7 Hz, 1H), 0.94 (s, 9H), 0.76 (dd, J = 5.1, 14.7 Hz, 1H), 0.03 (s, 9H).

### Beispiel 35A

### N-[(2S)-2-Benzyloxycarbonylamino-4,4-dimethyl-pentyl]-β-trimethylsilyl-alaninmethylesterhydrotrifluoracetat

### (Z-L-β-t-Butyl-Ala(ΨCH₂NH)-L-β-(trimethylsilyl)-Ala-OMe)TFA

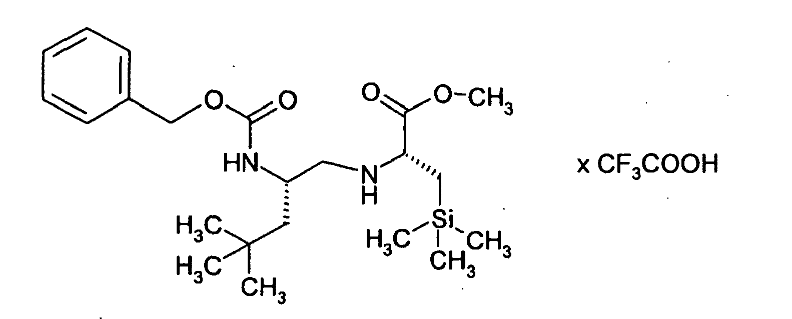

Herstellung aus Beispiel 16A und Beispiel 8A. Ausbeute 106 mg (37% d. Th.).
HPLC (Methode 15): Rₜ = 4.8 min.
MS (DCI/NH₃): m/z = 423 (100) [M+H]⁺.
¹H-NMR (CDCl₃, 300 MHz): δ = 7.32 (br.s, 5H), 8.76 (br.d, J = 9 Hz, 1H), 5.15 (d, J = 12 Hz, 1H), 5.05 (d, J = 12 Hz, 1H), 4.03 (m, 1H), 3.94 (dd, J = 12.6, 3.8 Hz, 1H), 3.78 (s, 3H), 3.34 (dd, J = 10, 12 Hz, 1H), 2.87 (dd, J = 4, 12 Hz, 1H), 1.53 (dd, J = 8.9, 14.7 Hz, 1H), 1.40-1.22 (m, 2H), 1.12 (dd, J = 12.5, 13.5 Hz, 1H), 0.92 (s, 9H), 0.06 (s, 9H).

### Beispiel 36A

### N-[(2R)-2-Benzyloxycarbonylamino-3-trimethylsilyl-propyl]-β-trimethylsilyl-alaninmethylesterhydrotrifluoracetat

### (Z-L-β-Trimethylsilyl-Ala(ΨCH₂NH)-L-β-(trimethylsilyl)-Ala-OMeTFA

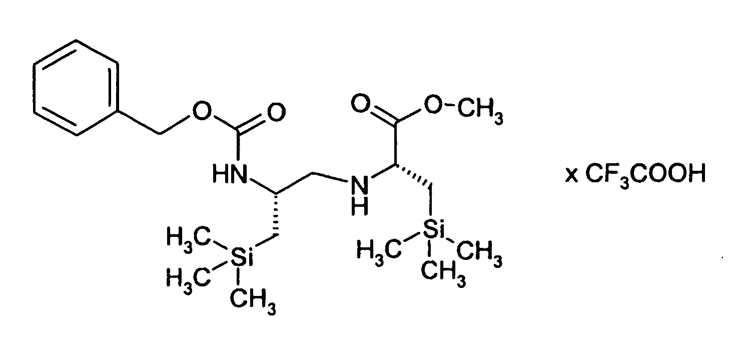

Herstellung aus Beispiel 11A und Beispiel 8A. Ausbeute 54 mg (24% d. Th.).
HPLC (Methode 15): Rₜ = 4.93 min.
¹H-NMR (CDCl₃, 400 MHz): δ = 7.36-7.26 (m, 5H), 5.47 (br d, J = 6 Hz, 1H), 5.16 (d, J = 12.4 Hz, 1H), 5.04 (d, J = 12.4 Hz, 1H), 4.04 (m, 1H), 3.96 (d, J = 10.8 Hz, 1H), 3.70 (s, 3H), 3.32 (t, J = 9.7 Hz, 1H), 2.91 (d, J = 11.3 Hz, 1H), 1.31 (br.d, J = 14.2 Hz, 1H), 1.12 (t, J = 12.7 Hz, 1H), 0.92 (dd, J = 9.8, 14.9 Hz, 1H), 0.74 (dd, J = 4.8, 14.4 Hz, 1H), 0.06 (s, 9H), 0.02 (s, 9H).

### Beispiel 37A

### N-[(2S)-2-Benzyloxycarbonylamino-4,4-dimethylpentyl]-O⁵-tert-butyl-asparaginsäuremethylesterhydrotrifluoracetat

### (Z-L-β-tBu-Ala(ΨCH₂NH)-L-Asp(tBu)-OMe)TFA

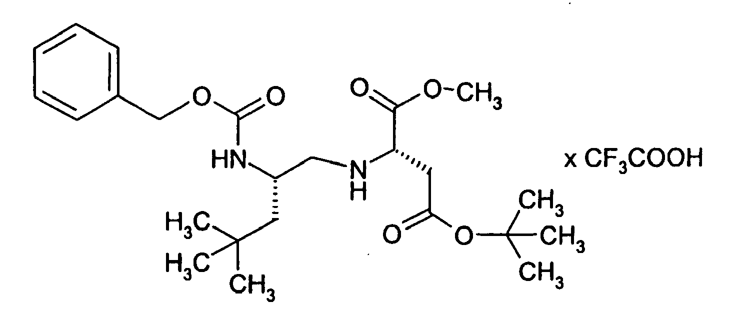

Herstellung aus Beispiel 16A und H-Asp(tBu)-OMe Hydrochlorid. Ausbeute 212 mg (46% d. Th.).
HPLC (Methode 15): Rₜ = 4.7 min.
MS (DCI/NH₃): m/z = 451 (100) [M+H]⁺, 317 (23).
¹H-NMR (CDCl₃, 300 MHz): δ = 7.37-7.27 (m, 5H), 8.76 (br.d, J = 9 Hz, 1H), 5.53 (d, J = 7.9 Hz, 1H), 5.09 (q [AB], J = 12.2 Hz, 2H), 4.13 (m, 1H), 4.07 (t, J = 4.7 Hz, 1H), 3.82 (s, 3H), 3.37 (br.t, J = 10.8 Hz, 1H), 3.21-2.99 (m, 3H), 1.53 (dd, J = 8.8, 14.5 Hz, 1H), 1.44 (s, 9H), 1.32 (dd, J = 2.7, 14.5 Hz, 1H), 0.92 (s, 9H).

### Desoxodipeptidsäure

Nach der allgemeinen Arbeitsvorschrift 4 werden folgende Desoxodipeptidsäuren (Beispiele 38A bis 49A) hergestellt:

### Beispiel 38A

### N-[(2R)-2-Benzyloxycarbonylamino-4,4-dimethylpentyl]-β-tert-butyl-alanin-hydrotrifluoracetat (Z-D-β-tBu-Ala(ΨCH₂NH)-L-β-tBu-Ala-OH)TFA

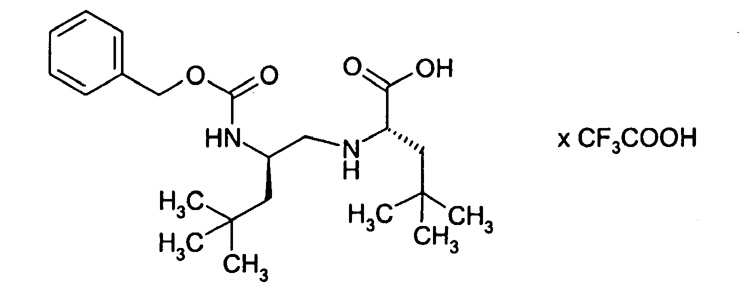

Herstellung aus Beispiel 25A. Ausbeute: 103 mg (51% d. Th.).
HPLC (Methode 15): Rₜ=4.5 min.;
MS (ESI pos.): m/z = 393 [M+H]⁺, (ESI neg.): 391 ([M-H]⁻.
¹H-NMR (DMSO, 300 MHz): δ = 9.30-8.60 (br, 1H), 7.40-7.22 (m, 6H), 5.13 (d, J = 12.7 Hz, 1H), 4.97 (d, J = 12.7 Hz, 1H), 3.92 (m, 1H), 3.84 brd, J = 8.9 Hz, 1H), 2.94 (br.d, J = 12.1 Hz, 1H), 2.81 (dd, , J = 10.2, 12.2 Hz, 1H), 1.80 (dd, J = 9.5, 13.8 Hz, 1H), 1.67 (br.d, J = 9.5 Hz, 1H), 1.44 (dd, J = 8.6, 14.2 Hz, 1H), 1.30 (dd, J = 2.2, 14.2 Hz, 1H), 0.93 (s, 9H), 0.87 (s, 9H).

### Beispiel 39A

### N-[(2S)-2-Benzyloxycarbonylamino-4,4-dimethylpentyl]-β-tert-butyl-alanin-hydrotrifluoracetat (Z-L-β-tBu-Ala(ΨCH₂NH)-L-β-tBu-Ala-OH)TFA

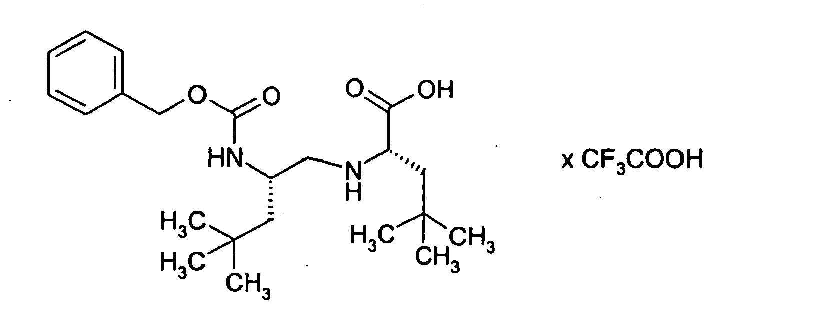

Herstellung aus Beispiel 26A. Ausbeute: 895 mg (83% d. Th.).
HPLC (Methode 15): Rₜ= 4.5 min.
MS (ESI pos.): m/z = 393 [M+H]⁺ (100); ES-: 391 (4).
¹H-NMR (DMSO, 300 MHz): δ = 7.40-7.27 (m, 5H), 7.07 (d, J = 9.0 Hz, 1H), 5.12 (d, J = 12.7 Hz, 1H), 4.99 (d, J = 12.7 Hz, 1H), 3.99-3.85 (m, 2H), 2.98 (m, 1H), 2.88 (m, 1H), 1.81 (dd, J = 9.7, 14 Hz, 1H), 1.64 (dd, J = 2, 14 Hz, 1H), 1.45-1.30 (m, 2H), 0.93 (s, 9H), 0.89 (s, 9H).

### Beispiel 40A

### N-[(2R)-2-Benzyloxycarbonylamino-4-methyl-pentyl]-β-(3-pyridyl)-alanin-bis-hydrotrifluoracetat (Z-D-Leu(ΨCH₂NH)-L-β-(3-pyridyl)-Ala-OH) 2 TFA

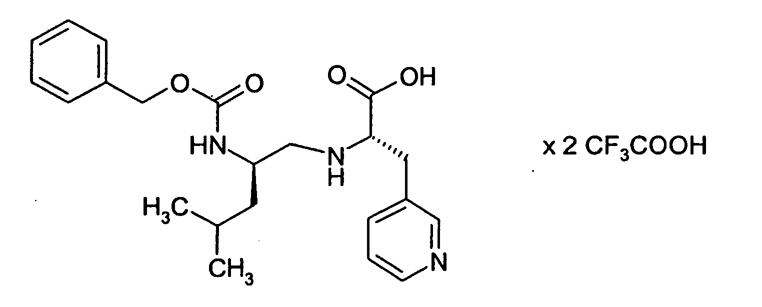

Herstellung aus Beispiel 27A. Ausbeute: 744 mg (95% d. Th.).
LC-MS (Methode 14): Rₜ = 1.17 min. ES⁺: m/z = 400 [M+H]⁺, ES⁻: 398 [M -H]⁻.
¹H-NMR (DMSO, 300 MHz): δ = 9.5-8.9 (br, 2H), 8.62 (d, J = 5.1 Hz, 1H), 8.61 (s, 1H), 7.97 (d, J = 7.8 H, 1H), 7.61 (dd, J = 5.1, 7.8 Hz, 1H), 7.41-7.28 (m, 6H), 5.13 (d, J = 12.5 Hz, 1H), 5.00 (d, J = 12.5 Hz, 1H), 4.29 (dd, J = 5.0, 8.8 Hz, 1H), 3.81 (m, 1H), 3.39 (dd, J = 4.8, 14.2 Hz, 1H), 3.16 (dd, J = 9.0, 14.2 Hz, 1H), 3.12 (br.d, J = 12.4 Hz, 1H), 2.97 (dd, J = 9.6, 12.4 Hz, 1H), 1.59 (m, 1H), 1.39 (ddd, J = 4.9, 9.5, 13.6 Hz, 1H), 1.23 (ddd, J = 4.7, 8.9, 13.6 Hz, 1H), 0.89 (d, J = 6.6 Hz, 3H), 0.86 (d, J = 6.3 Hz, 3H).

### Beispiel 41A

### N-[(2S)-2-Benzyloxycarbonylamino-4-methyl-pentyl]-β-(3-pyridyl)-alanin-bis-hydrotrifluoracetat

### (Z-L-Leu(ΨCH₂NH)-L-β-(3-pyridyl)-Ala-OH) 2 TFA

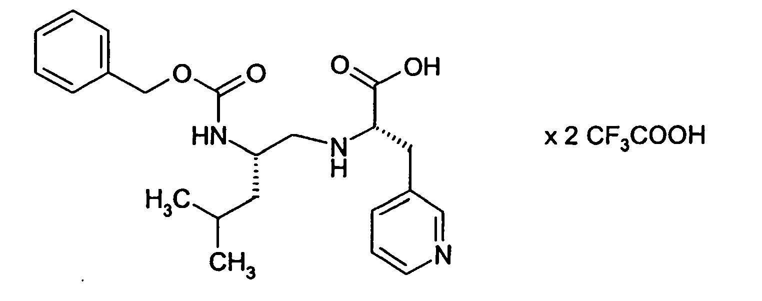

Herstellung aus Beispiel 28A. Ausbeute: 420 mg (74% d. Th.).
LC-MS (Methode 14): Rₜ= 1.15 min.
MS: ES⁺: m/z = 400 [M+H]⁺, ES⁻: 398 [M-H]⁻.
¹H-NMR (DMSO, 300 MHz): δ = 9.5-8.6 (br, 1H), 8.62 (br.s, 2H), 7.97 (d, J = 7.9 H, 1H), 7.61 (dd, J = 5.2, 7.6 Hz, 1H), 7.43-7.27 (m, 6H), 5.10 (d, J = 12.5 Hz, 1H), 5.02 (d, J = 12.5 Hz, 1H), 4.32 (dd, J = 5.0, 8.4 Hz, 1H), 3.89 (m, 1H), 3.16 (dd, J = 4.8, 14.2 Hz, 1H), 3.18 (dd, J = 8.9, 14.2 Hz, 1H), 3.14-2.98 (m, 2H), 1.59 (m, 1 H), 1.39 (ddd, J = 4.9, 8.7, 13.8 Hz, 1H), 1.26 (ddd, J = 4.7, 8.8, 13.8 Hz, 1H), 0.89 (d, J = 6.6 Hz, 3H), 0.86 (d, J = 6.3 Hz, 3H).

### Beispiel 42A

### N-[(2S)-2-tert-Butoxycarbonylamino-4,4-dimethylpentyl]-β-(3-pyridyl)-alanin-bis-hydrotrifluoracetat

### (Boc-L-(3-tBu-Ala(ΨCH₂NH)-L-β(3-pyridyl)-Ala-OH) 2 TFA

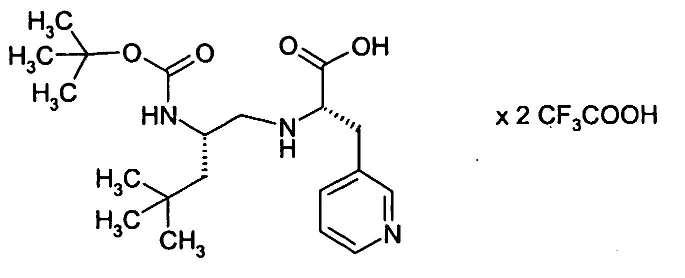

Herstellung aus Beispiel 29A. Ausbeute: 128 mg (77% d. Th.).
HPLC (Methode 15): Rₜ= 3.9 min.
MS: ES⁺: m/z = 380 [M+H]⁺ (100), 324 (85), 280 (88).
¹H-NMR (DMSO, 300 MHz): δ = 9.4-8.3 (br, 1H), 8.59 (d, J = ca. 5 Hz, 1H), 8.58 (s, 1H), 7.93 (d, J = 7.8 H, 1H), 7.56 (dd, J = 5.1, 7.8 Hz, 1H), 6.89 (d, J = 8.9 Hz, 1H), 4.31 (m, 1H), 3.86 (m, 1H), 3.34 (dd, J = 4.9, 14.1 Hz, 1H), 3.34 (dd, J = 8.9, 14.1 Hz, 1H), 3.10-2.90 (m, 2H), 1.44 (dd, J = 8.9, 14.4 Hz, 1H), 1.39 (s, 9H), 1.32 (dd, J = 2.5, 14.4 Hz, 1H), 0.90 (s, 9H).

### Beispiel 43A

### N-[(2S)-2-Benzyloxycarbonylamino-4-methylpentyl]-β-(3,4-dimethoxyphenyl)-alaninhydrotrifluoracetat

### (Z-L-Leu-(ΨCH₂NH)-L-β-(3,4-dimethoxyphenyl)-Ala-OH)TFA

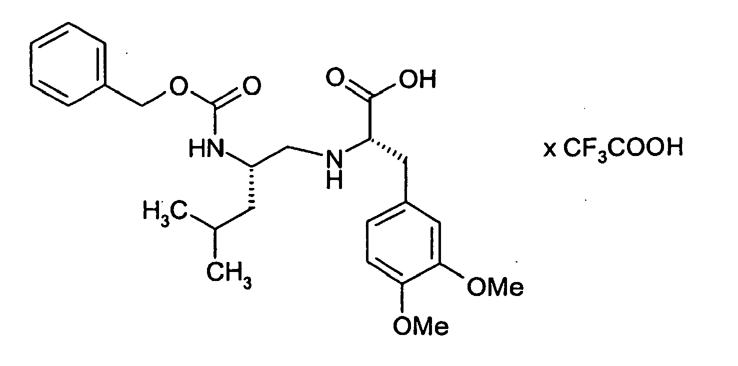

Herstellung aus Beispiel 30A. Ausbeute 140 mg (72% d. Th.).
LC-MS (Methode 17): Rₜ= 1.91 min. ES⁺: m/z = 459 [M+H]⁺, ES⁻: 457 [M-H]⁻.
¹H-NMR (DMSO, 400 MHz): δ = 9.3-8.4 (br, 2H), 7.41-7.29 (m, 5H), 7.26 (d, J = 8.7 Hz, 1H), 6.88 (d, J = 8.2 Hz, 1H), 6.87 (s, 1H), 6.75 (d, J = 8.2 Hz, 1H), 5.08 (d, J = 11.0 Hz, 1H), 5.00 (d, J = 11.0 Hz, 1H), 4.11 (m, 1H), 3.82 (m, 1H), 3.76 (s, 3H), 3.72 (s, 3H), 3.12 (dd, J = 5.2, 14.1 Hz, 1H), 3.02 (dd, J = 7.5, 14.1 Hz, 1H), 3.0-2.83 (m, 2H), 1.55 (m, 1H), 1.34 (ddd, J = 4.9, 9.8, 13.9 Hz, 1H), 1.21 (ddd, J = 4.4, 8.5,13.6 Hz, 1H), 0.87 (d, J = 6.6 Hz, 3H), 0.84 (d, J = 6.4 Hz, 3H).

### Beispiel 44A

### N-[(2R)-2-Benzyloxycarbonylamino-4-methylpentyl]-β-(3,4-dimethoxyphenyl)-alaninhydrotrifluoracetat

### (Z-D-Leu-(ΨCH₂NH)-L-β-(3,4-dimethoxyphenyl)-Ala-OH)TFA

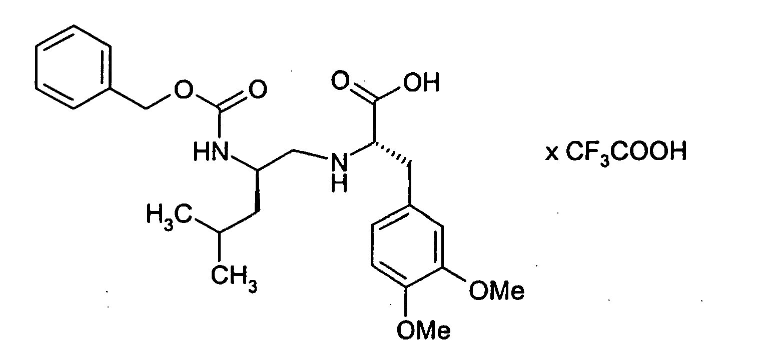

Herstellung aus Beispiel 31A. Ausbeute 126 mg (99% d. Th.).
LC-MS (Methode 17): Rₜ= 1.92 min. ES⁺: m/z = 459 [M+H]⁺, ES⁻: 457 [M-H]⁻.
¹H-NMR (DMSO, 400 MHz): δ = 9.4-8.4 (br, 2H), 7.41-7.29 (m, 5H), 7.27 (d, J = 8.7 Hz, 1H), 6.88 (d, J = 8.0 Hz, 1H), 6.86 (s, 1H), 6.75 (d, J = 8.0 Hz, 1H), 5.11 (d, J = 12.3 Hz, 1H), 5.00 (d, J = 12.3 Hz, 1H), 4.09 (m, 1H), 3.88 (m, 1H), 3.74 (s, 3H), 3.72 (s, 3H), 3.15 (dd, J = 4.9, 13.6 Hz, 1H), 2.99 (dd, J = 7.9, 14.1 Hz, 1H), 2.97 (br.d, J = ca. 12 Hz, 1H), 2.87 (dd, J = 9.7, 11.8 Hz, 1H), 1.55 (m, 1H), 1.35 (ddd, J = 4.9, 9.8, 13.9 Hz, 1H), 1.21 (ddd, J = 4.9, 8.5, 13.6 Hz, 1H), 0.87 (d, J = 7.8 Hz, 3H), 0.83 (d, J = 7.9 Hz, 3H).

### Beispiel 45A

### N-[(2R)-2-tert-Butoxycarbonylamino-3-trimethylsilyl-propyl]-β-(3-pyridyl)-alaninhydrotrifluoracetat

### (Boc-L-β-Trimethylsilyl-Ala(ΨCH₂NH)-L-β-(3-pyridyl)-Ala-OH TFA)

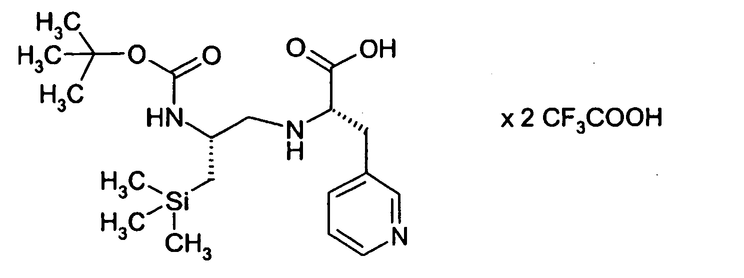

Herstellung aus Beispiel 32A. Ausbeute 56 mg (20% d. Th.).
LC-MS (Methode 17): Rₜ= 1.55 min. ES⁺: m/z = 396 [M+H]⁺, ES⁻: 394 [M-H]⁻.
¹H-NMR (CDCl₃, 400 MHz) (Hauptrotamer): δ = 9.10 (s, 1H), 8.68-8.58 (m, 2H), 7.81 (t, J = 6.6 Hz, 1H), 5.19 (br.d, J = ca. 6 Hz, 1H), 4.19 (m, 1H), 3.99 (m, 1H), 3.71-3.57 (m, 2H), 3.30-3.13 (m, 2H), 1.42 (s, 9H), 0.90 (dd, J = 10.8, 14.8 Hz, 1H), 0.75 (dd, J = 4.4, 15 Hz, 1H), 0.03 (s, 9H).

### Beispiel 46A

### N-[(2R)-2-Benzyloxycarbonylamino-3-trimethylsilyl-propyl]-β-tert-butyl-alaninhydrotrifluoracetat.

### (Z-L-β-Trimethylsilyl-Ala(ΨCH₂NH)-L-β-tBu-Ala-OH)TFA

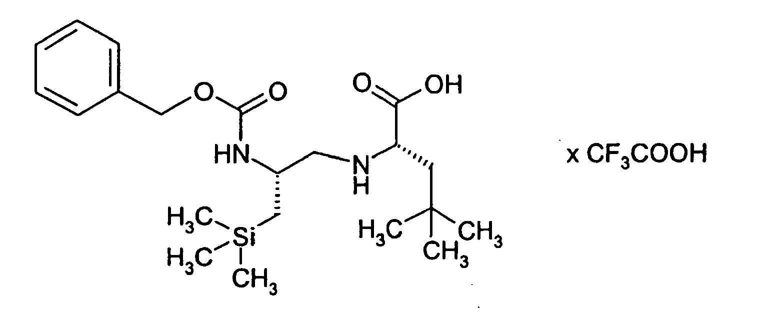

Herstellung aus Beispiel 34A. Ausbeute: 33 mg (47% d. Th.).
HPLC (Methode 15): Rₜ= 4.6 min.;
MS (ESI pos): m/z (%) = 409 (100) [M+H]⁺, ESI neg.: 407 (10) [M-H]⁻.
¹H-NMR (DMSO, 300 MHz): δ = 7.56-7.43 (m, 5H), 7.29 (d, J = 9.0 Hz, 1H), 5.13 (d, J = 12 Hz, 1H), 5.01 (d, J = 12 Hz, 1H), 4.03-3.80 (m, 2H), 3.10 (m, 1H), 2.88 (m, 1H), 1.84 (dd, J = 9.0, 14.5 Hz, 1H), 1.68 (br.d, J = 14.5 Hz, 1H), 0.95 (s, 9H), 0.82 (m, 2H), 0.03 (s, 9H).

### Beispiel 47A

### N-[(2S)-2-Benzyloxycarbonylamino-4,4-dimethyl-pentyl]-β-trimethylsilyl-alaninhydrotri fluoracetat

### (Z-L-β-t-Butyl-Ala(ΨCH₂NH)-L-β-(trimethylsilyl)-Ala-OH)TFA

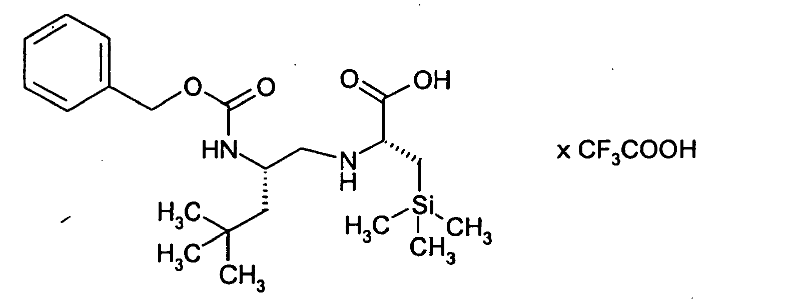

Herstellung aus Beispiel 35A. Ausbeute: 86 mg (88% d.Th.).
HPLC (Methode 15): Rₜ = 4.6 min.
MS: ES⁺: m/z = 409 (100) [M+H]⁺; ES⁻: 407 (9) [M-H]⁻, 113 (100).
¹H-NMR (DMSO, 300 MHz): δ = 9.00-8.65 (br., 2H), 7.42-7.25 (m, 5H), 7.28 (d, J = 9.1 Hz, 1H), 5.13 (d, J = 12.6 Hz, 1H), 4.99 (d, J = 12.6 Hz, 1H), 3.95-3.80 (m, 2H), 3.10 (m, 1H), 2.89 (m, 1H), 1.44 (dd, J = 10.3, 14.3 Hz, 1H), 1.35 (br.d, J = 14.3 Hz, 1H), 1.21 (dd, J = 3.9, 14.0 Hz, 1H), 0.88 (s, 9H), 0.07 (s, 9H).

### Beispiel 48A

### N-[(2R)-2-Benzyloxycarbonylamino-3-trimethylsilyl-propyl]-β-trimethylsilyl-alaninhydrotrifluoracetat

### (Z-L-β-Trimethylsilyl-Ala(ΨCH₂NH)-L-β-(trimethylsilyl)-Ala-OH)TFA

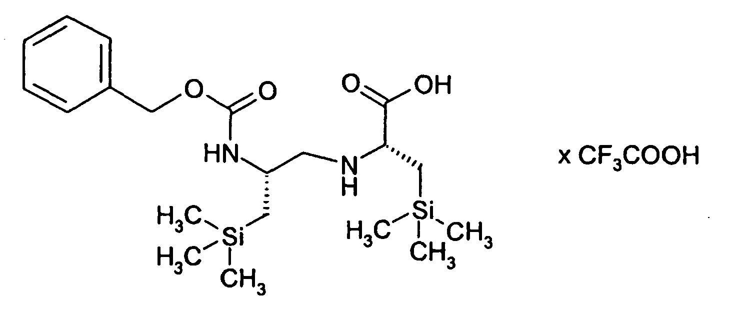

Herstellung aus Beispiel 36A. Ausbeute 43 mg (82% d. Th.).
LC-MS (Methode 16): Rₜ= 2.15 min. ES⁺: m/z = 425 [M+H]⁺, ES⁻: 423 [M-H]⁻.
¹H-NMR (DMSO, 300 MHz): δ = 9.00-8.55 (br,s, 2H), 7.39-7.22 (m, 5H), 7.25 (d, J = 10.2 Hz, 1H), 5.10 (d, J = 12.3 Hz, 1H), 4.98 (d, J = 12.3 Hz, 1H), 4.00-3.82 (m, 2H), 3.01 (m, 1H), 2.89 (m, 1H), 1.20 (dd, J = 3.9, 14.1 Hz, 1H), 1.03 (t, J = 13.3 Hz, 1H), 0.89-0.75 (m, 2H), 0.10 (s, 9H), 0.05 (s, 9H).

### Beispiele 49A

### N-[(2S)-2-Benzyloxycarbonylamino-4,4-dimethylpentyl]-O⁵-tert-butyl-asparaginsäurehydrotrifluoracetat

### (Z-L-β-tBu-Ala(ΨCH₂NH)-L-Asp(tBu)-OH)TFA

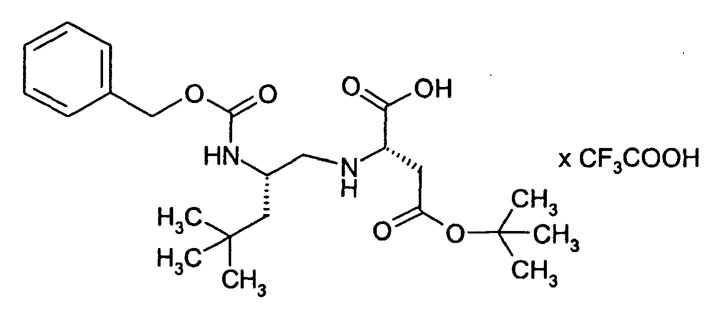

Herstellung aus Beispiel 37A. Ausbeute 78 mg (42% d. Th.).
HPLC (Methode 15): Rₜ= 4.5 min.
MS: ES⁺: m/z=437 (100) [M+H]⁺, ES⁻: 435 (12) [M-H]⁻.
¹H-NMR (DMSO, 300 MHz): δ = 9.5-8.0 (br., 2H), 7.39-7.27 (m, 6H), 5.10 (d, J = 12.6 Hz, 1H), 4.99 (d, J = 12.6 Hz, 1H), 4.12 (br.s, 1H), 3.94-3.83 (m, 1H), 3.00-2.82 (m, 2H), 2.82 (d, = 5.6 Hz, 2H), 1.43 (m, 1H), 1.41 (s, 9H), 1.30 (dd, J = 2.7, 14.2 Hz, 1H), 0.89 (s, 9H).

Nach der allgemeinen Arbeitsvorschrift 5 wird folgendes Beispiel 50A hergestellt:

### Beispiel 50A

### N-[(2R)-2-tert-Butoxycarbonylamino-3-trimethylsilyl-propyl]-leucin-hydrotrifluoracetat

### (Boc-L-β-Trimethylsilyl-Ala(ΨCH₂NH)-L-Leu-OH)TFA

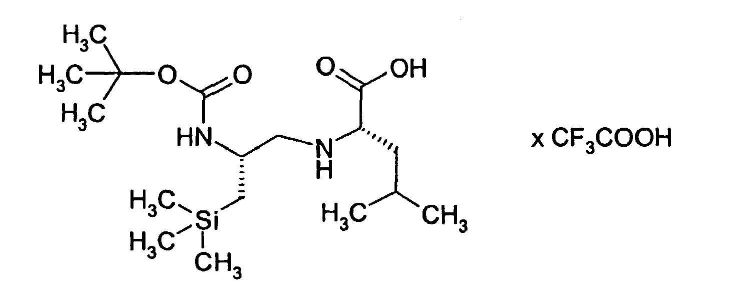

Herstellung aus Beispiel 33A. Ausbeute 155 mg (95% d. Th.).
MS (DCI/NH₃): m/z = 361 (100) [M+H]⁺, 315 (85).
¹H-NMR (DMSO, 300 MHz): δ = 9.1-8.5 (br, 2H), 6.85 (d, J = 9.0, 1H), 3.98-3.82 (m, 2H), 3.00 (dd, 7.6, 12.3 Hz, 1H), 2.87 (dd, J = 5.3, 12.3 Hz, 1H), 1.85-1.60 (m, 3H), 1.40 (s, 9H), 0.95 (d, J = ca. 2 Hz, 3H), 0.93 (d, J = ca. 2 Hz, 3H), 0.85-0.78 (m, 2H), 0.03 (s, 9H).

### Reduktive Aminierung aus Aminoaldehyden und Beispiel 3A

Nach der allgemeinen Arbeitsvorschrift 3 werden aus Beispiel 3A die Beispiele 51A bis 54A hergestellt. Die Ausbeute wird nicht auf dieser Stufe, sondern erst nach der Abspaltung der N-terminalen Schutzgruppe bestimmt.

| | | | |
|---|---|---|---|
| 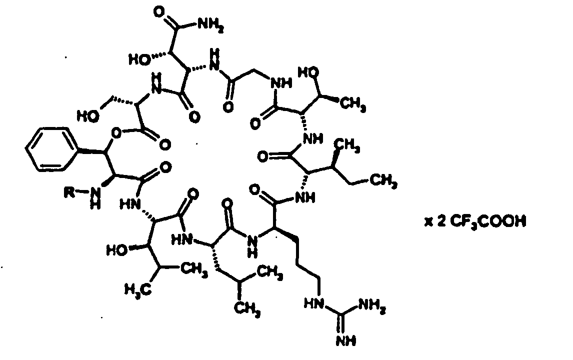 | | | |

| **Bsp-Nr.** | **Name** | **R** | **Ausgafagsverbindungen / Analytik** |
|---|---|---|---|
| **51A** | *N-*[(2S)-2-*tert*-Butoxycarbonylamino-4-methyl-pent-1-yl]-des(1-D-leucin)-lysobactin-bis-hydrotrifluoracetat | 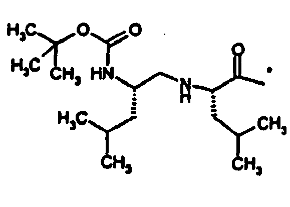 | aus Beispiel 3A und Boc-Leu-H. HPLC (Methode 15): Rₜ = 4.52 min. LC/MS: (Methode 17): Rₜ = 2.42 min.; ES⁺: 1363 [M+H]⁺, 682 [M+2H]²⁺; ES⁻: 1361 [M-H]⁻. |
| **52A** | *N-*[(*2S*)-2*-tert-*Butoxycarbonylamino-4,4-dimethyl-pent- 1-yl]-des(1-D-leucin)-lysobactin-bis-hydrotrifluoracetat | 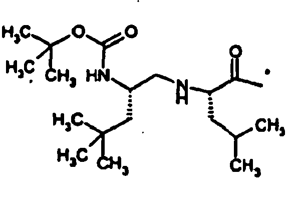 | aus Beispiel 3A und Beispiel 20A. HPLC (Methode 15): Rₜ = 4.44 min. LC/MS: (Methode 17): Rₜ = 1.86 min.; ES⁺: 1377 [M+H]⁺, 689.4 [M+2H]²⁺; ES⁻: 1375 [M-H]⁻. HR-MS: [M+H]⁺ gefunden: 1376.8138 berechnet: 1376.8153 |
| **53A** | *N-*[(*2R*)-2-*tert-*Butoxycarbonylamino-4,4-dimethyl-pent-1-yl]-des(1-D-leucin)-lysobactin-bis-hydrotrifluoracetat | 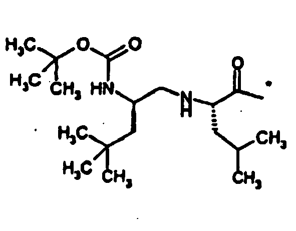 | aus Beispiel 3A und Beispiel 21A. HPLC (Methode 15): Rₜ = 4.42 min. LC/MS: (Methode 17):Rₜ = 1.85 min.; ES⁺: 1377 [M+H]⁺; 689.4 [M+2H]²⁺; ES⁻: 1375 [M-H]⁻. HR-MS: [M+H]⁺ gefunden: 1376.8147 berechnet: 1376.8153 |
| **54A** | *N-*[(*2R*)-2-Benzyloxycarbony-lamino-4-methyl-pent-1-yl]-des(1-D-leucin)-lysobactin-bis-hydrotrifluoracetat | 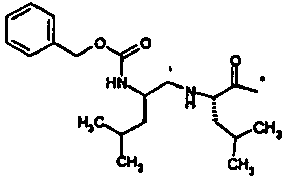 | aus Beispiel 3A und Beispiel 19A. HPLC (Methode 15): Rₜ = 3.88 min. LC/MS: (Methode 17): Rₜ = 1.93 min.; ES⁺: 1397 [M+H]⁺; ES⁻: 1395 [M-H]⁻ |

### Kupplung der Desoxodipentidsäure an Beispiel 5A

Nach der allgemeinen Arbeitsvorschrift 6 werden aus Beispiel 5A folgende Beispiele 55A bis 67A hergestellt. Die Ausbeute wird nicht auf dieser Stufe, sondern erst nach der Abspaltung der N-terminalen Schutzgruppe bestimmt.

| | | | |
|---|---|---|---|
| 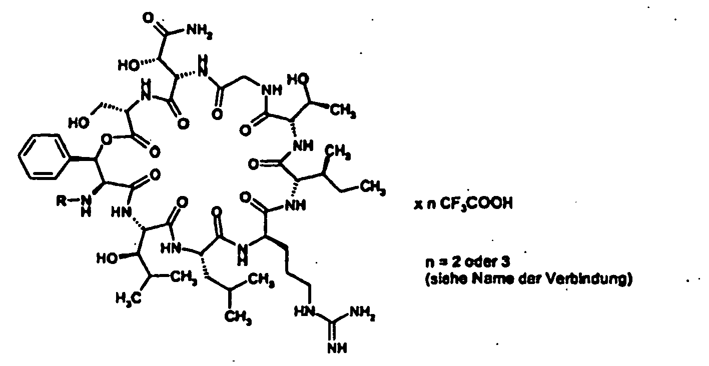 | | | |

| **Bsp-Nr.** | **Name** | **R** | **Ausgangsverbindungen / Analytik** |
|---|---|---|---|
| **55A** | *N-*{*N-*[(*2S*)-2*-tert-*Butoxycarbonylamino-4,4-dimethyl-pent-1-yl]-(β-3-pyridyl-Ala)}-des(1-D-leucin-2-leucin)-lysobactin-tris-hydrotrifluoracetat | 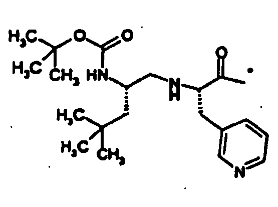 | aus Beispiel 5A und Beispiel 42A. HPLC (Methode 19): Rₜ = 3.67 min. LC/MS: (Methode 17): Rₜ = 1.78 min.; ES⁺: 706.9 [M+2H]²⁺; ES⁻: 1410 [M-H]⁻ |
| **56A** | *N-*{*N-*[(*2R*)-2*-tert-*butoxycarbonylamino-3-trimethylsilyl-prop-1-yl]}-des-(1-D-leucin)-lysobactin-bis-hydrotrifluoracetat | 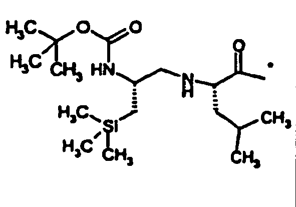 | aus Beispiel 5A und Beispiel 50A. LC/MS: (Methode 17): Rₜ =1.95 min.; ES⁺: 1393 [M+H]⁺, 697.3 [M+2H]²⁺; ES⁻: 1391 [M-H]⁻ |
| **57A** | *N-*{*N-*[(*1R*)-2-*tert*-Butoxycarbonylamino-3-trimethylsilyl-prop-1-yl]-[β-(3-pyridyl)-Ala]}-des-(1-D-leucin-2-leucin)-lysobactin-tris-hydrotrifluoracetat | 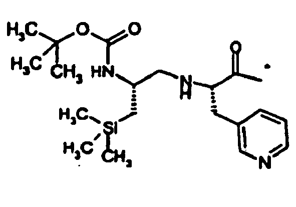 | aus Beispiel 5A und Beispiel 45A. HPLC (Methode 19): Rₜ = 3.72 min. (nicht charakterisiert, direkt weiter umgesetzt zu Beispiel 6) |
| **58A** | *N*-{*N-*[(*2S*)-2-Benzyloxycarbonyla-mino-4,4-dimethyl-pent-1-yl]-β-tBu-Ala}-des(1-D-leucin-2-leucin)-lysobactin-bis-hydrotrifluoracetat | 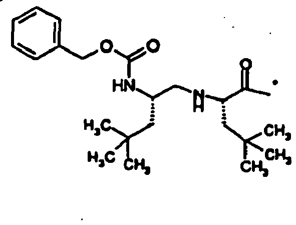 | Aus Beispiel 5A und Beispiel 39A. HPLC (Methode 19): Rₜ = 3.91 min. LC/MS: (Methode 17): Rₜ = 1.96 min.; ES⁺: 713.4 [M+H]⁺; ES⁻: 1423 [M-H]⁻. |
| **59A** | *N-*{*N-*[(*2R*)-*2*-Benzyloxycarbonylamino-4,4-dimethylmino-4,4-dimethyl-pent-1-yl]-β-tBu-Ala}-des(1-D-leucin-2-leucin)-lysobactin-bis-hydrotrifluoracetat | 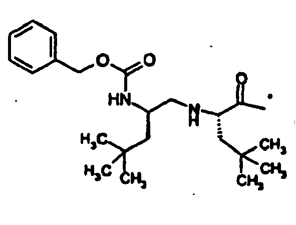 | Aus Beispiel 5A und Beispiel 38A. LC/MS: (Methode 17): Rₜ=2.01 min.; ES⁺: 713.2 [M+2H]²⁺; ES⁻: 1423 [M-H]⁻. |
| **60A** | *N-*{*N-*[(*2S*)-2-Benzyloxycarbonyla-mino-4-methyl-pent-1-yl]-[β-(3,4-dimethoxyphenyl Ala]}-des(1-D-leucin-2-leucin)-lysobactin-bis-hydrotrifluoracetat | 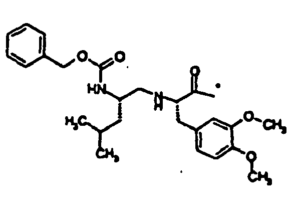 | Aus Beispiel 5A und Beispiel 43A. LC/MS: (Methode 17): Rₜ = 1.96 min.; ES⁴⁺: 746.4 [M+2H]²⁺; ES⁻: 1490 [M-H]⁻. |
| **61A** | *N-*{*N-*[(*2R*)-2-Benzyloxycarbonyla-mino-4-methyl-pent-t-yl]-[β-(3,4-dimetlioxyphenyl)-Ala]}-des(1-D-leucin-2-leucin)-lysobactin-bis-hydrotrifluoracetat | 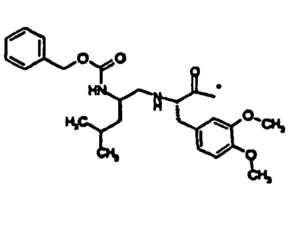 | Aus Beispiel 5A und Beispiel 44A. LC/MS: (Methode 17): Rₜ = 1.95 min.; ES⁺: 746.4 [M+2H]²⁺; ES⁻: 1490 [M-H]⁻. |
| **62A** | *N-*{*N-*[(2*S*)-*2*-Benzyloxycarbonylamino-4-methyl-pent-1-yl]-[β-(3-pyridyl)-Ala]}-des(1-D-leucin-2-leucin)-lysobactin-tris-hydrotrifluoracetat | 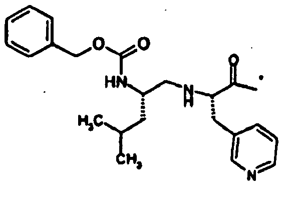 | aus Beispiel 5A und Beispiel 41A. HPLC (Methode 19): Rₜ= 3.63 min. LC/MS: (Methode 17): Rₜ = 1.74 min.; ES⁺: 716.8 [M+2H]²⁺; ES⁻: 1430 [M-H]⁻. |
| **63A** | *N*-{*N-*[(*2R*)-*2*-Benzyloxycarbonylamino-4-methyl-pent-1-yl]-(β-3-pyridyl-Ala)}-des(1-D-leucin-2-leucin)-lysobactin-tris-hydrotrifluoracetat | 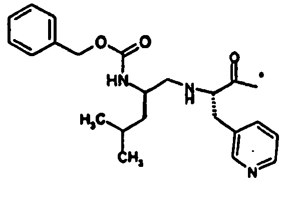 | aus Beispiel 5A und Beispiel 40A. HPLC (Methode 19): Rₜ = 3.63 min. LC/MS: (Methode 17): Rₜ = 1.67 min.; ES⁺: 716.7 [M+2H]⁺; ES⁻: 1430 [M-H]⁻. |
| **64A** | *N-*{*N-*[(*2R*)-2-Benzyloxycarbonylamino-3-trimethylsilyl-prop-1-yl]-(β-trimethylsilyl-Ala)}-des(1-D-leucin-2-leucin)-lysobactin-bis-hydrotrifluoracetat | 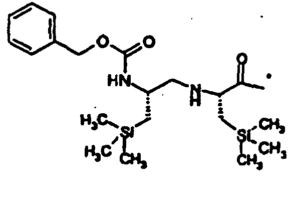 | Aus Beispiel 5A und Beispiel 48A. LC/MS: (Methode 14): Rₜ= 1.95 min.; ES⁺: 1458 [M+H]⁺, 729.4 [M+2H]²⁺; ES⁻: 1456 [M-H]⁻. |
| **65A** | *N-*{*N*-[(*2R*)-2-Benzyloxycarbonylamino-3-trimethylsilyl-prop-1-yl-(β-tBu-Ala)}-des(1-D-leucin-2-leucin)-lysobactin-bis-hydrotrifluoracetat | 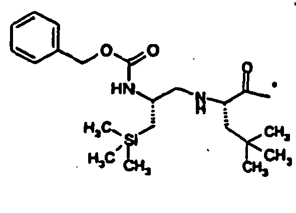 | Aus Beispiel 5A und Beispiel 46A. LC/MS: (Methode 17): Rₜ = 1.90 min.; ES⁺: 1441 [M+H]⁺, 721.3 [M+2H]²⁺; ES⁻: 1439 [M-H]⁻. |
| **66A** | *N*-{*N-*[(*2S*)-2-Benzyloxycarbonylamino-4,4-dimethyl-pent-1-yl]-(β-trimethylsilyl-Ala)}-des(1-D-leucin-2-leucin)-lysobactin-bis-hydrotrifluoracetat | 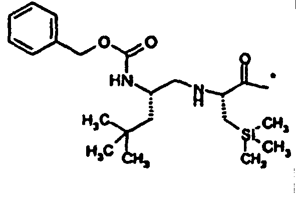 | aus Beispiel 5A und Beispiel 47A. LC/MS: (Methode 17): Rₜ = 1.96 min.; ES⁺: 1441 [M+H]⁺, 721.2 [M+2H]²⁺; ES⁻: 1439 [M-H]'. |
| **67A** | *N-*{*N-*[(*2S*)-2-benzyloxycarbonylamino-4,4-dimethyl-pent-1-yl]-Asp(tBu)-des(1-D-leucin-2-leucin)-lysobactin-bis-hydrotrifluoracetat | 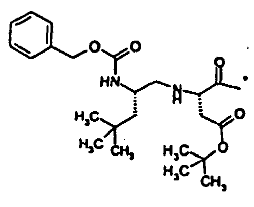 | Aus Beispiel 5A und Beispiel 49A. LC/MS: (Methode 17): Rₜ = 2.13 min.; ES⁺: -1469 [M+H]⁺, 735.3 [M+2H]²⁺; ES⁻: 1467 [M-H]⁻. |

### Beispiel 68A

### (2RS, 3S)-3-Benzyloxycarbonylamino-5,5-dimethyl-2-hydroxy-hexan

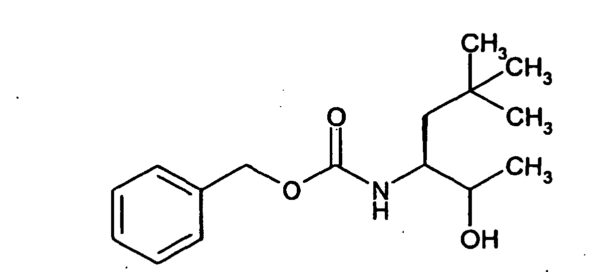

635 µl (0.89 mmol) einer Lösung von Methylmagnesiumbromid (1.4N in Toluol:THF 3:1) werden unter Argon bei RT mit einer Lösung von 180 mg (0.68 mmol) der Verbindung aus Beispiel 16A in 1 ml wasserfreiem Diethylether tropfenweise versetzt. Danach wird die Reaktionsmischung 2 h in einem warmen Ölbad (50°C) erhitzt, abgekühlt und mit einem Eiswürfel gequencht. Es wird mit 1N Salzsäure angesäuert und dreimal mit Essigsäureethylester extrahiert. Die vereinigten organischen Phasen werden über Natriumsulfat getrocknet und am Rotavapor eingeengt. Der Rückstand wird durch präparative HPLC (Methode 25) gereinigt. Man erhält 50 mg (20% d. Th.) der Titelverbindung.
LC-MS (Methode 17): Rₜ = 2.34 min. MS (ES⁺): m/z = 280.3 [M+H]⁺.

### Beispiel 69A

### (3S)-3-Benzyloxycarbonylamino-5,5-dimethyl-2-oxo-hexan

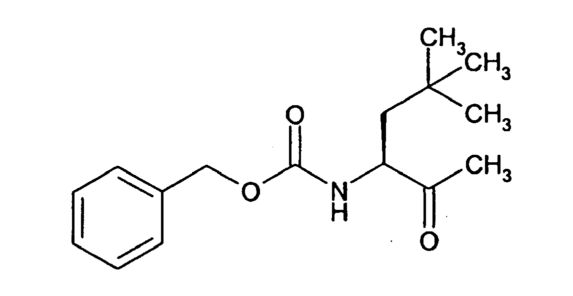

Eine Lösung der Verbindung aus Beispiel 68A (50 mg, 0.18 mmol) und N,N-Diisopropylethylamin (310 µl, 1.79 mmol) in 2 ml DMSO wird bei 10°C mit Pyridin-SO₃-Komplex (285 mg, 1.79 mmol) versetzt und langsam auf RT erwärmt. Nach 2 h werden zur Reaktionsmischung 50 ml Eis/Wasser zugegeben. Man extrahiert viermal mit Diethylether. Die vereinigten organischen Phasen werden dreimal mit einer 10%-igen Zitronensäure-Lösung, dann dreimal mit Wasser und zweimal mit ges. Natriumchlorid-Lösung gewaschen, über Magnesiumsulfat getrocknet und bei 20°C am Rotavapor eingeengt. Das Rohprodukt (30 mg, 60% d. Th.) wird ohne zusätzliche Reinigung weiter umgesetzt.
LC-MS (Methode 14): Rₜ = 2.26 min. MS (ES⁺): m/z = 278.4 [M+H]⁺.
¹H-NMR (CDCl₃, 300 MHz): δ = 7.40-7.28 (m, 5H), 5.15- 5.06 (m, 3H), 4.40 (dt, J = 2.3, 9.1 Hz, 1H), 2.21 (s, 3H), 1.68 (dd, J = 2.3, 14.7 Hz, 1H), 1.24 (dd, J = 9.1, 14.7 Hz, 1H), 0.99 (s, 9H).

### Beispiel 70A

### N-{(2RS,3S)-2-{[(Benzyloxy)carbonyl]amino}-5,5-dimethyl-hex-2-yl}-4-methyl-L-leucinmethylester

### (Z-L-β-tert-Butyl-Ala(ΨCHMeNH)-L-β-tert-Butyl-Ala-OMe)

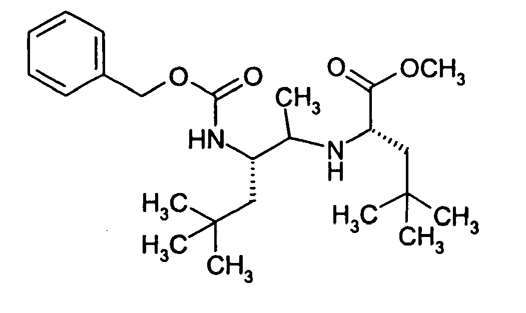

Die Titelverbindung wird nach der allgemeinen Arbeitsvorschrift 3 aus der Verbindung aus Beispiel 69A (27 mg) und der Verbindung aus Beispiel 24A (29 mg) hergestellt. Ausbeute 6 mg (13% d. Th.).
LC-MS (Methode 17): Rₜ = 2.29 min. MS (ES⁺): m/z = 421.2 [M+H]⁺.

### Beispiel 71A

### N-{(2RS,3S)-2-{[(Benzyloxy)carbonyl]amino}-5,5-dimethyl-hex-2-yl}-4-methyl-L-leucin

### (Z-L-β-tert-Butyl-Ala(ΨCHMeNH)-L-β-tert-Butyl-Ala-OH)

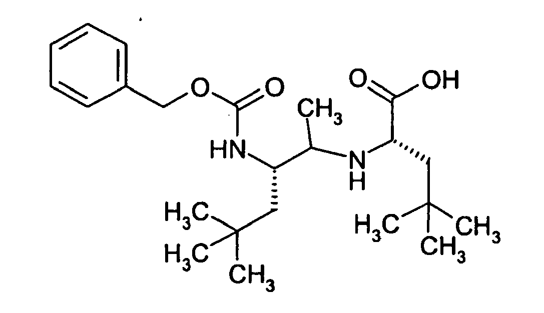

Die Titelverbindung wird nach der allgemeinen Arbeitsvorschrift 4 aus der Verbindung aus Beispiel 70A (6 mg) hergestellt. Ausbeute 3 mg (50% d. Th.). Die analytischen Daten deuten auf ein Diastereomer hin.
LC-MS (Methode 17): Rₜ = 2.11 min. MS (ES⁺): m/z = 407.3 [M+H]⁺, (ES⁻): m/z = 405.2 [M-H]⁻.
¹H-NMR (DMSO-d₆, 400 MHz): δ = 7.38-7.25 (m, 5H), 7.20 (d, J = 9.1 Hz, 1H), 5.04 und 4.98 (jeweils d, [AB-System], J = 12.7, jeweils 1H), 3.51 (m, 1H), 3.18 (t, J = 6.2 Hz, 1H), 2.53 (m, 1H), 2.50 (s, 3H), 1.51 (dd, J = 3.6, 13.9 Hz, 1H), 1.34-1.23 (m, 3H), 0.92 (s, 9H), ca.0.9 (3H), 0.84 (s, 9H).

### Ausführungsbeispile

Nach der allgemeinen Arbeitsvorschrift 7 werden die Beispiele 1 bis 6 hergestellt:

| | | | |
|---|---|---|---|
| 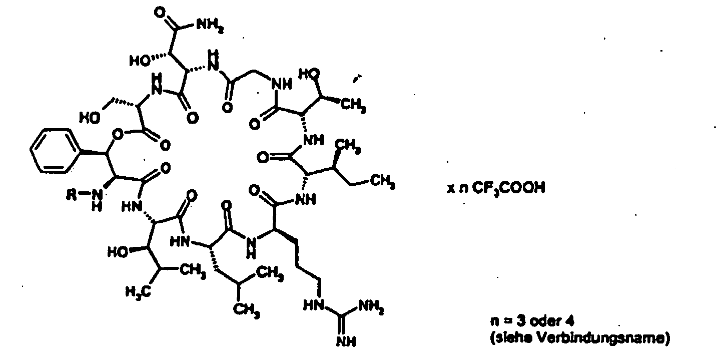 | | | |

| **Bsp-Nr.** | **Name** | **R** | **Ausbeute/Analytik (Ausbeute ausgehend von Beispiel 3A oder 5A)** |
|---|---|---|---|
| **1** | *N*-[(*2S*)-2-Amino-4-methyl-pent-1-yl]-des(1-D-leucin)-lysobactin-tris-hydrotrifluoracetat | 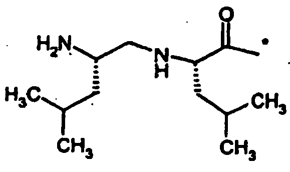 | aus Beispiel 51A; 46.6 mg (17% d. Th.) HPLC (Methode 15): Rₜ = 3.44 min. LC/MS: (Methode 17): Rₜ = 1.47 min.; ES⁺: 1263 [M+H]⁺; ES⁻: 1261 [M-H]⁻. HR-MS: [M+H]⁺ gefunden: 1262.7522 berechnet: 1262.7472 Bestätigung durch Maldi (Methode 20) von der nach Methode 9 behandelten Verbindung. |
| **2** | *N*-[(*2S*)-2-Amino-4,4-dimethyl-pent-1-yl]-des(1-D-leucin)-lysobactin-tris-hydrotrifluoracetat | 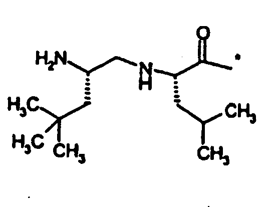 | aus Beispiel 52A; 52 mg (29% d. Th.) HPLC (Methode 15): Rₜ = 3.95 min. LC/MS: (Methode 17): Rₜ = 1.48 min.; ES⁺: 1277 [M+H]⁺; ES⁻: 1275 [M-H]⁻. HR-MS: [M+H]⁺ gefunden: 1276.7631 berechnet: 1276.7629 |
| **3** | *N*-[(*2R*)-2-Amino-4,4-dimethyl-pent-1-yl]-des(1-D-leucin)-lysobactin-tris-hydrotrifluoracetat | 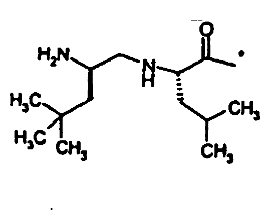 | aus Beispiel 53A; 44 mg (23% d. Th.) HPLC (Methode 15): Rₜ = 3.87 min. LC/MS: (Methode 17):Rₜ = 1.53 min.; ES⁺: 1277 [M+H]⁺; ES⁻: 1275 [M-H]⁻. HR-MS: [M+H]⁺ gefunden: 1276.7622 berechnet: 1276.7629 |
| **4** | *N-*{*N-*[(*2S*)*-*2- Amino-4,4-dimethyl-pent-1-yl]-(-β-3-pyridyl-Ala)}-des(1-D-leucin-2-leucin)-lysobactin-tetrahydrotrifluoracetat | 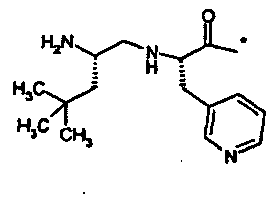 | aus Beispiel 55A; 81 mg (43% d. Th.) HPLC (Methode 15): Rₜ = 3.87 min. LC/MS: (Methode 17): Rₜ = 1.35 min.; ES⁺: 656.8 [M+2H]²⁺; ES⁻: 1310 [M-H]⁻. HR-MS: [M+H]⁺ gefunden: 1311.7446 berechnet: 1311.7425 |
| **5** | *N*-[(*2R*)-2-Amino-3-trimethylsilyl-prop-1-yl]-des-(1-D-leucin)-lysobactin-tris-hydrotrifluoracetat | 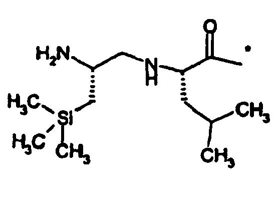 | aus Beispiel 56A, 117 mg (34% d. Th.). HPLC (Methode 15): Rₜ = 3.92 min. LC/MS: (Methode 17): Rₜ = 1.51 min.; ES⁺: 647.3 [M+2H]²⁺; ES⁻: 1291 [M-H]⁻ HR-MS: [M+H]⁺ gefunden: 1292.7382 berechnet: 1292.7398 |
| **6** | *N-*{*N-*[(*2R*)*-2-*Amino-3-trimethylsilyl-prop-1-yl]-[β-(3-pyridyl)-Ala]}-des-(1-D-leucin-2-leucin)-lysobactin-tetra-hydrotrifluoracetat | 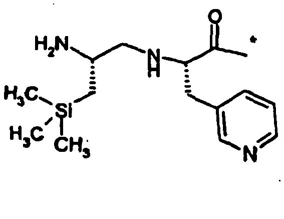 | aus Beispiel 57A, 31 mg (49% d. Th.). HPLC (Methode 15): Rₜ = 3.98 min. LC/MS: (Methode 17): Rₜ = 1.33 min.; ES⁺: 1328 [M+H]⁺, 664.7 [M+2H]²⁺; ES⁻: 1326 [M-H]⁻. HR-MS: [M+H]⁺ gefunden: 1327.7228 berechnet: 1327.7194 |
| **7** | *N*-[(*2R*)-2-Amino-4-methyl-pent-1-yl]-des(1-D-leucin)-lysobactin-tris-hydrotrifluoracetat | 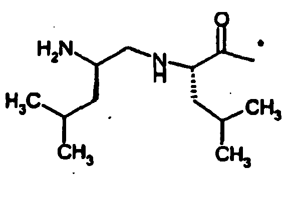 | aus Beispiel 54A, 34 mg (16% d. Th.). HPLC (Methode 15): Rₜ = 3.47 min. LC/MS: (Methode 17): Rₜ = 1.55 min.; ES⁺: 1263 [M+H]⁺; ES⁻: 1261 [M-H]⁻. HR-MS: [M+H]⁺ gefunden: 1262.7479 berechnet: 1272.7472 |
| **8** | *N-*{*N-*[(*2S*)-2-Amino-4,4-dimethyl-pent-1-yl]-β-tBu-Ala-des(1-D-leucin-2-leucin)-lysobactin-tris-hydrotrifluoracetat | 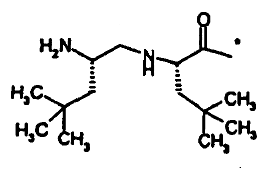 | aus Beispiel 58A, 41 mg (28% d. Th.). HPLC (Methode 15): Rₜ= 4.00 min. LC/MS: (Methode 17): Rₜ = 1.55 min.; ES⁺: 1291 [M+H]⁺; ES⁻: 1290 [M-H]⁻. HR-MS: [M+H]⁺ gefunden: 1290.7799 berechnet: 1290.7785 Bestätigung durch Maldi (Methode 20) von der nach Methode 9 behandelten Verbindung |
| **9** | *N-*{*N-*[(*2R*)-2-Amino-4,4-dimethyl-pent-1-yl]-β-tBu-Ala-des(1-D-leucin-2-leucin)-lysobactin-tris-hydrotrifluoracetat | 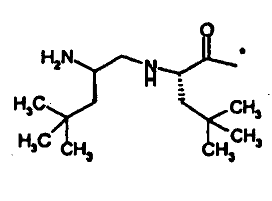 | aus Beispiel 59A, 105 mg (47% d. Th.). HPLC (Methode 15): Rₜ=3.91 min. LC/MS: (Methode 17):Rₜ = 1.61 min.; ES⁺: 646.3 [M+2H]²⁺; ES⁻: 1290 [M-H]⁻. HR-MS: [M+H]⁺ gefunden: 1290.7803 berechnet: 1290.7785 |
| **10** | *N-*{*N-*[(*2S*)-2-Amino-4-methyl-pent-1-yl]-(β-3,4-dimethoxyphenyl-Ala)-des(1-D-leucin-2-leucin)-lysobactin-tris-hydrotrifluoracetat | 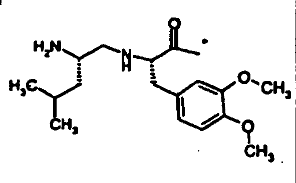 | aus Beispiel 60A, 5 mg (13% d. Th.). LC/MS: (Methode 14): Rₜ = 1.23 min.; ES⁺: 1356.7 [M+H]⁺; ES⁻: 1355 [M-H]⁻, 1400.9 [M-H+HCOOH]⁻. HR-MS: [M+H]⁺ gefunden: 1356.7549 berechnet: 1356.7527 |
| **11** | *N-*{*N*-[(*2R*)-2-Amino-4-methyl-pent-1-yl]-(β-3,4-dimethoxyphenyl-Ala)-des(1-D-leucin-2-leucin)-lysobactin-tris-hydrotrifluoracetat | 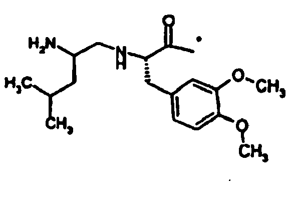 | aus Beispiel 61A, 5 mg (33% d. Th.) LC/MS: (Methode 17): Rₜ = 1.52 min.; ES⁺: 1356 [M+H]⁺, 679.3 [M+2H]²⁺; ES⁻: 1355[M-H]⁻. HR-MS: [M+H]⁺ gefunden: 1356.7512 berechnet: 1356.7527 |
| **12** | *N-*{*N-*[(*2S*)-2-Amino-4-methyl-pent-1-yl]-(β-3-pyridyl-Ala)}-des(1-D-leucin-2-leucin)-lysobactin-tetrahydrotrifluoracetat | 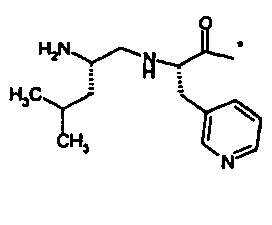 | Aus Beispiel 62A, 61 mg (22% d. Th.) HPLC (Methode 15): Rₜ = 3.30 min. LC/MS: (Methode 17): Rₜ = 1.23 min.; ES⁺: 648.8 [M+2H]²⁺; ES⁻: 1296 [M-H]⁻, 1342 [M-H+HCOOH]⁻. HR-MS: [M+H]⁺ gefunden: 1297.7244 berechnet: 1297.7268 |
| **13** | *N-*{*N*-[(*2R*)-2-Amino-4-methyl-pent-t-yl]-(β-3-pyridyl-Ala)}-des(1-D-leucin-2-leucin)-lysobactin-tetra-hydrotrifluoracetat | 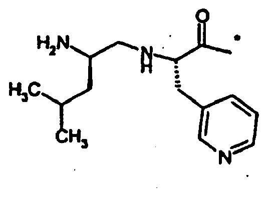 | aus Beispiel 63A, 60 mg (19% d. Th.). LC/MS: (Methode 17): Rₜ = 1.25 min.; ES⁺: 649.7 [M+2H]²⁺; ES': 1296 [M-H]⁻, 1342 [M-H+HCOOH]⁻. HR-MS: [M+H]⁺ gefunden: 1297.7301 berechnet: 1297.7268 |
| **14** | *N-*{*N-*[(2*R*)-2-Amino-3-trimethylsilyl-prop-1-yl]-(β-trimethylsilyl-Ala)}-des(1-D-leucin-2-leucin)-lysobactin-tris-hydrotrifluoracetat | 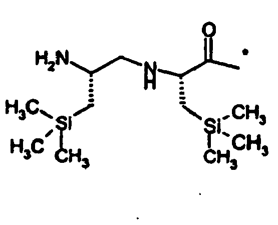 | aus Beispiel 64A, 8 mg (12% d. Th.). LC/MS: (Methode 17): Rₜ = 1.74 min.; ES⁺: 662.3 [M+2H]²⁺; ES⁻: 1321 [M-H]⁻. HR-MS: [M+H]⁺ gefunden: 1322.7292 berechnet: 1322.7324 |
| **15** | *N-*{*N-*[(*2R*)-2-Amino-3-tri-methylsilyl-prop-1-yl]-(β-tBu-Ala)}-des(1-D-leucin-2-leucin)-lysobactin-tris-hydrotrifluoracetat | 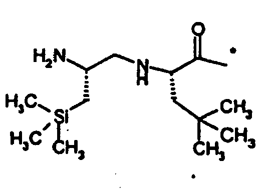 | aus Beispiel 65A, 17 mg (35% d. Th.). HPLC (Methode 15):Rₜ= 3.94 min. LC/MS: (Methode 17): Rₜ = 1.61 min.; ES⁺: 654.3 [M+2H]²⁺; ES⁻: 1306 [M-H]⁻, 1352 [M-H+HCOOH]⁻ HR-MS: [M+H]⁺ gefunden: 1306.7526 berechnet: 1306.7555 |
| **16** | *N-*{*N-*[(*2S*)-2-Amino-4,4-dimethyl-pent-1-yl)-(β-trimethylsilyl-Ala)}-des(1-D-leucin-2-leucin)-lysobactin-tris-hydrotrifluoracetat | 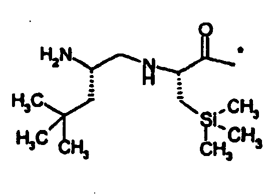 | aus Beispiel 66A, 24 mg (25% d. Th). HPLC (Methode 15): Rₜ = 3.94 min. LC/MS: (Methode 17): Rₜ = 1.56 min.; ES⁺: 654.3 [M+2H]²⁺; ES⁻: 1305 [M-H]⁻, 1351 [M-H+HCOOH]⁻. HR-MS: [M+H]⁺ gefunden: 1306.7593 berechnet: 1306.7555 |
| **17** | *N-*{*N-*[(*2S*)-2-Amino-4,4-dimethyl-pent-1-yl]-Asp(tBu)-des(1-D-leucin-2-leucin)-lysobactin-tris-hydrotrifluoracetat | 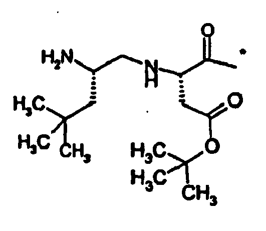 | aus Beispiel 67A, 43 mg (37% d. Th.). HPLC (Methode 15): Rₜ = 3.97 min. LC/MS: (Methode 17): Rₜ = 1.60 min.; ES⁺: 668.3 [M+2H]²⁺; ES⁻: 1333 [M-H]⁻, 1379 [M-H+HCOOH]⁻. HR-MS: [M+H]⁺ gefunden: 1334.7729 berechnet: 1334.7684 |

### Beispiel 18

### N-{N-[(2RS,3S)-3-Amino-5,5-dimethyl-pent-2-yl]-β-tBu-Ala-des(1-D-leucin-2-leucin)-lysobactin-tris-hydrotrifluoracetat

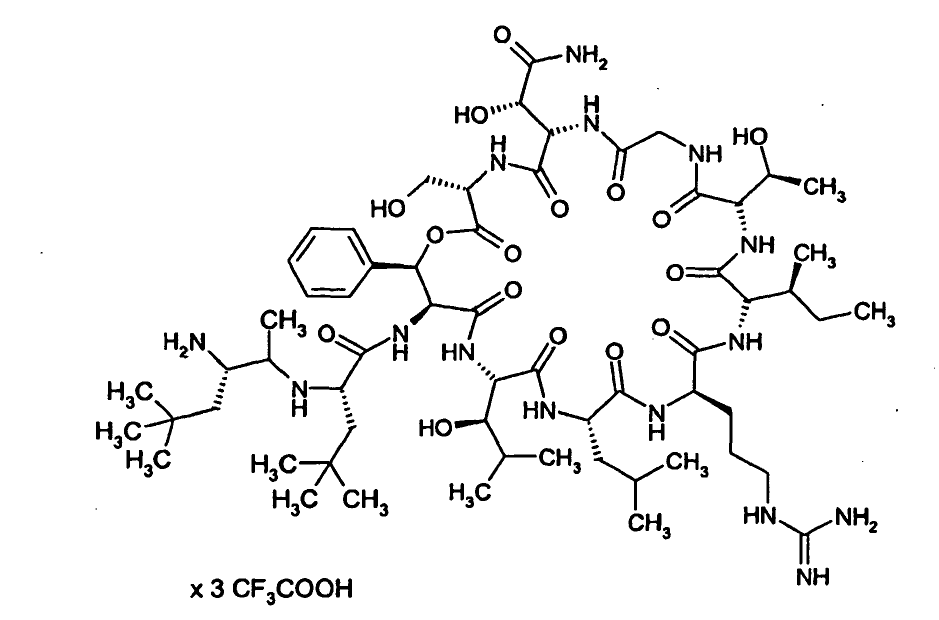

5.7 mg (4.4 µmol) der Verbindung aus Beispiel 5A und 3.0 mg (6.6 µmol) der Verbindung aus Beispiel 71A werden in 0.5 ml DMSO unter Argon vorgelegt. 20 µl (4.5 Äq.) N-Methylmorpholin, 2.7 mg (1.6 Äq) HATU und nach 15 min noch einmal 20 µl (4.5 Äq.) N-Methylmorpholin werden zugegeben. Die Mischung wird über Nacht bei RT gerührt und dann direkt über die präparative HPLC (Methode 7) gereinigt. Die Kontrolle der Fraktionen erfolgt durch LC-MS (Methode 17). Nach Lyophilisierung der geeigneten Fraktionen (Rₜ = 2.02, ES (pos): m/z = 1440 [M+H]⁺) wird die N-Benzyloxycarbonyl-geschützte Zwischenstufe sofort in 200 µl Isopropanol mit 2 mg Palladium (10% auf Kohle) unter 1 atm Wasserstoff hydriert. Nach 4 h wird die Mischung filtriert, eingeengt und über die präparative HPLC gereinigt (Methode 10). Man erhält 0.5 mg (7% d. Th.) der Titelverbindung.
LC-MS (Methode 17): Rₜ = 1.53 min. ES⁺: m/z = 1290.7 [M+H]⁺, 646 [M+2H]²⁺, ES⁻: m/z = 1288.7 [M-H]⁻, 644 [M-2H]⁻.

### B. Bewertung der physiologischen Wirksamkeit

Die *in vitro*-Wirkung der erfindungsgemäßen Verbindungen kann in folgenden Assays gezeigt werden:

### Bestimmung der minimalen Hemmkonzentration (MHK):

Die MHK wird im Flüssigdilutionstest gemäß der NCCLS-Richtlinien bestimmt. Übernachtkulturen von *Staphylococcus aureus* 133, *Entercococcus faecalis* 27159, *E. faecium* 4147 und *Streptococcus pneumoniae* G9a werden mit den beschriebenen Testsubstanzen in einer 1:2 Verdünnungsreihe inkubiert. Die MHK-Bestimmung wird mit einer Zellzahl von 10⁵ Keimen pro ml in Isosensitest-Medium (Fa. Difco, Irvine/USA) durchgeführt, mit Ausnahme von *S. pneumoniae,* der in BHI-Bouillon (Fa. Difco, Irvine/USA) mit 10% Rinderserum bei einer Zellzahl von 10⁶ Keimen pro ml getestet wird. Die Kulturen werden bei 37°C für 18-24 Stunden inkubiert, *S. pneumoniae* in Gegenwart von 10% CO₂.

Die jeweils niedrigste Substanzkonzentration, bei der kein sichtbares Bakterienwachstum mehr auftritt, wird als MHK definiert. Die MHK-Werte werden in µg/ml angegeben.

Repräsentative in-vitro-Wirkdaten für die erfindungsgemäßen Verbindungen sind in Tabelle A wiedergegeben:

**Tabelle A**

| Beispiel Nr. | MHK *S. aureus* 133 | MHK *S. pneumoniae* | MHK *E. faecalis ICB 27159* |
|---|---|---|---|
| 5 | 0.5 | 0.063 | 1 |
| 8 | 0.5 | 0.063 | 1 |
| 14 | 0.25 | 0.0156 | 0.125 |
| 15 | 0.5 | 0.063 | 1 |

Die Eignung der erfindungsgemäßen Verbindungen zur Behandlung von bakteriellen Infektionen kann im folgenden Tiermodell gezeigt werden:

### Systemische Infektion mit Staphylococcus aureus 133:

Zellen von *S*. *aureus* 133 werden über Nacht in BHI-Bouillon (Fa. Oxoid, New York/ USA) angezüchtet. Die Übemachtkultur wird 1:100 in frische BHI-Bouillon verdünnt und für 3 Stunden inkubiert. Die dann in der logarithmischen Wachstumsphase befindlichen Zellen werden abzentrifugiert und zweimal mit gepufferter, physiologischer Kochsalzlösung gewaschen. Danach wird photometrisch eine Zellsuspension in Kochsalzlösung mit einer Extinktion von 50 Einheiten eingestellt. Nach einem Verdünnungsschritt (1:15) wird diese Suspension 1:1 mit einer 10%igen Mucinlösung gemischt. Von dieser Infektionslösung werden 0.25 ml/20 g Maus intraperitoneal (entsprechend 1x10⁶ Keimen/Maus) appliziert. Die Therapie erfolgt intraperitoneal oder intravenös 30 Minuten nach der Infektion. Für den Infektionsversuch werden weibliche CFWI-Mäuse verwendet. Das Überleben der Tiere wird über 6 Tage protokolliert.

**Die Löslichkeit** einer Verbindung wird nach dem Fachmann bekannten Methoden bestimmt.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der Verbindung von Beispiel 1, 50 mg Lactose (Monohydrat), 50 mg Maisstärke (nativ), 10 mg Polyvinylpyrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus Wirkstoff, Lactose und Stärke wird mit einer 5%-igen Lösung (m/m) des PVPs in Wasser granuliert. Das Granulat wird nach dem Trocknen mit dem Magnesiumstearat für 5 min gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der Verbindung von Beispiel 1, 1000 mg Ethanol (96%), 400 mg Rhodigel (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, der Wirkstoff wird der Suspension zugefügt. Unter Rühren erfolgt die Zugabe des Wassers. Bis zum Abschluss der Quellung des Rhodigels wird ca. 6h gerührt.

### Intravenös applizierbare Lösung:

### Zusammensetzung:

100-200 mg der Verbindung von Beispiel 1, 15 g Polyethylenglykol 400 und 250 g Wasser für Injektionszwecke.

### Herstellung:

Die Verbindung von Beispiel 1 wird zusammen mit Polyethylenglykol 400 in dem Wasser unter Rühren gelöst. Die Lösung wird sterilfiltriert (Porendurchmesser 0.22 µm) und unter aseptischen Bedingungen in hitzesterilisierte Infusionsflaschen abgefüllt. Diese werden mit Infusionsstopfen und Bördelkappen verschlossen.

## Patentansprüche

1. Verbindung der Formel 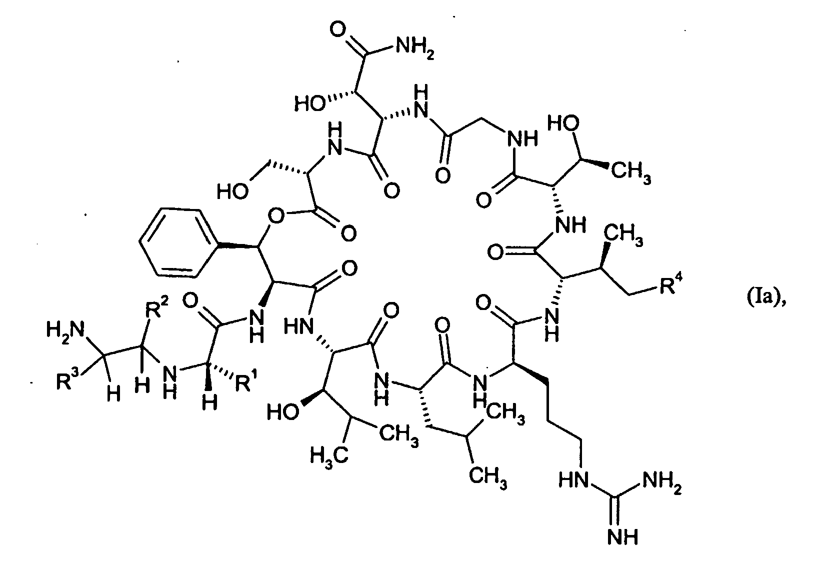 in welcher
R¹ C₁-C₂-Alkyl bedeutet,
wobei C₁-C₂-Alkyl substituiert ist mit einem Substituenten ausgewählt aus der Gruppe bestehend aus Trimethylsilyl, Alkoxycarbonyl, C₁-C₆-Cycloalkyl, Alkoxy substituiertes Phenyl, 2-Pyridyl und 3-Pyridyl,
oder
C₄-C₈-Alkyl bedeutet,
R² Wasserstoff oder C₁-C₄-Alkyl bedeutet,
R³ C₁-C₂-Alkyl bedeutet,
wobei C₁-C₂-Alkyl substituiert ist mit einem Substituenten Trimethylsilyl,
oder
C₄-C₆-Alkyl bedeutet,
R⁴ Wasserstoff oder Methyl bedeutet,
oder eines ihrer Salze, ihrer Solvate oder der Solvate ihrer Salze.

2. Verbindung nach Anspruch 1, **dadurch gekennzeichnet, dass** sie der Formel 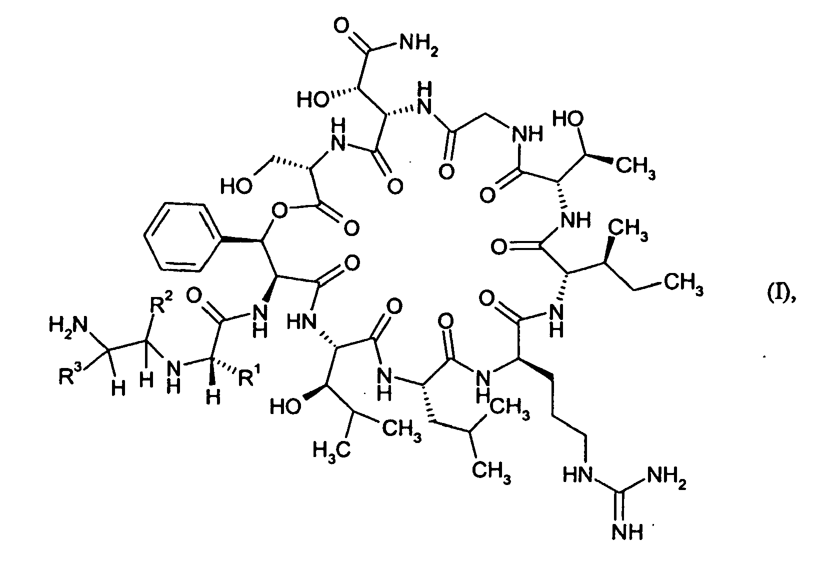 entspricht, in welcher
R¹ C₁-C₂-Alkyl bedeutet,
wobei C₁-C₂-Alkyl substituiert ist mit einem Substituenten ausgewählt aus der Gruppe bestehend aus Trimethylsilyl, Alkoxycarbonyl, C₃-C₆-Cycloalkyl, Alkoxy substituiertes Phenyl, 2-Pyridyl und 3-Pyridyl,
oder
C₄-C₈-Alkyl bedeutet,
R² Wasserstoff oder C₁-C₄-Alkyl bedeutet,
R³ C₁-C₂-Alkyl bedeutet,
wobei C₁-C₂-Alkyl substituiert ist mit einem Substituenten Trimethylsilyl,
oder
C₄-C₆-Alkyl bedeutet,
oder eines ihrer Salze, ihrer Solvate oder der Solvate ihrer Salze.

3. Verbindung nach Anspruch 2, **dadurch gekennzeichnet, dass**
R¹ C₁-C₂-Alkyl bedeutet
wobei C₁-C₂-Alkyl substituiert ist mit einem Substituenten Trimethylsilyl,
oder
C₄-C₆-Alkyl bedeutet,
R² Wasserstoff oder Methyl bedeutet,
R³ C₁-C₂-Alkyl bedeutet,
wobei C₁-C₂-Alkyl substituiert ist mit einem Substituenten Trimethylsilyl,
oder
C₄-C₆-Alkyl bedeutet,
oder eines ihrer Salze, ihrer Solvate oder der Solvate ihrer Salze.

4. Verbindung nach einem der Ansprüche 2 oder 3, **dadurch gekennzeichnet, dass**
R¹ Trimethylsilylmethyl oder 2,2-Dimethylprop-1-yl bedeutet,
R² Wasserstoff bedeutet,
R³ Trimethylsilylmethyl oder 2,2-Dimethylprop-1-yl bedeutet,
oder eines ihrer Salze, ihrer Solvate oder der Solvate ihrer Salze.

5. Verfahren zur Herstellung einer Verbindung der Formel (Ia) nach Anspruch 1, **dadurch gekennzeichnet, dass** eine Verbindung der Formel 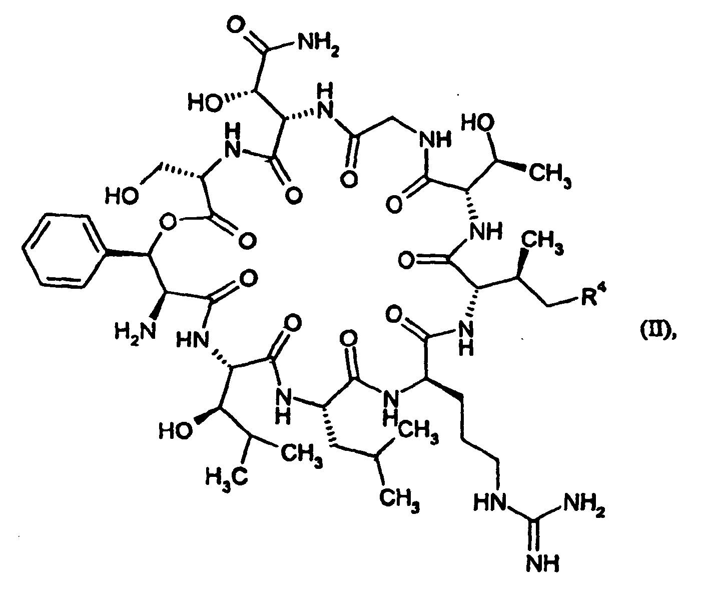 in welcher
R⁴ die in Anspruch 1 angegebene Bedeutung hat,
mit einer Verbindung der Formel 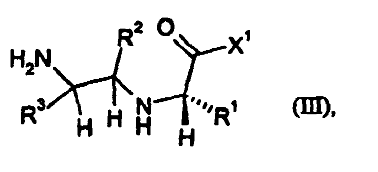 in welcher
R¹, R² und R³ die in Anspruch 1 angegebene Bedeutung haben, und
X¹ Halogen, bevorzugt Brom Chlor oder Fluor, oder Hydroxy bedeutet,
umgesetzt wird.

6. Verbindung nach einem der Ansprüche 1 bis 4 zur Behandlung und/oder Prophylaxe von Krankheiten.

7. Verwendung einer Verbindung nach einem der Ansprüche 1 bis 4 zur Herstellung eines Arzneimittels zur Behandlung und/oder Prophylaxe von bakteriellen Infektionen.

8. Arzneimittel enthaltend eine Verbindung nach einem der Ansprüche 1 bis 4 in Kombination mit einem inerten, nichttoxischen, pharmazeutisch geeigneten Hilfsstoff.

9. Arzneimittel nach Anspruch 8 zur Behandlung und/oder Prophylaxe von bakteriellen Infektionen.

10. Verwendung einer antibakteriell wirksamen Menge mindestens einer Verbindung nach einem der Ansprüche 1 bis 4, eines Arzneimittels nach Anspruch 8 oder 9 oder eines nach Anspruch 7 enthaltenen Arzneimittels, zur Bekämpfung von bakteriellen Infektionen in Menschen und Tieren.

## Claims

1. Compound of formula 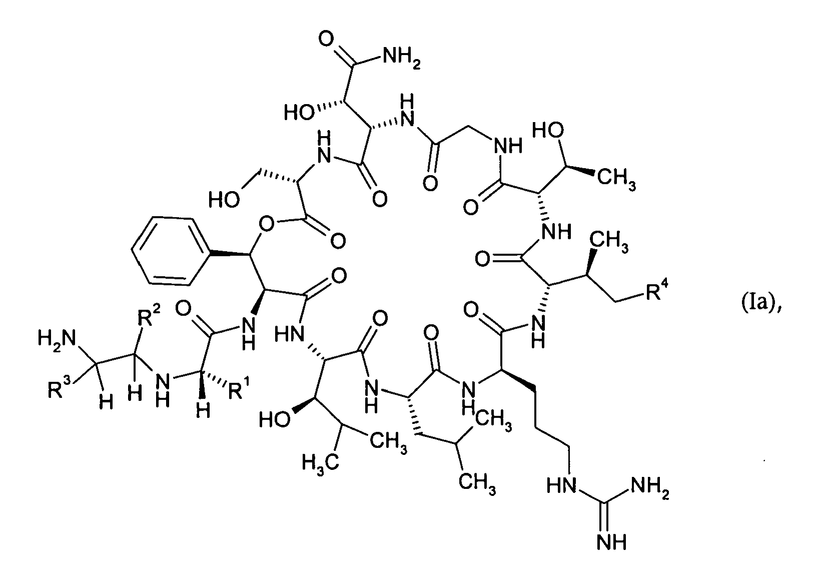 in which
R¹ represents C₁-C₂-alkyl,
whereby C₁-C₂-alkyl is substituted with a substituent selected from the group consisting of trimethylsilyl, alkoxycarbonyl, C₃-C₆-cycloalkyl, alkoxy-substituted phenyl, 2-pyridyl and 3-pyridyl,
or
represents C₄-C₈-alkyl,
R² represents hydrogen or C₁-C₄-alkyl,
R³ represents C₁-C₂-alkyl,
whereby C₁-C₂-alkyl is substituted with a substituent trimethylsilyl,
or
represents C₄-C₆-alkyl,
R⁴ represents hydrogen or methyl,
or one of its salts, its solvates or the solvates of its salts.

2. Compound according to claim 1, **characterized in that** it corresponds to formula 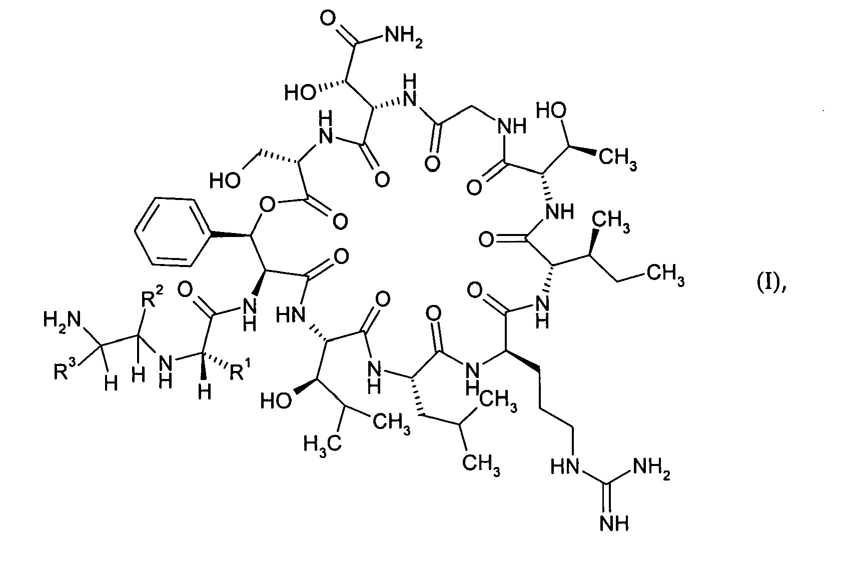 in which
R¹ represents C₁-C₂-alkyl,
whereby C₁-C₂-alkyl is substituted with a substituent selected from the group consisting of trimethylsilyl, alkoxycarbonyl, C₃-C₆-cycloalkyl, alkoxy-substituted phenyl, 2-pyridyl and 3-pyridyl,
or
represents C₄-C₈-alkyl,
R² represents hydrogen or C₁-C₄-alkyl,
R³ represents C₁-C₂-alkyl,
whereby C₁-C₂-alkyl is substituted with a substituent trimethylsilyl,
or
represents C₄-C₆-alkyl,
or one of its salts, its solvates or the solvates of its salts.

3. Compound according to claim 2, **characterized in that**
R¹ represents C₁-C₂-alkyl
whereby C₁-C₂-alkyl is substituted with a substituent trimethylsilyl,
or
represents C₄-C₆-alkyl,
R² represents hydrogen or methyl,
R³ represents C₁-C₂-alkyl,
whereby C₁-C₂-alkyl is substituted with a substituent trimethylsilyl,
or
represents C₄-C₆-alkyl,
or one of its salts, its solvates or the solvates of its salts.

4. Compound according to one of claims 2 or 3, **characterized in that**
R¹ represents trimethylsilylmethyl or 2,2-dimethylprop-1-yl,
R² represents hydrogen,
R³ represents trimethylsilylmethyl or 2,2-dimethylprop-1-yl,
or one of its salts, its solvates or the solvates of its salts.

5. Method for preparing a compound of formula (Ia) according to claim 1, **characterized in that** a compound of formula 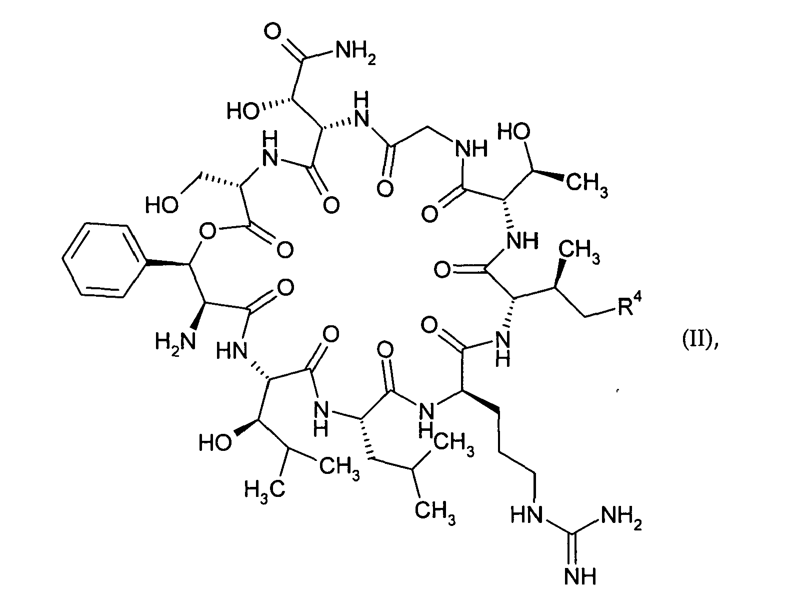 in which
R⁴ has the meaning indicated in claim 1,
is reacted with a compound of formula 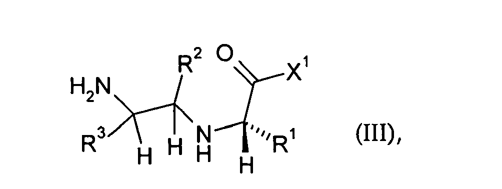 in which
R¹, R² and R³ have the meaning indicated in claim 1, and
X¹ represents halogen, preferably bromine, chlorine or fluorine, or hydroxy.

6. Compound according to one of claims 1 to 4 for the treatment and/or prophylaxis of diseases.

7. Use of a compound according to one of claims 1 to 4 for the production of a medicament for the treatment and/or prophylaxis of bacterial infections.

8. Medicament comprising a compound according to one of claims 1 to 4 in combination with an inert, non-toxic, pharmaceutically acceptable excipient.

9. Medicament according to claim 8 for the treatment and/or prophylaxis of bacterial infections.

10. Use of an antibacterially effective amount of at least one compound according to one of claims 1 to 4, of a medicament according to claim 8 or 9 or of a medicament obtained according to claim 7 for controlling bacterial infections in humans and animals.

## Revendications

1. Composé de formule 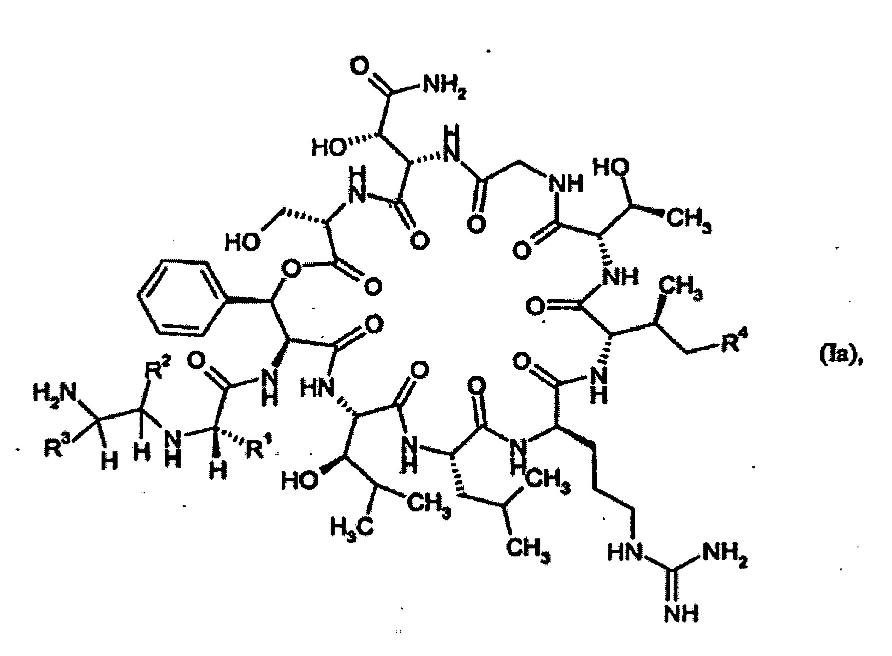 dans laquelle
R¹ est un groupe alkyle en C₁ à C₂
le groupe alkyle en C₁ à C₂ étant substitué par un substituant choisi dans le groupe comprenant le triméthylsilyle, l'alcoxycarbonyle, les groupes cycloalkyle en C₃ à C₆, phényle substitué par alcoxy, 2-pyridyle et 3-pyridyle,
ou
un groupe alkyle en C₄ à C₈,
R² est un atome d'hydrogène ou un groupe alkyle en C₁ à C₄,
R³ est un groupe alkyle en C₁ à C₂,
le groupe alkyle en C₁ à C₂ étant substitué par un substituant triméthylsilyle
ou
un groupe alkyle en C₄ à C₆,
R⁴ est un atome d'hydrogène ou un groupe méthyle
ou un de leurs sels, solvates ou solvates de leurs sels.

2. Composé selon la revendication 1, **caractérisé en ce qu'**il correspond à la formule 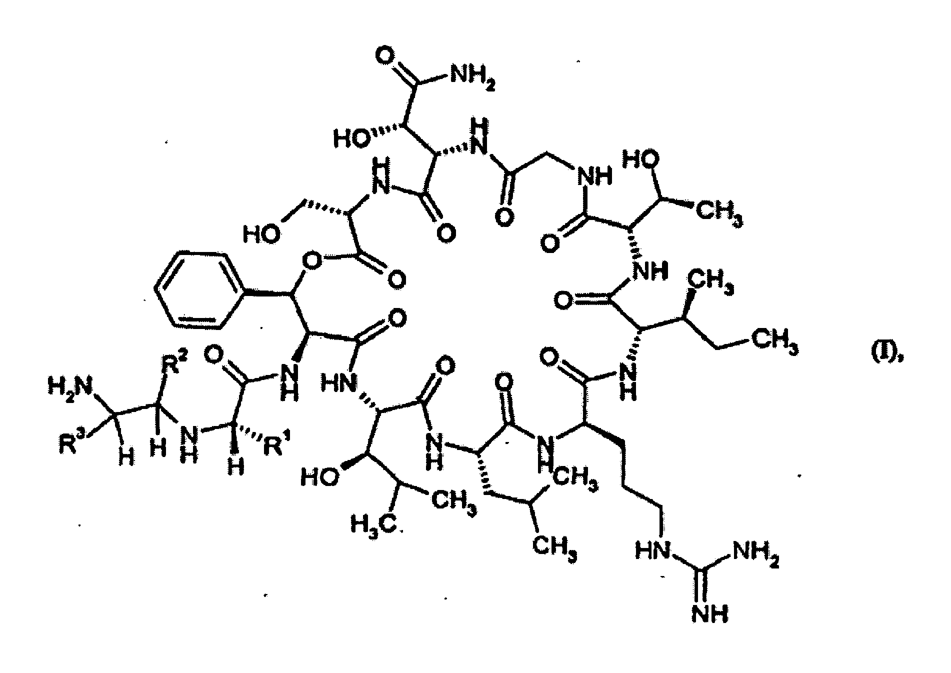 dans laquelle
R¹ est un groupe alkyle en C₁ à C₂
le groupe alkyle en C₁ à C₂ étant substitué par un substituant choisi dans le groupe comprenant le triméthylsilyle, l'alcoxycarbonyle, les groupes cycloalkyle en C₃ à C₆, phényle substitué par alcoxy, 2-pyridyle et 3-pyridyle,
ou
un groupe alkyle en C₄ à C₈,
R² est un atome d'hydrogène ou un groupe alkyle en C₁ à C₄,
R³ est un groupe alkyle en C₁ à C₂,
le groupe alkyle en C₁ à C₂ étant substitué par un substituant triméthylsilyle
ou
un groupe alkyle en C₄ à C₆,
ou un de leurs sels, solvates ou solvates de leurs sels.

3. Composé selon la revendication 2, **caractérisé en ce que**
R¹ est un groupe alkyle en C₁ à C₂
le groupe alkyle en C₁ à C₂ étant substitué par un substituant triméthylsilyle,
ou
un groupe alkyle en C₄ à C₆,
R² est un atome d'hydrogène ou un groupe méthyle,
R³ est un groupe alkyle en C₁ à C₂,
le groupe alkyle en C₁ à C₂ étant substitué par un substituant triméthylsilyle
ou
un groupe alkyle en C₄ à C₆,
ou un de leurs sels, solvates ou solvates de leurs sels.

4. Composé selon l'une des revendications 2 ou 3, **caractérisé en ce que**
R¹ est un groupe triméthylsilylméthyle ou 2,2-diméthylprop-1-yle,
R¹ est un atome d'hydrogène,
R³ est un groupe triméthylsilylméthyle ou 2,2-diméthylprop-1-yle,
ou un de leurs sels, solvates ou solvates de leurs sels.

5. Procédé de production d'un composé de formule (la) selon la revendication 1, **caractérisé en ce qu'**un composé de formule 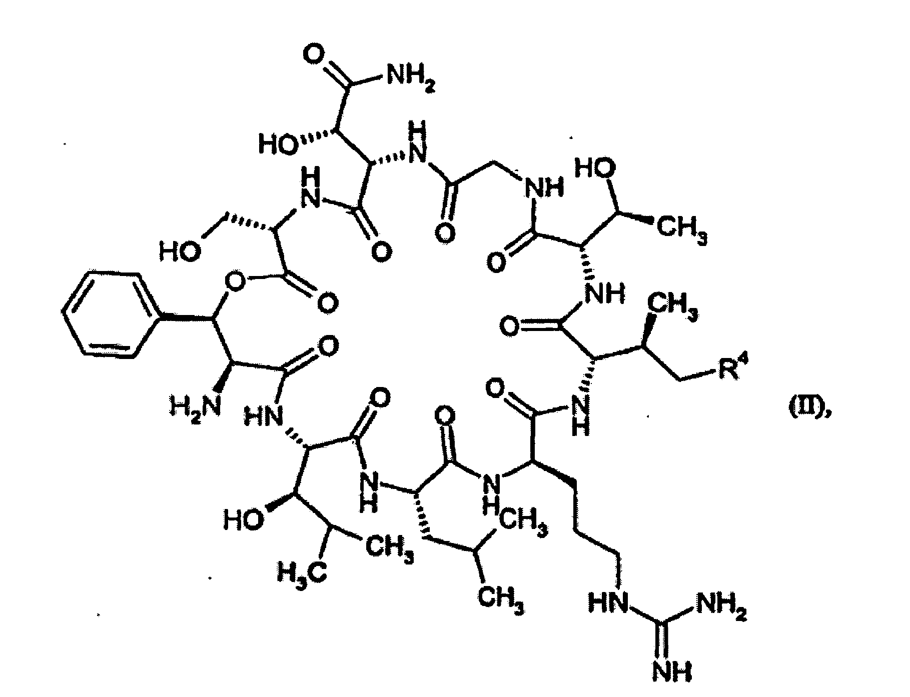 dans laquelle
R⁴ a la signification indiquée à la revendication 1
est converti avec un composé de formule 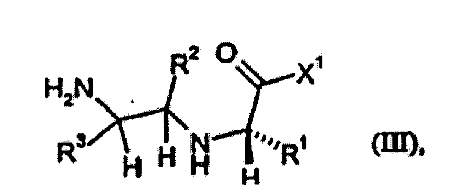
dans laquelle
R¹, R² et R³ ont les significations indiquées à la revendication 1, et
X¹ est un atome d'halogène, de préférence de brome, de chlore ou de fluor, ou un groupe hydroxy.

6. Composé selon l'une des revendications 1 à 4, pour le traitement et/ou la prophylaxie de maladies.

7. Utilisation d'un composé selon l'une des revendications 1 à 4, pour la production d'un médicament destiné au traitement et/ou à la prophylaxie d'infections bactériennes.

8. Médicament contenant un composé selon l'une des revendications 1 à 4, en combinaison avec un adjuvant inerte, non toxique, pharmaceutiquement approprié.

9. Médicament selon la revendication 8, pour le traitement et/ou la prophylaxie d'infections bactériennes.

10. Utilisation d'une quantité efficace de façon antibactérienne d'au moins un composé selon l'une des revendications 1 à 4, d'un médicament selon la revendication 8 ou 9 ou d'un médicament obtenu selon la revendication 7, pour lutter contre les infections bactériennes chez l'être humain et l'animal.
